# EUROPEAN PATENT SPECIFICATION

(11) **EP 2 794 616 B1**
(45) Date of publication and mention of the grant of the patent: **29.06.2016**
(21) Application number: 12787384.2
(22) Date of filing: 19.10.2012
(51) Int. Cl.: C07D 495/04, A61K 31/519, A61P 31/12

(54) **PYRIMIDIN-4-ONE DERIVATIVES AND THEIR USE IN THE TREATMENT, AMELIORATION OR PREVENTION OF A VIRAL DISEASE**
PYRIMIDIN-4-ON-DERIVATE UND IHRE VERWENDUNG BEI DER BEHANDLUNG, LINDERUNG ODER PRÄVENTION EINER VIRUSERKRANKUNG
DÉRIVÉS DE PYRIMIDIN-4-ONE ET LEUR UTILISATION DANS LE TRAITEMENT, L'AMÉLIORATION OU LA PRÉVENTION D'UNE MALADIE VIRALE

(30) Priority: 21.10.2011 US 201161550057 P
(43) Date of publication of application: 29.10.2014
(73) Proprietor: F.Hoffmann-La Roche AG, 4070 Basel (CH); Savira pharmaceuticals GmbH, 1210 Vienna (AT); European Molecular Biology Laboratory, 69117 Heidelberg (DE)
(72) Inventor: CLASSEN-HOUBEN, Dirk, A-8641 St. Marein im Mürztal (AT); WOLKERSTORFER, Andrea, A-1170 Vienna (AT); SZOLAR, Oliver, A-1190 Vienna (AT); SMITH, Mark, San Francisco, CA 94109 (US); SO, Sung-Sau, Verona, New Jersey 07044 (US); CUSACK, Stephen, F-38170 Seyssinet (FR); LANGER, Thierry, 1170 Wien (AT); GIETHLEN, Bruno, F-67120 Altorf (FR); MORICE, Christophe, F-68320 Widensolen (FR); MICHAUT-SIMON, Céline, F-67400 Illkirch Graffenstaden (FR); JUNG, Laurence, F-67118 Geispolsheim (FR)
(74) Representative: Vossius & Partner Patentanwälte Rechtsanwälte mbB
(86) International application number: PCT/EP2012/070760
(87) International publication number: WO 2013/057253

(56) References cited:
- WO-A1-03/074530
- WO-A1-2009/062258
- WO-A2-2011/000566
- ALEEM GANGJEE ET AL: "SYNTHESIS OF CLASSICAL AND NONCLASSICAL 2-AMINO-4-OXO-6-BENZYLTHIENO-[2,3-D]PYRIMI DINES AS POTENTIAL THYMIDYLATE SYNTHASE INHIBITORS", JOURNAL OF HETEROCYCLIC CHEMISTRY, WILEY-BLACKWELL PUBLISHING, INC, US, vol. 41, no. 6, 1 January 2004 (2004-01-01), pages 941-946, XP002512674, ISSN: 0022-152X, DOI: 10.1002/JHET.5570410613 cited in the application

## Description

### Field of the invention

The present invention relates to a compound having the general formula II, optionally in the form of a pharmaceutically acceptable salt, solvate, polymorph, tautomer, racemate, enantiomer, or diastereomer or mixture thereof, 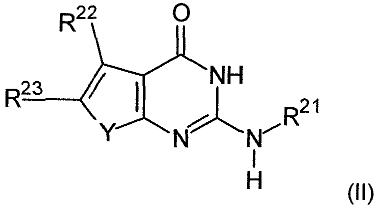 which is useful in treating, ameliorating or preventing a viral disease. Furthermore, specific combination therapies are disclosed.

### Background of the invention

In recent years the serious threat posed by influenza virus to worldwide public health has been highlighted by, firstly, the ongoing low level transmission to humans of the highly pathogenic avian H5N1 strain (63% mortality in infected humans, http://www.who.int/ csr/disease/avian_influenza/en/) and secondly, the unexpected emergence in 2009 of a novel pandemic strain A/H1N1 that has rapidly spread around the entire world (http://www.who.int/csr/disease/swineflu/en/). Whilst the new strain is highly contagious but currently generally only gives mild illness, the future evolution of this virus is unpredictable. In a much more serious, but highly plausible scenario, H5N1 could have been more easily transmissible between humans or the new A/H1N1 could have been more virulent and could have carried the single point mutation that confers Tamiflu resistance (Neumann et al., Nature, 2009 (18; 459(7249) 931-939), as many seasonal H1N1 strains have recently done (Dharan et al., The Journal of the American Medical Association, 2009 Mar 11; 301 (10), 1034-1041; Moscona et al., The New England Journal of Medicine, 2009 (Mar 5;360(10) pp 953-956). In this case, the delay in generating and deploying a vaccine (∼6 months in the relatively favourable case of A/H1N1 and still not a solved problem for H5N1) could have been catastrophically costly in human lives and societal disruption.

It is widely acknowledged that to bridge the period before a new vaccine becomes available and to treat severe cases, as well as to counter the problem of viral resistance, a wider choice of anti-influenza drugs is required. Development of new anti-influenza drugs has therefore again become a high priority, having been largely abandoned by the major pharmaceutical companies once the anti-neuraminidase drugs became available.

An excellent starting point for the development of antiviral medication is structural data of essential viral proteins. Thus, the crystal structure determination of e.g. the influenza virus surface antigen neuraminidase (Von Itzstein, M. et al., (1993), Nature, 363, pp. 418-423) led directly to the development of neuraminidase inhibitors with anti-viral activity preventing the release of virus from the cells, however, not the virus production. These and their derivatives have subsequently developed into the anti-influenza drugs, zanamivir (Glaxo) and oseltamivir (Roche), which are currently being stockpiled by many countries as a first line of defence against an eventual pandemic. However, these medicaments only provide a reduction in the duration of the clinical disease. Alternatively, other anti-influenza compounds such as amantadine and rimantadine target an ion channel protein, i.e., the M2 protein, in the viral membrane interfering with the uncoating of the virus inside the cell. However, they have not been extensively used due to their side effects and the rapid development of resistant virus mutants (Magden, J. et al., (2005), Appl. Microbiol. Biotechnol., 66, pp. 612-621). In addition, more unspecific viral drugs, such as ribavirin, have been shown to work for treating of influenza and other virus infections (Eriksson, B. et al., (1977), Antimicrob. Agents Chemother., 11, pp. 946-951). However, ribavirin is only approved in a few countries (Furuta et al., Antimicrobial Agents and Chemotherapy, 2005 Mar 49(3); 981-986), probably due to severe side effects. Clearly, new antiviral compounds are needed, preferably directed against different targets.

Influenza virus as well as Thogotovirus belong to the family of Orthomyxoviridae which, as well as the family of the Bunyaviridae, including the Hantavirus, Nairovirus, Orthobunyavirus, and Phlebovirus, are negative stranded RNA viruses. Their genome is segmented and comes in ribonucleoprotein particles that include the RNA dependent RNA polymerase which carries out (i) the initial copying of the single-stranded virion RNA (vRNA) into viral mRNAs and (ii) the vRNA replication. This enzyme, a trimeric complex composed of subunits PA, PB1 and PB2, is central to the life cycle of the virus since it is responsible for the replication and transcription of viral RNA. In previous work the atomic structure of two key domains of the polymerase, the mRNA cap-binding domain in the PB2 subunit (Guilligay et al., Antimicrobial Agents and Chemotherapy, 2005 Mar 49(3); pp 981-986) and the endonuclease-active site in the PA subunit (Dias et al., Nature 2009; Apr 16;458(7240); 914-918) have been identified and determined. These two sites are critical for the unique cap-snatching mode of transcription that is used by influenza virus to generate viral mRNAs. For the generation of viral mRNA the polymerase makes use of the so called "cap-snatching" mechanism (Plotch, S. J. et al., (1981), Cell, 23, pp. 847-858; Kukkonen, S. K. et al (2005), Arch. Virol., 150, pp. 533-556; Leahy, M. B. et al, (2005), J. Virol., 71, pp. 8347-8351; Noah, D. L. et al., (2005), Adv. Virus Res., 65, pp. 121-145). A 5' cap (also termed an RNA cap, RNA 7-methylguanosine cap or an RNA m7G cap) is a modified guanine nucleotide that has been added to the 5' end of each cellular messenger RNA. The 5'RNA cap consists of a terminal 7-methylguanosine residue which is linked through a 5'-5'-triphosphate bond to the first transcribed nucleotide. Upon influenza virus infection the 5'RNA cap of cellular mRNA molecules is bound by the viral polymerase complex, specifically the cap-binding domain within the PB2 subunit of the polymerase complex, and the RNA cap together with a stretch of 10 to 15 nucleotides is cleaved by the viral endonuclease which resides within the PA subunit of the viral polymerase complex. The capped RNA fragments then serve as primers for the synthesis of viral mRNA.

The cap-binding domain in the PB2 subunit of the viral polymerase has been unequivocally identified and structurally characterized by Guilligay et al., 2008. Binding the capped host cell mRNA via the cap-binding site and hence bringing the host cell mRNA strand into close spatial vicinity of the endonuclease active site is a prerequisite for the endonuclease to snatch off the cap. Therefore the cap-binding site in PB2 is essential for cap-dependent transcription by the viral RNPs and mandatory for the viral replication cycle. This together with the fact that the PB2 cap-binding domain is structually distinct from other cap binding proteins, this suggests that the ligand binding site is a good target for the development of new antiviral drugs.

Generally, the polymerase complex seems to be an appropriate antiviral drug target since it is essential for synthesis of viral mRNA and viral replication and contains several functional active sites likely to be significantly different from those found in host cell proteins (Magden, J. et al., (2005), Appl. Microbiol. Biotechnol., 66, pp. 612-621). Thus, for example, there have been attempts to interfere with the assembly of polymerase subunits by a 25-amino-acid peptide resembling the PA-binding domain within PB1 (Ghanem, A. et al., (2007), J. Virol., 81, pp. 7801-7804). Furthermore, the endonuclease activity of the polymerase has been targeted and a series of 4-substituted 2,4-dioxobutanoic acid compounds has been identified as selective inhibitors of this activity in influenza viruses (Tomassini, J. et al., (1994), Antimicrob. Agents Chemother., 38, pp. 2827-2837). In addition, flutimide, a substituted 2,6-diketopiperazine, identified in extracts of Delitschia confertaspora, a fungal species, has been shown to inhibit the endonuclease of influenza virus (Tomassini, J. et al., (1996), Antimicrob. Agents Chemother., 40, pp. 1189-1193). Moreover, there have been attempts to interfere with viral transcription by nucleoside analogs, such as 2'-deoxy-2'-fluoroguanosine (Tisdale, M. et al., (1995), Antimicrob. Agents Chemother., 39, pp. 2454-2458).

Specific bicyclic heterocycles, such as thienopyrimidines, are disclosed as being allegedly suitable for treating immune and auto-immune disorders, as well as organ and cells transplant rejections in WO 2010/103130.

The synthesis of 2-amino-4-oxo-6-benzylthieno[2,3-d]pyrimidines as potential thymidylate synthase inhibitors is disclosed in Journal of Heterocyclic Chemistry (2004), 41(6), 941-946.

The synthesis of specific azolothienopyrimidine and pyrimidothienotriazine derivatives is described in the Egyptian Journal of Chemistry (1995), 38(6), 635-44.

N-containing heterocyclic compounds useful for the treatment viral diseases are described in WO2009/062258.

So far, the cap-binding domain in PB2 has not yet been adressed as a target for anti-influenza drug development. It is an object of the present invention to identify compounds which specifically target the influenza virus cap-binding domain and hence are effective against viral diseases and which have improved pharmacological properties.

### Summary of the invention

The present invention is as defined as in the appended claims.

Accordingly, in a first embodiment, the present invention provides a compound having the general formula (II).

It is understood that throughout the present specification the term "a compound having the general formula (II)" encompasses pharmaceutically acceptable salts, solvates, polymorphs, tautomers, racemates, enantiomers, or diastereomers or mixtures thereof unless mentioned otherwise.

A further embodiment of the present invention relates to a pharmaceutical composition comprising a compound having the general formula (II) and optionally one or more pharmaceutically acceptable excipient(s) and/or carrier(s).

The compounds having the general formula (II) are useful for treating, ameliorating or preventing viral diseases.

### Detailed description of the invention

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### Definitions

The term "alkyl" refers to a saturated straight or branched carbon chain.

The term "cycloalkyl" represents a cyclic version of "alkyl". The term "cycloalkyl" is also meant to include bicyclic, tricyclic and polycyclic versions thereof. Unless specified otherwise, the cycloalkyl group can have 5 to 12 carbon atoms.

"Hal" represents F, Cl, Br and I.

The term "aryl" preferably refers to an aromatic monocyclic ring containing 6 carbon atoms, an aromatic bicyclic ring system containing 10 carbon atoms or an aromatic tricyclic ring system containing 14 carbon atoms. Examples are phenyl, naphthyl or anthracenyl, preferably phenyl.

The term "5- or 6-membered heterocycle" or "5- or 6-membered heterocyclic" covers any five or six-membered ring wherein at least one of the carbon atoms in the ring has been replaced by 1, 2, 3, or 4 (for the five membered ring) or 1, 2, 3, 4, or 5 (for the six membered ring) of the same or different heteroatoms, whereby the heteroatoms are selected from O, N and S. The term "heterocyclic ring" also covers heteroaryl rings. Examples include pyrrole, pyrrolidine, oxolane, furan, imidazolidine, imidazole, pyrazole, oxazolidine, oxazole, thiazole, piperidine, pyridine, morpholine, piperazine, and dioxolane.

The term "5- to 10-membered mono- or bicyclic heteroring" covers any mono- or bicyclic ring system which contains at least one heteroatom selected from N, O and S. In a preferred embodiment, the 5- to 10-membered mono- or bicyclic heteroring is

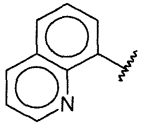

The term "heteroaryl" preferably refers to a five or six-membered aromatic ring wherein one or more of the carbon atoms in the ring have been replaced by 1, 2, 3, or 4 (for the five membered ring) or 1, 2, 3, 4, or 5 (for the six membered ring) of the same or different heteroatoms, whereby the heteroatoms are selected from O, N and S. Examples of the heteroaryl group are given above.

The term "heterocyclyl" covers any five or six-membered ring wherein at least one of the carbon atoms in the ring has been replaced by 1, 2, 3, or 4 (for the five membered ring) or 1, 2, 3, 4, or 5 (for the six membered ring) of the same or different heteroatoms, whereby the heteroatoms are selected from O, N and S. The term "heterocyclyl" also covers heteroaryl rings. Examples include pyrrole, pyrrolidine, oxolane, furan, imidazolidine, imidazole, pyrazole, oxazolidine, oxazole, thiazole, piperidine, pyridine, morpholine, piperazine, and dioxolane.

The term "carbocycle" or "carbocyclic" covers any five or six-membered ring which does not include heteroatoms in the ring. The term "carbocyclic ring" also covers aryl rings.

If a compound or moiety is referred to as being "optionally substituted" it can in each instance include 1 or more of the indicated substituents, whereby the substituents can be the same or different.

The term "pharmaceutically acceptable salt" refers to a salt of a compound of the present invention. Suitable pharmaceutically acceptable salts include acid addition salts which may, for example, be formed by mixing a solution of compounds of the present invention with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compound carries an acidic moiety, suitable pharmaceutically acceptable salts thereof may include alkali metal salts (e.g., sodium or potassium salts); alkaline earth metal salts (e.g., calcium or magnesium salts); and salts formed with suitable organic ligands (e.g., ammonium, quaternary ammonium and amine cations formed using counteranions such as halide, hydroxide, carboxylate, sulfate, phosphate, nitrate, alkyl sulfonate and aryl sulfonate). Illustrative examples of pharmaceutically acceptable salts include, but are not limited to, acetate, adipate, alginate, ascorbate, aspartate, benzenesulfonate, benzoate, bicarbonate, bisulfate, bitartrate, borate, bromide, butyrate, calcium edetate, camphorate, camphorsulfonate, camsylate, carbonate, chloride, citrate, clavulanate, cyclopentanepropionate, digluconate, dihydrochloride, dodecylsulfate, edetate, edisylate, estolate, esylate, ethanesulfonate, formate, fumarate, gluceptate, glucoheptonate, gluconate, glutamate, glycerophosphate, glycolylarsanilate, hemisulfate, heptanoate, hexanoate, hexylresorcinate, hydrabamine, hydrobromide, hydrochloride, hydroiodide, 2-hydroxy-ethanesulfonate, hydroxynaphthoate, iodide, isothionate, lactate, lactobionate, laurate, lauryl sulfate, malate, maleate, malonate, mandelate, mesylate, methanesulfonate, methylsulfate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, 3-phenylpropionate, phosphate/diphosphate, picrate, pivalate, polygalacturonate, propionate, salicylate, stearate, sulfate, subacetate, succinate, tannate, tartrate, teoclate, tosylate, triethiodide, undecanoate, valerate, and the like (see, for example, S. M. Berge et al., "Pharmaceutical Salts", J. Pharm. Sci., 66, pp. 1-19 (1977)).

When the compounds of the present invention are provided in crystalline form, the structure can contain solvent molecules. The solvents are typically pharmaceutically acceptable solvents and include, among others, water (hydrates) or organic solvents. Examples of possible solvates include ethanolates and iso-propanolates.

### Compounds having the general formula (II)

The present invention provides a compound having the general formula (II):

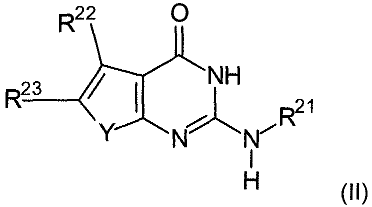

In the appended claims certain provisos are recited. It is understood that any of the compounds which are included in any of the provisos can be excluded, either individually or in combination with other compounds, from one or more of the independent claims having a different category even if it is not currently disclaimed in the independent claim of this category. It is also understood that the disclaimer covers the compounds in the form of their pharmaceutically acceptable salts, solvates, polymorphs, tautomers, racemates, enantiomers, and diastereomers.

The present invention provides a compound having the general formula (II) in which the following definitions apply.
**Y** is S.
**R²¹** is selected from -H, -C₁₋₆alkyl, -(CH₂)_{q}-aryl, -(CH₂)_{q}-heterocyclyl, -(CH₂)_{q}-cycloalkyl, -(CH₂)ₚ-OR²⁵, and -(CH₂)ₚ-NR²⁵R²⁶. Preferably R²¹ is -H, -C₁₋₆ alkyl, or -(CH₂)ₚ-OR²⁵, in a more preferred aspect of this embodiment R²⁵ is H.
**R²²** is selected from -H, -C₁₋₆ alkyl, -(CH₂)_{q}-cycloalkyl, -Hal, -CF₃ and -CN. Preferably R²² is -H, -C₁₋₆ alkyl or Hal (preferably Hal = CI).
**R²³** is selected from -aryl, -heterocyclyl, -cycloalkyl, -C(-R²⁸)(-R²⁹)-aryl, -C(-R²⁸)(-R²⁹)-heterocyclyl, and -C(-R²⁸)(-R²⁹)-cycloalkyl. In a preferred embodiment, R²³ is -(CH₂)_{q}-aryl, or -(CH₂)_{q}-heteroaryl, wherein the aryl group and/or heteroaryl group can be optionally substituted with one or more substituents R²⁷. More preferably R²³ is -phenyl, -benzyl or -pyridyl, wherein the one or more substituents R²⁷ are independently selected from -Hal, -CF₃, -CN, -C₁₋₆ alkyl, -C(O)-C₁₋₆ alkyl, or -(CH₂)_{q}NR²⁵R²⁶, wherein R²⁵ and R²⁶ are independently selected from H and -C₁₋₆ alkyl.
**R²⁵** is selected from -H, -C₁₋₆ alkyl, and -(CH₂CH₂O)ᵣH. Preferably R²⁵ is selected from -H and -C₁₋₆ alkyl.
**R²⁶** is selected from -H, and -C₁₋₆ alkyl.
**R²⁷** is independently selected from -C₁₋₆ alkyl, -C(O)-C₁₋₆ alkyl, -Hal, -CF₃, -CN, -COOR²⁵, -OR²⁵, -(CH₂)_{q}NR²⁵R²⁶, -C(O)-NR²⁵R²⁶, and -NR²⁵-C(O)-C₁₋₆ alkyl. Preferably R²⁷ is independently selected from -Hal, -CF₃, -CN, -C₁₋₆ alkyl, -C(O)-C₁₋₆ alkyl, or -(CH₂)_{q}NR²⁵R²⁶, wherein R²⁵ and R²⁶ are independently selected from H and -C₁₋₆ alkyl.
**R²⁸** and **R²⁹** are independently selected from -H, -C₁₋₆ alkyl, -(CH₂)_{q}-aryl, -(CH₂)_{q}-heterocyclyl, -(CH₂)_{q}-cycloalkyl, -OH, -O-C₁₋₆ alkyl, -O-(CH₂)_{q}-aryl, -O-(CH₂)_{q}-heterocyclyl, and -O-(CH₂)_{q}-cycloalkyl. Preferably R²⁸ and R²⁹ are independently selected from -H and -C₁₋₆ alkyl.
In an alternative embodiment **R²⁸** and **R²⁹** are together =O, -CH₂CH₂-, -CH₂CH₂CH₂-, or -CH₂CH₂CH₂CH₂-.
**p** is 1 to 4.
**q** is 0 to 4, preferably q is 0 or 1.
**r** is 1 to 3.

In the above definitions, the aryl group, heterocyclyl group and/or cycloalkyl group can be optionally substituted with one or more substituents R²⁷, which can be the same or different. The compounds of the present invention can be administered to a patient in the form of a pharmaceutical composition which can optionally comprise one or more pharmaceutically acceptable excipient(s) and/or carrier(s).

The compounds of the present invention can be administered by various well known routes, including oral, rectal, intragastrical, intracranial and parenteral administration, e.g. intravenous, intramuscular, intranasal, intradermal, subcutaneous, and similar administration routes. Oral, intranasal and parenteral administration are particularly preferred. Depending on the route of administration different pharmaceutical formulations are required and some of those may require that protective coatings are applied to the drug formulation to prevent degradation of a compound of the invention in, for example, the digestive tract.

Thus, preferably, a compound of the invention is formulated as a syrup, an infusion or injection solution, a spray, a tablet, a capsule, a capslet, lozenge, a liposome, a suppository, a plaster, a band-aid, a retard capsule, a powder, or a slow release formulation. Preferably the diluent is water, a buffer, a buffered salt solution or a salt solution and the carrier preferably is selected from the group consisting of cocoa butter and vitebesole.

Particular preferred pharmaceutical forms for the administration of a compound of the invention are forms suitable for injectionable use and include sterile aqueous solutions or dispersions and sterile powders for the extemporaneous preparation of sterile injectable solutions or dispersion. In all cases the final solution or dispersion form must be sterile and fluid. Typically, such a solution or dispersion will include a solvent or dispersion medium, containing, for example, water-buffered aqueous solutions, e.g. biocompatible buffers, ethanol, polyol, such as glycerol, propylene glycol, polyethylene glycol, suitable mixtures thereof, surfactants or vegetable oils. A compound of the invention can also be formulated into liposomes, in particular for parenteral administration. Liposomes provide the advantage of increased half life in the circulation, if compared to the free drug and a prolonged more even release of the enclosed drug.

Sterilization of infusion or injection solutions can be accomplished by any number of art recognized techniques including but not limited to addition of preservatives like antibacterial or anti-fungal agents, e.g. parabene, chlorobutanol, phenol, sorbic acid or thimersal. Further, isotonic agents, such as sugars or salts, in particular sodium chloride may be incorporated in infusion or injection solutions.

Production of sterile injectable solutions containing one or several of the compounds of the invention is accomplished by incorporating the respective compound in the required amount in the appropriate solvent with various ingredients enumerated above as required followed by sterilization. To obtain a sterile powder the above solutions are vacuum-dried or freeze-dried as necessary. Preferred diluents of the present invention are water, physiological acceptable buffers, physiological acceptable buffer salt solutions or salt solutions. Preferred carriers are cocoa butter and vitebesole. Excipients which can be used with the various pharmaceutical forms of a compound of the invention can be chosen from the following nonlimiting list:
a) binders such as lactose, mannitol, crystalline sorbitol, dibasic phosphates, calcium phosphates, sugars, microcrystalline cellulose, carboxymethyl cellulose, hydroxyethyl cellulose, polyvinyl pyrrolidone and the like;
b) lubricants such as magnesium stearate, talc, calcium stearate, zinc stearate, stearic acid, hydrogenated vegetable oil, leucine, glycerids and sodium stearyl fumarates,
c) disintegrants such as starches, croscarmellose, sodium methyl cellulose, agar, bentonite, alginic acid, carboxymethyl cellulose, polyvinyl pyrrolidone and the like.

In one embodiment the formulation is for oral administration and the formulation comprises one or more or all of the following ingredients: pregelatinized starch, talc, povidone K 30, croscarmellose sodium, sodium stearyl fumarate, gelatin, titanium dioxide, sorbitol, monosodium citrate, xanthan gum, titanium dioxide, flavoring, sodium benzoate and saccharin sodium.

If a compound of the invention is administered intranasally in a preferred embodiment, it may be administered in the form of a dry powder inhaler or an aerosol spray from a pressurized container, pump, spray or nebulizer with the use of a suitable propellant, e.g., dichlorodifluoromethane, trichlorofluoromethane, dichlorotetrafluoroethane, a hydrofluoro-alkane such as 1,1,1,2-tetrafluoroethane (HFA 134A™) or 1,1,1,2,3,3,3-heptafluoropropane (HFA 227EA™), carbon dioxide, or another suitable gas. The pressurized container, pump, spray or nebulizer may contain a solution or suspension of the compound of the invention, e.g., using a mixture of ethanol and the propellant as the solvent, which may additionally contain a lubricant, e.g., sorbitan trioleate.

Other suitable excipients can be found in the Handbook of Pharmaceutical Excipients, published by the American Pharmaceutical Association, which is herein incorporated by reference.

It is to be understood that depending on the severity of the disorder and the particular type which is treatable with one of the compounds of the invention, as well as on the respective patient to be treated, e.g. the general health status of the patient, etc., different doses of the respective compound are required to elicit a therapeutic or prophylactic effect. The determination of the appropriate dose lies within the discretion of the attending physician. It is contemplated that the dosage of a compound of the invention in the therapeutic or prophylactic use of the invention should be in the range of about 0.1 mg to about 1 g of the active ingredient (i.e. compound of the invention) per kg body weight. However, in a preferred use of the present invention a compound of the invention is administered to a subject in need thereof in an amount ranging from 1.0 to 500 mg/kg body weight, preferably ranging from 1 to 200 mg/kg body weight. The duration of therapy with a compound of the invention will vary, depending on the severity of the disease being treated and the condition and idiosyncratic response of each individual patient. In one preferred embodiment of a prophylactic or therapeutic use, between 100 mg to 200 mg of the compound is orally administered to an adult per day, depending on the severity of the disease and/or the degree of exposure to disease carriers.

As is known in the art, the pharmaceutically effective amount of a given composition will also depend on the administration route. In general the required amount will be higher, if the administration is through the gastrointestinal tract, e.g., by suppository, rectal, or by an intragastric probe, and lower if the route of administration is parenteral, e.g., intravenous. Typically, a compound of the invention will be administered in ranges of 50 mg to 1 g/kg body weight, preferably 100 mg to 500 mg/kg body weight, if rectal or intragastric administration is used and in ranges of 10 to 100 mg/kg body weight, if parenteral administration is used.

If a person is known to be at risk of developing a disease treatable with a compound of the invention, prophylactic administration of the biologically active blood serum or the pharmaceutical composition according to the invention may be possible. In these cases the respective compound of the invention is preferably administered in above outlined preferred and particular preferred doses on a daily basis. Preferably, from 0.1 mg to 1 g/kg body weight once a day, preferably 10 to 200 mg/kg body weight. This administration can be continued until the risk of developing the respective viral disorder has lessened. In most instances, however, a compound of the invention will be administered once a disease/disorder has been diagnosed. In these cases it is preferred that a first dose of a compound of the invention is administered one, two, three or four times daily.

The compounds of the present invention are particularly useful for treating, ameliorating, or preventing viral diseases. The type of viral disease is not particularly limited. Examples of possible viral diseases include, but are not limited to, viral diseases which are caused by Poxviridae, Herpesviridae, Adenoviridae, Papillomaviridae, Polyomaviridae, Parvoviridae, Hepadnaviridae, Retroviridae, Reoviridae, Filoviridae, Paramyxoviridae, Rhabdoviridae, Orthomyxoviridae, Bunyaviridae, Arenaviridae, Coronaviridae, Picornaviridae, Hepeviridae, Caliciviridae, Astroviridae, Togaviridae, Flaviviridae, Deltavirus, Bornaviridae, and prions. Preferably viral diseases which are caused by Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae, Flaviviridae, more preferably viral diseases which are caused by orthomyxoviridae.

**Examples of the various viruses are given in the following table.**

| **Family** | **Virus (preferred examples)** |
|---|---|
| Poxviridae | Smallpox virus |
| | Molluscum contagiosum virus |
| Herpesviridae | Herpes simplex virus |
| | Varicella zoster virus |
| | Cytomegalovirus |
| | Epstein Barr virus |
| | Kaposi's sarcoma-associated |
| | herpesvirus |
| Adenoviridae | Human adenovirus A-F |
| Papillomaviridae | Papillomavirus |
| Polyomaviridae | BK-virus |
| | JC-Virsu |
| Parvoviridae | B19 virus |
| | Adeno associated virus 2/3/5 |
| Hepadnaviridae | Hepatitis B virus |
| Retroviridae | Human immunodeficiency virus |
| | types 1/2 |
| | Human T-cell leukemia virus |
| | Human foamy virus |
| Reoviridae | Reovirus 1/2/3 |
| | Rotavirus A/B/C |
| | Colorado tick fever virus |
| Filoviridae | Ebola virus |
| | Marburg virus |
| Paramyxoviridae | Parainfluenza virus 1-4 |
| | Mumps virus |
| | Measles virus |
| | Respiratory syncytial virus |
| | Hendravirus |
| Rhabdoviridae | Vesicular stomatitis virus |
| | Rabies virus |
| | Mokola virus |
| | European bat virus |
| | Duvenhage virus |
| Orthomyxoviridae | Influenza virus types A-C |
| Bunyaviridae | California encephalitis virus |
| | La Crosse virus |
| | Hantaan virus |
| | Puumala virus |
| | Sin Nombre virus |
| | Seoul virus |
| | Crimean- Congo hemorrhagic fever virus |
| | Sakhalin virus |
| | Rift valley virus |
| | Sandfly fever virus |
| | Uukuniemi virus |
| | |
| Arenaviridae | Lassa virus |
| | Lymphocytic choriomeningitis virus |
| | Guanarito virus |
| | Junin virus, |
| | Machupo virus |
| | Sabia virus |
| Coronaviridae | Human coronavirus |
| Picornaviridae | Human enterovirus types A-D (Poliovirus, |
| | Echovirus, Coxsackie virus A/B) |
| | Rhinovirus types A/B/C |
| | Hepatitis A virus |
| | Parechovirus |
| | Food and mouth disease virus |
| Hepeviridae | Hepatitis E virus |
| Caliciviridae | Norwalk virus |
| | Sapporo virus |
| Astroviridae | Human astrovirus 1 |
| Togaviridae | Ross River virus |
| | Chikungunya virus |
| | O'nyong-nyong virus |
| | Rubella virus |
| Flaviviridae | Tick-borne encephalitis virus |
| | Dengue virus |
| | Yellow Fever virus |
| | Japanese encephalitis virus |
| | Murray Valley virus |
| | St. Louis encephalitis virus |
| | West Nile virus |
| | Hepatitis C virus |
| | Hepatitis G virus |
| | Hepatitis GB virus |
| Deltavirus | Hepatitis deltavirus |
| Bornaviridae | Bornavirus |
| Prions | |

Preferably the compounds of the present invention are employed to treat influenza. Within the present invention, the term "influenza" includes influenza A, B, C, isavirus and thogotovirus and also covers bird flu and swine flu. The subject to be treated is not particularly restricted and can be any vertebrate, such as birds and mammals (including humans).

Without wishing to be bound by theory it is assumed that the compounds of the present invention are capable of inhibiting binding of host mRNA cap structures to the cap-binding domain (CBD), particularly of the influenza virus. More specifically it is assumed that they directly interfere with the CBD of the influenza PB2 protein. However, delivery of a compound into a cell may represent a problem depending on, e.g., the solubility of the compound or its capabilities to cross the cell membrane. The present invention not only shows that the claimed compounds have *in vitro* polymerase inhibitory activity but also *in vivo* antiviral activity.

A possible measure of the *in vitro* polymerase inhibitory activity of the compounds having the formula (I) is the FRET endonuclease activity assay disclosed herein. Preferably the compounds exhibit a % reduction of at least about 50 % at 25 µM in the FRET assay. In this context, the % reduction is the % reduction of the initial reaction velocity (v0) of substrate cleavage of compound-treated samples compared to untreated samples. Preferably the compounds exhibit an IC₅₀ of at least about 40 µM, more preferably at least about 20 µM, in the FRET assay. The half maximal inhibitory concentration (IC₅₀) is a measure of the effectiveness of a compound in inhibiting biological or biochemical function and was calculated from the initial reaction velocities (v0) in a given concentration series ranging from maximum 100 µM to at least 2 nM.

A possible measure of the *in vivo* antiviral activity of the compounds having the formula (I) or (II) is the CPE assay disclosed herein. Preferably the compounds exhibit a % reduction of at least about 30 % at 50 µM. In this connection, the reduction in the virus-mediated cytopathic effect (CPE) upon treatment with the compounds was calculated as follows: The cell viability of infected-treated and uninfected-treated cells was determined using an ATP-based cell viability assay (Promega). The response in relative luminescent units (RLU) of infected-untreated samples was subtracted from the response (RLU) of the infected-treated samples and then normalized to the viability of the corresponding uninfected sample resulting in % CPE reduction. Preferably the compounds exhibit an IC₅₀ of at least about 45 µM, more preferably at least about 10 µM, in the CPE assay. The half maximal inhibitory concentration (IC₅₀) is a measure of the effectiveness of a compound in inhibiting biological or biochemical function and was calculated from the RLU response in a given concentration series ranging from maximum 100 µM to at least 100 nM.

A possible measure of the *in vitro* polymerase inhibitory activity of the compounds having the formula (II) is the Biacore binding assay disclosed herein. The Biacore system is based on an optical phenomenon known as surface plasmon resonance (SPR). This technique is the basis for measuring adsorption of material onto planar metal surfaces such as gold or silver. SPR is used as a powerful technique to measure biomolecular interactions in real-time in a label free environment. While one of the interactants is immobilized to the sensor surface, the other is free in solution and passed over the surface. Association and dissociation is measured in arbitrary units and displayed in a graph called the sensorgram.

The PB2 cap binding domain (CBD) of an avian H5N1 influenza virus was immobilized on the surface of a CM7 sensor chip (GE Healthcare) by amine coupling according to the manufacturer's protocol. The protein was diluted in a 10 mM phosphate buffer pH 6.5. As running buffer for immobilization a HBS-EP buffer (10mM HEPES, 150mM NaCl, 3mM EDTA, 0,005 % Surfactant p20) was used. Using a protein concentration of 30 µg/ml and a contact time of 12 min an immobilization level of approximately 8000 RU (relative response units) was achieved.

For compound screening a running buffer containing 10 mM TRIS, 3 mM EDTA, 150 mM NaCl, 0.005 % Surfactant p20 (GE Healthcare/Biacore), 1 mM DTT, 0.5 % DMSO was used. 2 mM DMSO stock solutions of each compound were diluted in 1.005X sample buffer without DMSO (1.005X TRIS/EDTA/NaCl/p20/DTT; diluted from a 10X stock) to a final compound concentration of 10 µM and 0.5 % DMSO. m7GTP (Sigma Aldrich) and SAV-7160 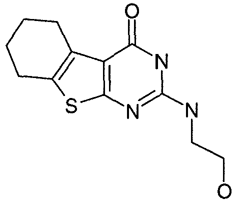 were used as references and chip stability controls at a concentration of 4 mM and 10 µM, respectively. Stock solutions of each reference compound were made and aliquots were stored at -20°C. In this context, the RU is a measure for the binding of the compound to the PB2-CBD and is generally assessed in relation to the binding in RU of SAV-7160.

For buffer bulk effects (matrix) was accounted by reducing the response obtained for the reference flow cell Fc1 from the active flow cell Fc2 resulting in relative response units (RU) reflecting binding of the compounds to the ligand. Organic solvents such as DMSO in the buffer cause high bulk effects which differ in the reference flow cell and the active flow cell due to ligand immobilization. To account for these differences, a calibration curve was established. Eight DMSO concentrations ranging from 0.1 % to 1.5 % in buffer were measured and a linear calibration curve was calculated by plotting Fc2-Fc1 vs. Fc1. The relative response of each sample was then corrected by the solvent factor given by the respective Fc1 signal on the calibration curve and the corresponding Fc2-Fc1 difference. To account for the different size of the compounds, the buffer and solvent corrected response units were normalized to the molecular weight.

Affinity constants (KD values) were determined by measuring the binding affinity of the analyte to the ligand over a concentration range ranging from 200 µM to 1 nM. The KD value is that concentration at which 50% of the binding sites are saturated and was calculated using a linear curve fit model.

In the Biacore assay the binding (RU) of the compounds to the immobilized PB2-CBD is preferably at most 15 RU, more preferably at most 7.5 RU. The affinity constant (KD) is preferably at most 50 µM, more preferably at most 10 µM.

The compounds having the general formula (II) can be used in combination with one or more other medicaments. The type of the other medicaments is not particularly limited and will depend on the disorder to be treated. Preferably the other medicament will be a further medicament which is useful in treating, ameliorating or preventing a viral disease, more preferably a further medicament which is useful in treating, ameliorating or preventing influenza.

The following combinations of medicaments are envisaged as being particularly suitable:
(i) The combination of endonuclease and cap binding inhibitors (particularly targeting influenza). The endonuclease inhibitors are not particularly limited and can be any endonuclease inhibitor, particularly any viral endonuclease inhibitor. Preferred endonuclease inhibitors are those having the general formula (I).

The cap binding inhibitors are not are not particularly limited either and can be any cap binding inhibitor, particularly any viral cap binding inhibitor. Preferred cap binding inhibitors are those having the general formula (II) and/or the compounds disclosed in WO2011/000566.

The compounds of WO2011/000566 have the general formula (XXI): 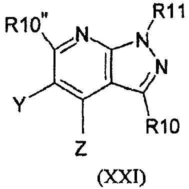 or a pharmaceutically effective salt, a solvate, a prodrug, a tautomer, a racemate, an enantiomer or a diastereomer thereof; wherein
one of Y and Z is -XR¹² and the other is R^{10'};
R¹⁰, R^{10'} and R^{10"} are each individually selected from the group consisting of hydrogen, C₁-C₆-alkyl, C₂-C₆-alkenyl, C₂-C₈-alkynyl, -(CH₂)ₙC(O)OH, -(CH₂)ₙC(O)OR¹⁶, -(CH₂)ₙOH, -(CH₂)ₙOR¹⁶, -CF₃, -(CH₂)ₙ-cycloalkyl, -(CH₂)ₙC(O)NH₂, -(CH₂)ₙC(O)NHR¹⁶, -(CH₂)ₙC(O)NR¹⁶R¹⁷, -(CH₂)ₙS(O)₂NH₂, -(CH₂)ₙS(O)₂NHR¹⁶, -(CH₂)ₙS(O)₂NR¹⁶R¹⁷, -(CH₂)ₙS(O)₂R¹⁶, halogen, -CN, -(CH₂)ₙ-aryl, -(CH₂)n-heteroaryl, -(CH₂)ₙNH₂, -(CH₂)ₙNHR¹⁶, and -(CH₂)ₙNR¹⁶R¹⁷; optionally substituted;
**R¹¹** is selected from the group consisting of hydrogen, C₁-C₆-alkyl, -CF₃, C₂-C₆-alkenyl, C₂-C₈-alkynyl, -(CH₂)ₙ-cycloalkyl, -(CH₂)ₙ-aryl, -(CH₂)ₙ-heterocycloalkyl and -(CH₂)ₙ-heteroaryl; optionally substituted;
**X** is selected from the group consisting of CH₂, C(O), C(S), CH(OH), CH(OR¹⁶), S(O)₂, -S(O)₂-N(H)-, -S(O)₂-N(R¹⁶)-, -N(H)-S(O)₂-, -N(R¹⁶)-S(O)₂-, C(=NH), C(=N-R¹⁶), CH(NH₂), CH(NHR¹⁶), CH(NR¹⁶R¹⁷), -C(O)-N(H)-, -C(O)-N(R¹⁶)-, -N(H)-C(O)-, -N(R¹⁶)-C(O)-, N(H), N(-R¹⁶) and O;
**R¹²** is selected from the group consisting of C₁-G₆-alkyl, -CF₃, C₂-C₆-alkenyl, C₂-C₈-alkynyl, -(CH₂)ₙ-cycloalkyl, -(CH₂)ₙ-heterocycloalkyl, -(CH₂)ₙ-aryl, -NR¹⁶R¹⁷, and -(CH₂)ₙ-heteroaryl; optionally substituted;
**R¹⁶** and **R¹⁷** are independently selected from the group consisting of C₁-C₆-alkyl, C₂-C₆-alkenyl, C₂-C₆-alkynyl, -(CH₂)ₙ-cycloalkyl, -(CH₂)ₙ-aryl, -CF₃, -C(O)R¹⁸ and -S(O)₂R¹⁸; optionally substituted;
**R¹⁸** is independently selected from the group consisting of C₁-C₆-alkyl, C₂-C₆-alkenyl, C₂-C₆-alkynyl, -(CH₂)ₙ-cycloalkyl and -CF₃; optionally substituted; and
**n** is in each instance selected from 0, 1 and 2.

In the context of WO2011/000566 the term "optionally substituted" in each instance refers to between 1 and 10 substituents, e.g. 1, 2, 3, 4, 5, 6, 7, 8, 9, or 10 substituents which are in each instance preferably independently selected from the group consisting of halogen, in particular F, Cl, Br or I; -NO₂, -CN, -OR', -NR'R", -(CO)OR', -(CO)OR"', -(CO)NR'R", -NR'COR"", -NR'COR', -NR"CONR'R", -NR"SO₂A, -COR"'; -SO₂NR'R", -OOCR"', -CR"'R""OH, -R"'OH, =O, and -E;
R' and R" is each independently selected from the group consisting of hydrogen, alkyl, alkenyl, alkynyl, -OE, cycloalkyl, heterocycloalkyl, aryl, heteroaryl, and aralkyl or together form a heteroaryl, or heterocycloalkyl; optionally substituted;
R'" and R"" is each independently selected from the group consisting of alkyl, alkenyl, alkynyl, cycloalkyl, heterocycloalkyl, alkoxy, aryl, aralkyl, heteroaryl, and -NR'R"; and
E is selected from the group consisting of alkyl, alkenyl, cycloalkyl, alkoxy, alkoxyalkyl, heterocycloalkyl, an alicyclic system, aryl and heteroaryl; optionally substituted.

Widespread resistance to both classes of licensed influenza antivirals (M2 ion channel inhibitors (adamantanes) and neuraminidase inhibitors (Oseltamivir)) occurs in both pandemic and seasonal viruses, rendering these drugs to be of marginal utility in the treatment modality. For M2 ion channel inhibitors, the frequency of viral resistance has been increasing since 2003 and for seasonal influenza A/H3N2, adamantanes are now regarded as ineffective. Virtually all 2009 H1N1 and seasonal H3N2 strains are resistant to the adamantanes (rimantadine and amantadine), and the majority of seasonal H1 N1 strains are resistant to oseltamivir, the most widely prescribed neuraminidase inhibitor (NAI). For oseltamivir the WHO reported on significant emergence of influenza A/H1N1 resistance starting in the influenza season 2007/2008; and for the second and third quarters of 2008 in the southern hemisphere. Even more serious numbers were published for the fourth quarter of 2008 (northern hemisphere) where 95% of all tested isolates revealed no Oseltamivir-susceptibility. Considering the fact that now most national governments have been stockpiling Oseltamivir as part of their influenza pandemic preparedness plan, it is obvious that the demand for new, effective drugs is growing significantly. To address the need for more effective therapy, preliminary studies using double or even triple combinations of antiviral drugs with different mechanisms of action have been undertaken. Adamantanes and neuraminidase inhibitors in combination were analysed in vitro and in vivo and found to act highly synergistically. However, it is known that for both types of antivirals resistant viruses emerge rather rapidly and this issue is not tackled by combining these established antiviral drugs.

Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. These two targets are located within distinct subunits of the polymerase complex and thus represent unique drug targets. Due to the fact that both functions are required for the so-called "cap-snatching" mechanism mandatory for viral transcription, concurrent inhibition of both functions is expected to act highly synergistically. This highly efficient drug combination would result in lower substance concentrations and hence improved dose-response-relationships and better side effect profiles.

Both of these active sites are composed of identical residues in all influenza A strains (e.g., avian and human) and hence this high degree of sequence conservation underpins the perception that these targets are not likely to trigger rapid resistant virus generation. Thus, endonuclease and cap-binding inhibitors individually and in combination are ideal drug candidates to combat both seasonal and pandemic influenza, irrespectively of the virus strain.

The combination of an endonuclease inhibitor and a cap-binding inhibitor or a dual specific polymerase inhibitor targeting both the endonuclease active site and the cap-binding domain would be effective against virus strains resistant against adamantanes and neuraminidase inhibitors and moreover combine the advantage of low susceptibility to resistance generation with activity against a broad range of virus strains.
(ii) The combination of inhibitors of different antiviral targets (particularly targeting influenza) focusing on the combination with (preferably influenza) polymerase inhibitors as dual or multiple combination therapy. Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. The combination of a polymerase inhibitor specifically addressing a viral intracellular target with an inhibitor of a different antiviral target is expected to act highly synergistically. This is based on the fact that these different types of antiviral drugs exhibit completely different mechanisms of action and pharmacokinetics properties which act advantageously and synergistically on the antiviral efficacy of the combination.

This highly efficient drug combination would result in lower substance concentrations and hence improved dose-response-relationships and better side effect profiles. Moreover, advantages described under (i) for polymerase inhibitors would prevail for combinations of inhibitors of different antiviral targets with polymerase inhibitors. Typically at least one compound selected from the first group of polymerase inhibitors is combined with at least one compound selected from the second group of polymerase inhibitors.

The first group of polymerase inhibitors which can be used in this type of combination therapy includes, but is not limited to, the compounds having the general formula (I) described below, the compounds having the general formula (II) described above and/or the compounds disclosed in WO2011/000566.

The second group of polymerase inhibitors which can be used in this type of combination therapy includes, but is not limited to, compounds disclosed in WO 2010/110231, WO 2010/110409, WO 2006/030807 and US 5,475,109 as well as flutimide and analogues, favipiravir and analogues, epigallocatechin gallate and analogues, as well as nucleoside analogs such as ribavirine.
(iii) The combination of polymerase inhibitors with neuramidase inhibitors

Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. The combination of a polymerase inhibitor specifically addressing a viral intracellular target with an inhibitor of a different extracellular antiviral target, especially the (e.g., viral) neuraminidase is expected to act highly synergistically. This is based on the fact that these different types of antiviral drugs exhibit completely different mechanisms of action and pharmacokinetic properties which act advantageously and synergistically on the antiviral efficacy of the combination.

This highly efficient drug combination would result in lower substance concentrations and hence improved dose-response-relationships and better side effect profiles. Moreover, advantages described under (i) for polymerase inhibitors would prevail for combinations of inhibitors of different antiviral targets with polymerase inhibitors. Typically at least one compound selected from the above mentioned first group of polymerase inhibitors is combined with at least one neuramidase inhibitor.

The neuraminidase inhibitor (particularly influenza neuramidase inhibitor) is not specifically limited. Examples include zanamivir, oseltamivir, peramivir, KDN DANA, FANA, and cyclopentane derivatives.
(iv) The combination of polymerase inhibitors with M2 channel inhibitors

Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. The combination of a polymerase inhibitor specifically addressing a viral intracellular target with an inhibitor of a different extracellular and cytoplasmic antiviral target, especially the viral M2 ion channel, is expected to act highly synergistically. This is based on the fact that these different types of antiviral drugs exhibit completely different mechanisms of action and pharmacokinetic properties which act advantageously and synergistically on the antiviral efficacy of the combination.

This highly efficient drug combination would result in lower substance concentrations and hence improved dose-response-relationships and better side effect profiles. Moreover, advantages described under (i) for polymerase inhibitors would prevail for combinations of inhibitors of different antiviral targets with polymerase inhibitors.

Typically at least one compound selected from the above mentioned first group of polymerase inhibitors is combined with at least one M2 channel inhibitor.

The M2 channel inhibitor (particularly influenza M2 channel inhibitor) is not specifically limited. Examples include amantadine and rimantadine.
(v) The combination of polymerase inhibitors with alpha glucosidase inhibitors

Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. The combination of a polymerase inhibitor specifically addressing a viral intracellular target, with an inhibitor of a different extracellular target, especially alpha glucosidase, is expected to act highly synergistically. This is based on the fact that these different types of antiviral drugs exhibit completely different mechanisms of action and pharmacokinetic properties which act advantageously and synergistically on the antiviral efficacy of the combination.

This highly efficient drug combination would result in lower substance concentrations and hence improved dose-response-relationships and better side effect profiles. Moreover, advantages described under (i) for polymerase inhibitors would prevail for combinations of inhibitors of different antiviral targets with polymerase inhibitors.

Typically at least one compound selected from the above mentioned first group of polymerase inhibitors is combined with at least one alpha glucosidase inhibitor.

The alpha glucosidase inhibitor (particularly influenza alpha glucosidase inhibitor) is not specifically limited. Examples include the compounds described in Chang et al., Antiviral Research 2011, 89, 26-34.
(vi) The combination of polymerase inhibitors with ligands of other influenza targets

Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. The combination of a polymerase inhibitor specifically addressing a viral intracellular target with an inhibitor of different extracellular, cytoplasmic or nucleic antiviral targets is expected to act highly synergistically. This is based on the fact that these different types of antiviral drugs exhibit completely different mechanisms of action and pharmacokinetic properties which act advantageously and synergistically on the antiviral efficacy of the combination.

This highly efficient drug combination would result in lower substance concentrations and hence improved dose-response-relationships and better side effect profiles. Moreover, advantages described under (i) for polymerase inhibitors would prevail for combinations of inhibitors of different antiviral targets with polymerase inhibitors.

Typically at least one compound selected from the above mentioned first group of polymerase inhibitors is combined with at least one ligand of another influenza target.

The ligand of another influenza target is not specifically limited. Examples include compounds acting on the sialidase fusion protein, e.g. Fludase (DAS181), siRNAs and phosphorothioate oligonucleotides, signal transduction inhibitors (ErbB tyrosine kinase, Abl kinase family, MAP kinases, PKCa-mediated activation of ERK signaling as well as interferon (inducers).
(vii) The combination of (preferably influenza) polymerase inhibitors with a compound used as an adjuvance to minimize the symptoms of the disease (antibiotics, antiinflammatory agents like COX inhibitors (e.g., COX-1/COX-2 inhibitors, selective COX-2 inhibitors), lipoxygenase inhibitors, EP ligands (particularly EP4 ligands), bradykinin ligands, and/or cannabinoid ligands (e.g., CB2 agonists). Influenza virus polymerase inhibitors are novel drugs targeting the transcription activity of the polymerase. Selective inhibitors against the cap-binding and endonuclease active sites of the viral polymerase severely attenuate virus infection by stopping the viral reproductive cycle. The combination of a polymerase inhibitor specifically addressing a viral intracellular target with an compound used as an adjuvance to minimize the symptoms of the disease address the causative and symptomatic pathological consequences of viral infection. This combination is expected to act synergistically because these different types of drugs exhibit completely different mechanisms of action and pharmacokinetic properties which act advantageously and synergistically on the antiviral efficacy of the combination.

This highly efficient drug combination would result in lower substance concentrations and hence improved dose-response-relationships and better side effect profiles. Moreover, advantages described under (i) for polymerase inhibitors would prevail for combinations of inhibitors of different antiviral targets with polymerase inhibitors.

### Compounds having the general formula (I)

In a disclosure the compounds having the general formula (I) are identified in the following.

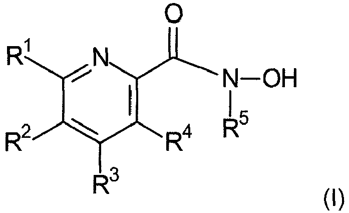

It is understood that throughout the present specification the term "a compound having the general formula (I)" encompasses pharmaceutically acceptable salts, solvates, polymorphs, prodrugs, tautomers, racemates, enantiomers, or diastereomers or mixtures thereof unless mentioned otherwise.

The following definitions apply with respect to the compounds having the general formula (I).

**R¹** is selected from -H, -C₁₋₆ alkyl, -(C₃₋₇ cycloalkyl) and -CH₂-(C₃₋₇ cycloalkyl). Preferably R¹ is selected from -H, and -C₁₋₆ alkyl. Even more preferably R¹ is -H. **R²** is selected from -H, 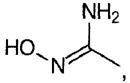 -C₁₋₆ alkyl, -Hal, -(C₃₋₇ cycloalkyl), -CH₂-(C₃₋₇ cycloalkyl), -(CH₂)ₘ-(optionally substituted aryl), and -(optionally substituted 5- or 6-membered heterocyclic ring which contains at least one heteroatom selected from N, O and S). Preferably R² is selected from -H, 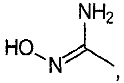 -C₁₋₆ alkyl, -(CH₂)ₘ-(optionally substituted aryl), -(optionally substituted 5- or 6-membered heterocyclic ring which contains at least one heteroatom selected from N, O and S). Even more preferably R² is selected from -H, -C₁₋₆ alkyl, -phenyl, with R² being -H being most preferred. With respect to R² the heterocyclic ring is not particularly limited but it is preferably piperidine or pyrrolidine.

The substituent(s) of the optionally substituted aryl and the optionally substituted heterocyclic ring are independently selected from -C₁₋₄ alkyl, -halogen, -CN, -CHal₃, -aryl, -NR⁶R⁷, and -CONR⁶R⁷. Preferred examples of the substituent being selected from -C₁₋₄ alkyl.
**R³** is selected from -H;
-C₁₋₆ alkyl;
-(CH₂)ₙ-NR⁶R⁸ (with respect to this substituent n is preferably 0 or 1, more preferably 0); and
-(optionally substituted 5- or 6-membered carbo- or heterocyclic ring wherein the heterocyclic ring contains at least one heteroatom selected from N, O and S). The heterocyclic ring can be any carbo- or heterocyclic ring but is preferably phenyl, piperidine, morpholine, or piperazine.

The substituent of the carbo- or heterocyclic ring is selected from -Hal, -C₁₋₄ alkyl, -NR⁹R¹⁰, -(CH₂)ₙ-OH, -C(O)-NR⁹R¹⁰, -SO₂-NR⁹R¹⁰, -NH-C(O)-O-R¹¹, -C(O)-O-R¹¹, and a 5- or 6-membered heterocyclic ring which contains at least one heteroatom selected from N, O and S (with respect to the substituent of the carbo- or heterocyclic ring the heterocyclic ring as a substituent is preferably pyrrolidine, piperidine, or dioxolane).

In a disclosure, R³ is selected from -H; -C₁₋₆ alkyl;
-NR⁶-SO₂-(CH₂)ₙ-(optionally substituted aryl), wherein the substituent is preferably selected from -Hal, and -CF₃;
-(optionally substituted aryl), wherein the substituent is preferably selected from Hal, -NR⁹R¹⁰, and -C(O)-O-R¹¹; and
-(optionally substituted 5- or 6-membered heterocyclic ring wherein the heterocyclic ring contains at least one heteroatom selected from N, O and S), wherein the substituent is preferably selected from -Hal, -NR⁹R¹⁰, -C(O)-O-R¹¹, and a 5- or 6-membered heterocyclic ring which contains at least one heteroatom selected from N, O and S such as pyrrolidine, piperidine, or dioxolane.

In one disclosure R¹ and R² taken together can form a phenyl ring.

In an alternative disclosure R² and R³ taken together can form a phenyl ring.

**R⁴** is -H.

**R⁵** is selected from the group consisting of -H or -(CH₂)ₙ-(optionally substituted aryl), preferably **R⁵** is selected from the group consisting of -H or -(CH₂)-(optionally substituted phenyl), even more preferably **R⁵** is -H. In the definition of R⁵ n is 0, 1, 2, or 3, preferably n is 0 or 1, more preferably n is 1. With respect to R⁵ the substituent is selected from -Hal and -C₁₋₄ alkyl.

In an alternative disclosure, R⁴ and R⁵ together form a methylene group -CH₂-, ethylene group -CH₂CH₂- or ethyne group -CHCH-, which can be optionally substituted by -C₁₋₄ alkyl, -halogen, -CHal₃, -R⁶R⁷, -OR⁶, -CONR⁶R⁷, -SO₂R⁶R⁷, aryl or heteroaryl.

**R⁶** is selected from -H and -C₁₋₄ alkyl and is, e.g., -H.

**R⁷** is selected from -H and -C₁₋₄ alkyl.

**R⁸** is selected from -H, -C₁₋₆ alkyl, -(CH₂)ₙ-(optionally substituted aryl), -SO₂-(CH₂)ₙ-(optionally substituted aryl), -SO₂-(CH₂)ₙ-(optionally substituted 5- to 10-membered mono-or bicyclic heteroring which contains at least one heteroatom selected from N, O and S),-(CH₂)ₙ-(optionally substituted 5- or 6-membered heterocyclic ring which contains at least one heteroatom selected from N, O and S) (preferably the heterocyclic ring is piperidine or pyrrolidine), wherein the substituent is selected from -Hal, -CF₃, -C₁₋₄ alkyl, and -(CH₂)ₙ-aryl. In a preferred option, R⁸ can be -SO₂-(CH₂)ₙ-(optionally substituted aryl), with n being preferably 0 or 1, more preferably being 1.

**R⁹** is selected from -H, -C₁₋₄ alkyl, and -C₁₋₄ alkylene-NR¹¹R¹¹.

**R¹⁰** is selected from -H, -C₁₋₄ alkyl, and -C₁₋₄ alkylene-NR¹¹R¹¹.

**R¹¹** is selected from -H, -CF₃, and -C₁₋₄ alkyl.

Each **m** is 0 or 1.

Each **n** is independently 0, 1, 2, or 3.

Without wishing to be bound by theory it is assumed that the compounds having the general formula (I) are capable of inhibiting endonuclease activity, particularly of the influenza virus. More specifically it is assumed that they directly interfere with the N-terminal part of the influenza PA protein, which harbours endonuclease activity. However, delivery of a compound into a cell may represent a problem depending on, e.g., the solubility of the compound or its capabilities to cross the cell membrane. The present disclosure not only shows that the compounds have *in vitro* polymerase inhibitory activity but also *in vivo* antiviral activity.

### REFERENCE EXAMPLES 1 TO 111

### FRET endonuclease activity assay

The influenza A virus (IAV) PA-Nter fragment (amino acids 1 - 209) harbouring the influenza endonuclease activity was generated and purified as described in Dias et al., 2009. The protein was dissolved in buffer containing 20mM Tris pH 8.0, 100mM NaCl and 10mM β-mercaptoethanol and aliquots were stored at-20 °C.

A 20 bases dual-labelled RNA oligo with 5'-FAM fluorophore and 3'-BHQ1 quencher was used as a substrate to be cleaved by the endonuclease activity of the PA-Nter. Cleavage of the RNA substrate frees the fluorophore from the quencher resulting in an increase of the fluorescent signal.

All assay components were diluted in assay buffer containing 20mM Tris-HCl pH 8.0, 100mM NaCl, 1 mM MnCl₂, 10mM MgCl₂ and 10mM β-mercaptoethanol. The final concentration of PA-Nter was 0.5µM and 1.6µM RNA substrate. The test compounds were dissolved in DMSO and generally tested at two concentrations or a concentration series resulting in a final plate well DMSO concentration of 0.5 %. In those cases where the compounds were not soluble at that concentration, they were tested at the highest soluble concentration. SAV-6004 was used as a reference in the assay at a concentration of 0.1 µM.

5µl of each compound dilution was provided in the wells of white 384-well microtiter plates (PerkinElmer) in eight replicates. After addition of PA-Nter dilution, the plates were sealed and incubated for 30min at room temperature prior to the addition of 1.6µM RNA substrate diluted in assay buffer. Subsequently, the increasing fluorescence signal of cleaved RNA was measured in a microplate reader (Synergy HT, Biotek) at 485nm excitation and 535nm emission wavelength. The kinetic read interval was 35sec at a sensitivity of 35. Fluorescence signal data over a period of 20min were used to calculate the initial velocity (v0) of substrate cleavage. Final readout was the % reduction of v0 of compound-treated samples compared to untreated. The half maximal inhibitory concentration (IC₅₀) is a measure of the effectiveness of a compound in inhibiting biological or biochemical function and was calculated from the initial reaction velocities (v0) ina given concentration series ranging from maximum 100 µM to at least 2 nM.

### Cytopathic effect (CPE) assay

The influenza A virus (IAV) was obtained from American Tissue Culture Collection (A/Aichi/2/68 (H3N2); VR-547). Virus stocks were prepared by propagation of virus on Mardin-Darby canine kidney (MDCK; ATCC CCL-34) cells and infectious titres of virus stocks were determined by the 50 % tissue culture infective dose (TCID₅₀) analysis as described in Reed, L. J., and H. Muench. 1938, Am. J. Hyg. 27:493-497..

MDCK cells were seeded in 96-well plates at 2×10⁴ cells/well using DMEM/Ham's F-12 (1:1) medium containing 10 % foetal bovine serum (FBS), 2 mM L-glutamine and 1 % antibiotics (all from PAA). Until infection the cells were incubated for 5 hrs at 37 °C, 5.0 % CO₂ to form a ∼80 % confluent monolayer on the bottom of the well. Each test compound was dissolved in DMSO and generally tested at 25 µM and 250 µM. In those cases where the compounds were not soluble at that concentration they were tested at the highest soluble concentration. The compounds were diluted in infection medium (DMEM/Ham's F-12 (1:1) containing 5 µg/ml trypsin, and 1 % antibiotics) for a final plate well DMSO concentration of 1 %. The virus stock was diluted in infection medium (DMEM/Ham's F-12 (1:1) containing 5 µg/ml Trypsin, 1 % DMSO, and 1 % antibiotics) to a theoretical multiplicity of infection (MOI) of 0.05.

After removal of the culture medium and one washing step with PBS, virus and compound were added together to the cells. In the wells used for cytotoxicity determination (i.e. in the absence of viral infection), no virus suspension was added. Instead, infection medium was added. Each treatment was conducted in two replicates. After incubation at 37 °C, 5 % CO₂ for 48 hrs, each well was observed microscopically for apparent cytotoxicity, precipitate formation, or other notable abnormalities. Then, cell viability was determined using CellTiter-Glo luminescent cell viability assay (Promega). The supernatant was removed carefully and 65 µl of the reconstituted reagent were added to each well and incubated with gentle shaking for 15 min at room temperature. Then, 60 µl of the solution was transferred to an opaque plate and luminescence (RLU) was measured using Synergy HT plate reader (Biotek).

Relative cell viability values of uninfected-treated versus uninfected-untreated cells were used to evaluate cytotoxicity of the compounds. Substances with a relative viability below 80 % at the tested concentration were regarded as cytotoxic and retested at lower concentrations.

Reduction in the virus-mediated cytopathic effect (CPE) upon treatment with the compounds was calculated as follows: The response (RLU) of infected-untreated samples was subtracted from the response (RLU) of the infected-treated samples and then normalized to the viability of the corresponding uninfected sample resulting in % CPE reduction. The half maximal inhibitory concentration (IC₅₀) is a measure of the effectiveness of a compound in inhibiting biological or biochemical function and was calculated from the RLU response in a given concentration series ranging from maximum 100 µM to at least 100 nM.

### Biacore assay

The PB2 cap binding domain (CBD) of an avian H5N1 influenza virus was immobilized on the surface of a CM7 sensor chip (GE Healthcare) by amine coupling according to the manufacturer's protocol. The protein was diluted in a 10 mM phosphate buffer pH 6.5. As running buffer for immobilization a HBS-EP buffer (10mM HEPES, 150mM NaCl, 3mM EDTA, 0,005 % Surfactant p20) was used. Using a protein concentration of 30 µg/ml and a contact time of 12 min an immobilization level of approximately 8000 RU (relative response units) was achieved.

For compound screening a running buffer containing 10 mM TRIS, 3 mM EDTA, 150 mM NaCl, 0.005 % Surfactant p20 (GE Healthcare/Biacore), 1 mM DTT, 0.5 % DMSO was used. 2 mM DMSO stock solutions of each compound were diluted in 1.005X sample buffer without DMSO (1.005X TRIS/EDTA/NaCl/p20/DTT; diluted from a 10X stock) to a final compound concentration of 10 µM and 0.5 % DMSO. m7GTP (Sigma Aldrich) and SAV-7160 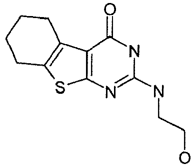 were used as references and chip stability controls at a concentration of 4 mM and 10 µM, respectively. Stock solutions of each reference compound were made and aliquots were stored at -20°C.

For buffer bulk effects (matrix) was accounted by reducing the response obtained for the reference flow cell Fc1 from the active flow cell Fc2 resulting in relative response units (RU) reflecting binding of the compounds to the ligand. Organic solvents such as DMSO in the buffer cause high bulk effects which differ in the reference flow cell and the active flow cell due to ligand immobilization. To account for these differences, a calibration curve was established. Eight DMSO concentrations ranging from 0.1 % to 1.5 % in buffer were measured and a linear calibration curve was calculated by plotting Fc2-Fc1 vs. Fc1. The relative response of each sample was then corrected by the solvent factor given by the respective Fc1 signal on the calibration curve and the corresponding Fc2-Fc1 difference. To account for the different size of the compounds, the buffer and solvent corrected response units were normalized to the molecular weight.

Affinity constants (KD values) were determined by measuring the binding affinity of the analyte to the ligand over a concentration range ranging from 200 µM to 1 nM. The KD value is that concentration at which 50% of the binding sites are saturated and was calculated using a linear curve fit model.

### Compounds having the general formula (I)

### Key Intermediate I

### O, N-Dibenzyl hydroxylamine hydrochloride

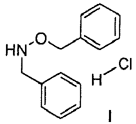

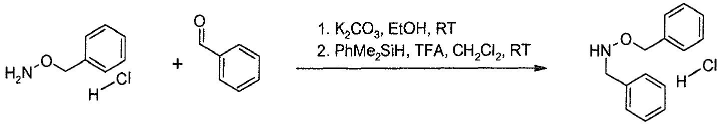

To a suspension of O-benzyl hydroxylamine hydrochloride (1.2 g, 10 mmol, 1 eq) in absolute ethanol (16 mL) was added potassium carbonate (1.5 g, 11 mmol, 1.1 eq) and benzaldehyde (1.0 mL, 10 mmol, 1 eq). The mixture was stirred at room temperature for 5 h and then was poured into water (50 mL). The mixture was extracted with ethyl acetate (3 x 50 mL). The organic layers were dried over magnesium sulfate, filtered and evaporated in vacuo. The residue was dissolved in dichloromethane (21 mL) and cooled down to 0 °C. To this solution were added drop wise under argon dimethylphenylsilane (2.3 mL, 14.3 mmol, 1.4 eq) and trifluoroacetic acid (2.6 mL, 35.6 mmol, 3.5 eq). The reaction mixture was stirred at room temperature for 16 h. The solvents were removed in vacuo and a 2N solution of hydrochloric acid (5 mL) was added into the residue diluted in dichloromethane (5 mL). The precipitate was filtered, washed with diethyl ether and dried in vacuo to afford the expected compound as a white powder (966 mg, 48 % yield).

### Key Intermediate II

### 4-Amino-pyridine-2-carboxylic acid methyl ester

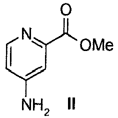

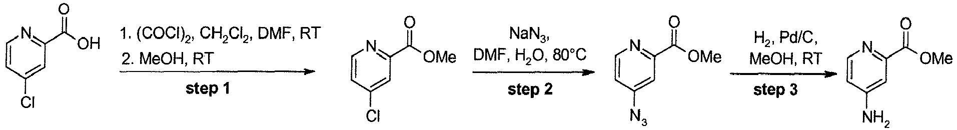

### Step 1:

Oxalyl chloride (6.7 mL, 76.8 mmol, 1.2 eq) was added to a solution of 4-chloro-pyridine-2-carboxylic acid (10.0 g, 63.4 mmol, 1 eq) in dichloromethane (270 mL). The solution was cooled down to 0 °C and dimethylformamide (1.1 mL) was added drop wise. The mixture was stirred at room temperature for 1.5 h and was evaporated to dryness. The orange residue was diluted in methanol (110 mL) and the mixture was stirred at room temperature for 30 min and evaporated to dryness. A 5% solution of sodium bicarbonate (50 mL) was poured on the residue and the aqueous phase was extracted with ethyl acetate (2 x 40 mL). The organic layers were washed with brine (3 x 20 mL), dried over magnesium sulfate, filtered and evaporated to afford 4-chloro-pyridine-2-carboxylic acid methyl ester as a beige powder (10.0 g, 92 % yield).

### Step 2:

4-Chloro-pyridine-2-carboxylic acid methyl ester (13.7 g, 79.9 mmol, 1 eq) was solubilized in a mixture of dimethylformamide (120 mL) and water (6 mL). Sodium azide was added (6.2 g, 95.9 mmol, 1.2 eq) and the mixture was heated at 80 °C during 24 h. After cooling down, the mixture was diluted with ethyl acetate (40 mL) and washed with water (30 mL) and brine (30 mL). The organic layers were dried over magnesium sulfate, filtered and evaporated in vacuo. At this stage, the reaction was not complete (15% of starting material detected) and the same procedure was run again with new reagents at 80 °C during 24h. After the same treatment, evaporation of the organic layers afforded 4-azido-pyridine-2-carboxylic acid methyl ester as an orange oil which crystallizes (10.2 g, 72 % yield).

### Step 3:

4-Azido-pyridine-2-carboxylic acid methyl ester (3.9 g, 22 mmol, 1 eq) was solubilized in methanol (50 mL) and palladium 10% w on carbon (400 mg) was added. The mixture was stirred at room temperature over 4 bars pressure of hydrogen until completion of the reaction. The mixture was then filtered over a short pad of celite, and rinsed with methanol to afford the expected compound as a yellow powder (3.0 g, 90 % yield).

### Key Intermediates III and IV

### 4-Bromo-pyridine-2-carboxylic acid benzyl-(tetrahydro-pyran-2-yloxy)-amide and 4-Amino-pyridine-2-carboxylic acid benzyl-(tetrahydro-pyran-2-yloxy)-amide

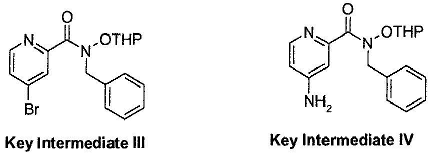

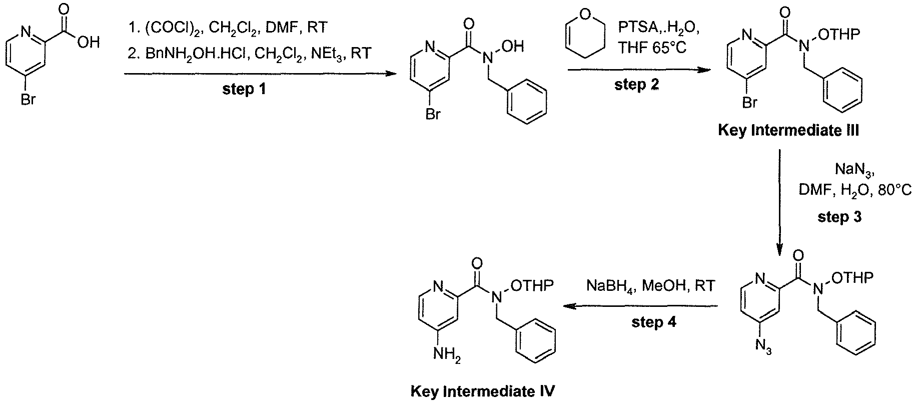

### Step 1:

Oxalyl chloride (5.1 mL, 58.6 mmol, 1.3 eq) was added to a solution of 4-bromo-pyridine-2-carboxylic acid (9.1 g, 45.0 mmol, 1 eq) in dichloromethane (250 mL). The solution was cooled down to 0 °C and dimethylformamide (0.6 mL) was added drop wise. The mixture was stirred at room temperature for 1.5 h and was evaporated to dryness. The residue was diluted in dichloromethane (250 mL) and N-benzylhydroxylamine hydrochloride (10.8 g, 67.5 mmol, 1.5 eq) was added. Triethylamine (18.8 mL, 135 mmol, 3 eq) was added drop wise at 0 °C and the mixture was stirred at room temperature for 18 h. The solution was then poured on a saturated solution of sodium bicarbonate (50 mL) and extracted with dichloromethane (3 x 50 mL). The organic layers were dried over magnesium sulfate, filtered and evaporated. The crude residue was purified by flash chromatography using cyclohexane and ethyl acetate (100/0 to 70/30) to afford 4-bromo-pyridine-2-carboxylic acid benzyl-hydroxy-amide as an orange oil (8.0 g, 58 % yield).

### Step 2:

Dihydropyrane (9.4 mL, 104 mmol, 4 eq) and paratoluene sulfonic acid (99 mg, 0.52 mmol, 0.02 eq) were added to a solution of 4-bromo-pyridine-2-carboxylic acid benzyl-hydroxy-amide (8.0 g, 26 mmol, 1 eq) in tetrahydrofuran (200 mL). The mixture was heated at 65 °C for 48 h. After cooling, the mixture was poured on a saturated solution of sodium bicarbonate (60 mL) and extracted with ethyl acetate (3 x 40 mL). The organic layers were dried over magnesium sulfate, filtered and evaporated. The crude residue was purified by flash chromatography using cyclohexane and ethyl acetate (100/0 to 80/20) to afford Key **Intermediate II** as a pale yellow oil which crystallised (7.8 g, 76 % yield).

### Step 3:

4-Bromo-pyridine-2-carboxylic acid benzyl-(tetrahydro-pyran-2-yloxy)-amide (5.0 g, 12.8 mmol, 1 eq) was solubilized in a mixture of dimethylformamide (41 mL) and water (3 mL). Sodium azide was added (997 mg, 15.3 mmol, 1.2 eq) and the mixture was heated at 80 °C during 24h. After cooling down, the mixture was diluted with ethyl acetate (40 mL) and washed with water (30 mL) and brine (30 mL). The organic layers were dried over magnesium sulfate, filtered and evaporated in vacuo. At this stage, the reaction was not complete and the same procedure was run again with new reagents at 80 °C during 24h. After the same treatment, the crude residue was purified by flash chromatography using cyclohexane and ethyl acetate (100/0 to 60/40) to afford 4-azido-pyridine-2-carboxylic acid benzyl-(tetrahydro-pyran-2-yloxy)-amide (2.8 g, 61 % yield).

### Step 4:

To a solution of 4-azido-pyridine-2-carboxylic acid benzyl-(tetrahydro-pyran-2-yloxy)-amide (2.5 g, 7.1 mmol, 1 eq) in methanol (55 mL) was added sodium borohydride (296 mg, 37.8 mmol, 1.1 eq) and the mixture was stirred at room temperature during 1 h. Water (20 mL) was then added and the mixture was evaporated to dryness. The residue was diluted with ethyl acetate (20 mL) and the organic layer was washed with water, dried over magnesium sulfate, filtered and evaporated in vacuo. The crude residue was purified by flash chromatography using ethyl acetate and methanol (100/0 to 90/10) to afford **Key Intermediate IV** as a colorless oil (883 mg, 38 % yield).

### Key Intermediates V and VI

### 5-(4,4,5,5-Tetramethyl-[1,3,2]dioxaborolan-2-yl)-3,4-dihydro-2H-pyridine-1-carboxylic acid tert-butyl ester and 5-(4,4,5,5-Tetramethyl-[1,3,2]dioxaborolan-2-yl)-3,6-dihydro-2H-pyridine-1-carboxylic acid tert-butyl ester

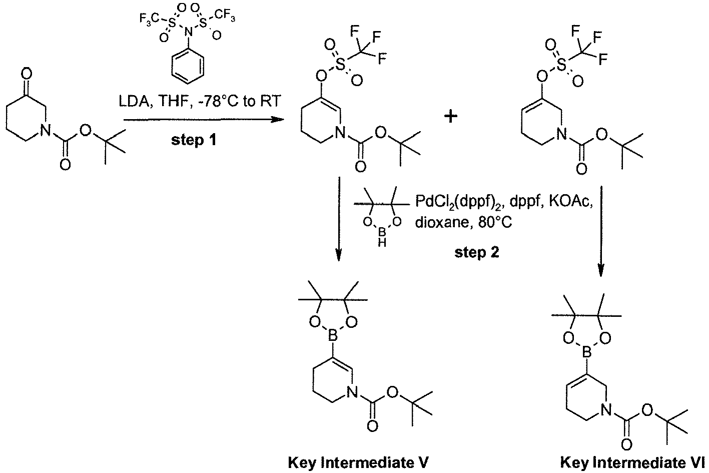

### Step 1:

At -78 °C, to a solution of lithium diisopropylamide 1.5 M in cyclohexane (8 mL, 12 mmol, 1.2 eq) in tetrahydrofuran (8 mL) was added drop wise a solution of 3-oxo-piperidine-1-carboxylic acid tert-butyl ester (2.0 g, 10 mmol, 1 eq) in tetrahydrofuran (8 mL). The mixture was stirred at -78 °C for 1 h and a solution of N-phenyl bis trifluoromethanesulfonamide (3.9 g, 11 mmol, 1.1 eq) in tetrahydrofuran (8 mL) was added. The mixture was stirred at -78 °C for 2 h and then was allowed to warm up to room temperature and stirred 18 additional hours at room temperature. The mixture was evaporated to dryness and the residue was taken with diethyl ether (20 mL). The organic layer was washed with water (10 mL), a 2 M solution of sodium hydroxide (3 x 10 mL), water (10 mL) and brine (10 mL). The organic layers were dried over magnesium sulfate, filtered and evaporated in vacuo. The crude residue was purified by flash chromatography using cyclohexane and dichloromethane (100/0 to 0/100) to afford separately 5-trifluoromethanesulfonyloxy-3,4-dihydro-2H-pyridine-1-carboxylic acid tert-butyl ester (980 mg, 29 % yield) and 5-trifluoromethanesulfonyloxy-3,6-dihydro-2H-pyridine-1-carboxylic acid tert-butyl ester (340 mg, 10 % yield).

### Step 2:

To a degassed solution of 5-trifluoromethanesulfonyloxy-dihydro-2H-pyridine-1-carboxylic acid tert-butyl ester (340 mg, 1.0 mmol, 1 eq) in dioxane (10 mL) was added bis-(pinacolato)-diboron (287 mg, 1.1 mmol, 1.1 eq), potassium acetate (302 mg, 3.0 mmol, 3 eq), 1,1'-bis(diphenylphosphino)ferrocene (17 mg, 0.03 mmol, 0.03 eq) and dichloro[1,1'-bis(diphenylphosphino)ferrocene]palladium) (23 mg, 0.03 mmol, 0.03 eq) were added. The mixture was stirred at 80 °C for 18 h. After cooling down, the mixture was filtered and the filtrate was concentrated and purified by flash chromatography using cyclohexane and ethyl acetate (100/0 to 96/4) to afford the corresponding boronic ester (225 mg, 70 % yield).

### General Procedure A

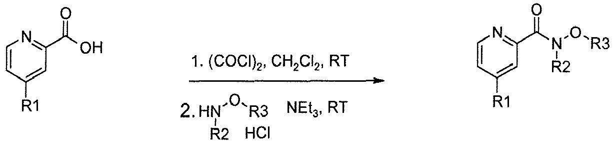

At 0 °C, to a solution of pyridinyl-2-carboxylic acid hydrochloride (1.0 mmol, 1 eq) in dichloromethane (8 mL) was added one drop of dimethylformamide and oxalyl chloride (1.3 mmol, 1.3 eq). The mixture was stirred at room temperature for 30 min and was evaporated to dryness. The residue was then solubilized in dichloromethane (8 mL) and cooled to 0 °C. Triethylamine (3.1 mmol, 3 eq) and hydroxylamine hydrochloride (2.1 mmol, 2 eq) were added drop wise and the mixture was stirred at room temperature for 20 h. The solvents were then evaporated and the crude residue was purified by flash chromatography using dichloromethane and methanol (100/0 to 80/20) to afford the expected compound.

### Example 1:

### 3,4,5,6-Tetrahydro-2H-[1,4']bipyridinyl-2'-carboxylic acid hydroxyamide chlorhydrate

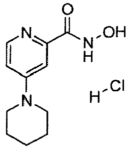

The expected compound was obtained according to **general procedure A** using 3,4,5,6-tetrahydro-2H-[1,4']bipyridinyl-2'-carboxylic acid hydrochloride and hydroxylamine hydrochloride. The expected compound was isolated as a white powder (6 % yield).
MS: 222.1
Mp: 200 °C - 202 °C

### Example 2:

### 3,4,5,6-Tetrahydro-2H-[1,4']bipyridinyl-2'-carboxylic acid (3,4,5,6-tetrahydro-2H-[1,4']bipyridinyl-2'-carbonyloxy)-amide

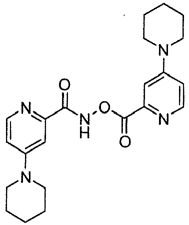

This compound was isolated as a by-product of **example 1** and obtained as a white powder (4 % yield).
MS: 410.2
Mp: 210 °C - 215 °C

### Example 3:

### 4-Morpholin-4-yl-pyridine-2-carboxylic acid ethoxy-amide chlorhydrate

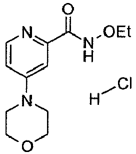

This compound was obtained according to **general procedure A** using 4-morpholin-4-yl-pyridine-2-carboxylic acid hydrochloride and O-ethyl hydroxylamine hydrochloride. The expected compound was isolated as a white powder (42 % yield).
MS: 252.1
Mp: 200 °C - 202 °C

### Example 4:

### 5-Pyrrolidin-1-yl-pyridine-2-carboxylic acid benzyl-hydroxy-amide

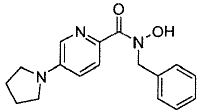

This compound was obtained according to general procedure A using 5-pyrrolidin-1-yl-pyridine-2-carboxylic acid and N-benzyl hydroxylamine hydrochloride. The expected compound was isolated as a white powder (32 % yield).
MS: 298.1
Mp: 115 °C -120 °C

### Example 5:

### 3,4,5,6-Tetrahydro-2H-[1,4']bipyridinyl-2'-carboxylic acid benzyl-hydroxy-amide

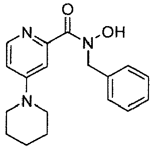

This compound was obtained according to **general procedure A** using 3,4,5,6-tetrahydro-2H-[1,4']bipyridinyl-2'-carboxylic acid hydrochloride and N-benzyl hydroxylamine hydrochloride. The expected compound was isolated as a yellow oil (15 % yield).
MS: 312.2

### Example 6:

### Isoquinoline-3-carboxylic acid hydroxy-methyl-amide

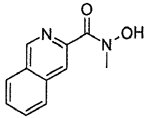

This compound was obtained according to **general procedure A** using isoquinoline-3-carboxylic acid and N-methyl hydroxylamine hydrochloride. The expected compound was isolated as a white powder (43 % yield).
MS: 203.0
Mp: 110 °C-115 °C

### Example 7:

### Isoquinoline-3-carboxylic acid benzyl-hydroxy-amide

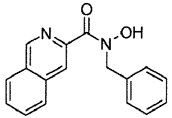

This compound was obtained according to **general procedure A** using isoquinoline-3-carboxylic acid and N-benzyl hydroxylamine hydrochloride. The expected compound was isolated as a white powder (19 % yield).
MS: 279.1
Mp: 120 °C - 125 °C

### General Procedure B

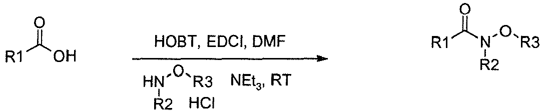

To a solution of carboxylic acid (3.6 mmol, 1 eq) in dimethylformamide (30 mL) were added HOBT (7.2 mmol, 2 eq), EDCI (7.2 mmol, 2 eq) and then hydroxylamine hydrochloride (7.2 mmol, 2 eq) and triethylamine (10.8 mmol, 3 eq). The mixture was stirred at room temperature for 20 h. Then the mixture was poured on brine solution (20 mL) and extracted with ethyl acetate (3 x 20 mL). The organic layers were dried over magnesium sulfate, filtered and evaporated in vacuo. The crude residue was purified by flash chromatography using dichloromethane and methanol (100/0 to 85/15) to afford the expected compound.

### Example 8:

### 4-Amino-pyridine-2-carboxylic acid ethoxy-amide chlorhydrate

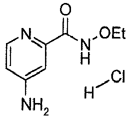

This compound was obtained according to **general procedure B** using 4-amino-pyridine-2-carboxylic acid and O-ethyl hydroxylamine hydrochloride. The expected compound was isolated as a colorless oil (3 % yield).
MS: 182.0
Mp: 114 °C - 120 °C

### Example 9:

### Pyridine-2-carboxylic acid ethoxy-amide

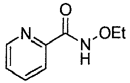

This compound was obtained according to **general procedure B** using pyridine-2-carboxylic acid and O-ethyl hydroxylamine hydrochloride. The expected compound was isolated as a colorless oil (63 % yield).
MS: 167.1

### Example 10:

### 6-Methyl-pyridine-2-carboxylic acid benzyloxy-amide

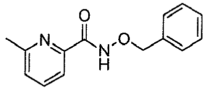

This compound was obtained according to **general procedure B** using 6-methyl-pyridine-2-carboxylic acid and O-benzyl hydroxylamine hydrochloride. The expected compound was isolated as a white powder (71 % yield).
MS: 243.1
Mp: 75 °C - 80 °C

### Example 11:

### 6-Methyl-pyridine-2-carboxylic acid ethoxy-amide

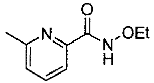

This compound was obtained according **general procedure B** using 6-methyl-pyridine-2-carboxylic acid and O-ethyl hydroxylamine hydrochloride. The expected compound was isolated as a colorless oil (83 % yield).
MS: 181.0

### Example 12:

### 5-Phenyl-pyridine-2-carboxylic acid benzyloxy-amide

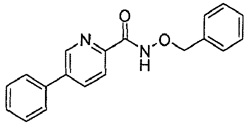

This compound was obtained according to **general procedure B** using 5-phenyl-pyridine-2-carboxylic acid and O-benzyl hydroxylamine hydrochloride. The expected compound was isolated as a white powder (79 % yield).
MS: 305.1
Mp: 155 °C - 160 °C

### Example 13:

### 5-Phenyl-pyridine-2-carboxylic acid ethoxy-amide

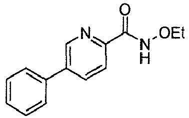

This compound was obtained according to **general procedure B** using 5-phenyl-pyridine-2-carboxylic acid and O-ethyl hydroxylamine hydrochloride. The expected compound was isolated as a white powder (64 % yield).
MS: 243.1
Mp: 100 °C - 105 °C

### Example 14:

### 3,4,5,6-Tetrahydro-2H-[1,4']bipyridinyl-2'-carboxylic acid benzyloxy-amide

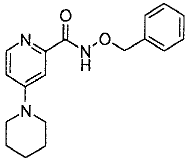

This compound was obtained according to **general procedure B** using 3,4,5,6-tetrahydro-2H-[1,4']bipyridinyl-2'-carboxylic acid hydrochloride and O-benzyl hydroxylamine hydrochloride. The expected compound was isolated as a white powder (26 % yield).
MS: 312.2
Mp: 135 °C - 140 °C

### Example 15:

### 5-Pyrrolidin-1-yl-pyridine-2-carboxylic acid benzyloxy-amide

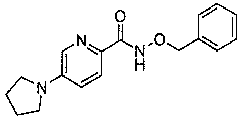

This compound was obtained according to **general procedure B** using 5-pyrrolidin-1-yl-pyridine-2-carboxylic acid and O-benzyl hydroxylamine hydrochloride. The expected compound was isolated as a white powder (54 % yield).
MS: 298.1
Mp: 165 °C - 170 °C

### Example 16:

### Isoquinoline-3-carboxylic acid benzyloxy-amide

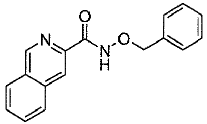

This compound was obtained according to **general procedure B** using isoquinoline-3-carboxylic acid and O-benzyl hydroxylamine hydrochloride. The expected compound was isolated as a white powder (77 % yield).
MS: 279.1
Mp: 85 °C - 90 °C

### Example 17:

### 5-Pyrrolidin-1-yl-pyridine-2-carboxylic acid benzyl-benzyloxy-amide

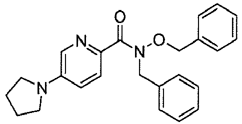

This compound was obtained according to **general procedure B** using 5-pyrrolidin-1-yl-pyridine-2-carboxylic acid and O, N-dibenzyl hydroxylamine hydrochloride **(Key Intermediate I).** The expected compound was isolated as a white powder (12 % yield).
MS: 388.2
Mp: 95 °C - 100 °C

### Example 18:

### Isoquinoline-3-carboxylic acid benzyl-benzyloxy-amide

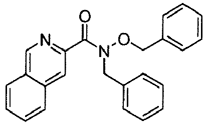

This compound was obtained according to **general procedure B** using isoquinoline-3-carboxylic acid and O, N-dibenzyl hydroxylamine hydrochloride **(Key Intermediate I).** The expected compound was isolated as a white powder (36 % yield).
MS: 369.2
Mp: 70 °C - 75 °C

### Example 19:

### Isoquinoline-3-carboxylic acid hydroxyamide

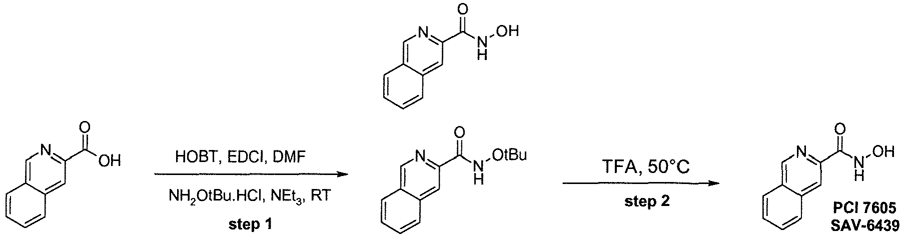

### Step 1:

Isoquinoline-3-carboxylic acid tert-butoxy-amide was obtained according to **general procedure B** using isoquinoline-3-carboxylic acid and O-tert-butyl hydroxylamine hydrochloride. The expected compound was isolated as a pale yellow powder (46 % yield).

### Step 2:

Isoquinoline-3-carboxylic acid tert-butoxy-amide (195 mg, 1 eq) and trifluoroacetic acid (4 mL) were heated at 50 °C during 20 h. The mixture was then evaporated to dryness. The residue was diluted in ethyl acetate (10 mL) and triethylamine (3 mL) was added. The mixture was absorbed on silica gel to be purified by flash chromatography using cyclohexane and ethyl acetate (100/0 to 0/100) to afford the expected compound as a pale pink powder (70 mg, 65 % yield).
MS: 189.0
Mp: 160 °C - 165 °C

### Example 20:

### 5-Pyrrolidin-1-yl-pyridine-2-carboxylic acid hydroxyamide

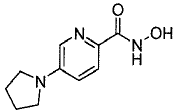

This compound was obtained according to the procedure of **example 19** using 5-pyrrolidin-1-yl-pyridine-2-carboxylic acid. The expected compound was isolated as a white powder.
MS: 208.0
Mp: 220 °C - 225 °C

### Example 21:

### 5-(3-Isopropyl-phenyl)-pyridine-2-carboxylic acid ethoxy-amide

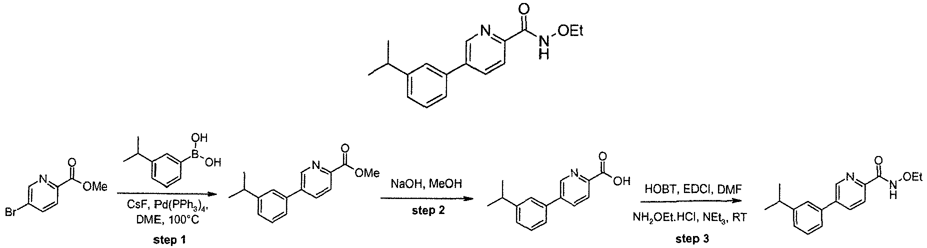

### Step 1:

To a solution of 5-bromo-pyridine-2-carboxylic acid methyl ester (500 mg, 2.3 mmol, 1 eq) in dimethoxyethane (6 mL) was added 3-isopropylphenylboronic acid (495 mg, 3 mmol, 1.3 eq) and cesium fluoride (1.05 g, 6.9 mmol, 3 eq). The mixture was degassed for 15 min and tetrakis(triphenylphosphine)palladium (133 mg, 0.12 mmol, 0.05 eq) was added. The mixture was heated at 100 °C for 15 min under microwave irradiation. After cooling, the mixture was poured on water (10 mL) and extracted with ethyl acetate (3 x 20 mL). The organic layers were dried over magnesium sulphate, filtered and evaporated to dryness. The crude residue was purified by flash chromatography using cyclohexane and ethyl acetate (100/0 to 0/100) to afford 5-(3-isopropyl-phenyl)-pyridine-2-carboxylic acid methyl ester as a colorless oil (380 mg, 64 % yield).

### Step 2:

5-(3-Isopropyl-phenyl)-pyridine-2-carboxylic acid methyl ester (380 mg, 1.5 mmol, 1 eq) diluted in methanol (6 mL) and a 5 N solution of sodium hydroxide (0.5 mL) were heated at 80 °C for 20 h in a sealed tube. After cooling, the mixture was evaporated and the residue was diluted in water (6 mL) and extracted with ethyl acetate (3 x 10 mL). The aqueous layer was then acidified with a 1 N solution of hydrochloric acid and extracted with ethyl acetate (3 x 20 mL). The organic layers were dried over magnesium sulphate, filtered and evaporated to dryness to afford 5-(3-isopropyl-phenyl)-pyridine-2-carboxylic acid as a colorless oil (230 mg, 64 % yield).

### Step 3:

This compound was obtained according to **general procedure B** using 5-(3-isopropylphenyl)-pyridine-2-carboxylic acid and O-ethyl hydroxylamine hydrochloride. The expected compound was isolated as a colorless oil (60 % yield).
MS: 285.2

### Example 22:

### 5-m-Tolyl-pyridine-2-carboxylic acid benzyl-hydroxy-amide

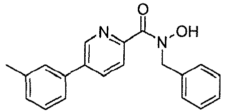

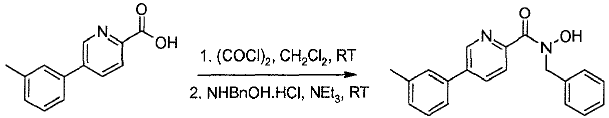

This compound was obtained according to **general procedure A** using 5-m-Tolyl-pyridine-2-carboxylic acid (obtained according the procedure of **example 21, steps 1 and 2**) and N-benzyl hydroxylamine hydrochloride. The expected compound was isolated as a white powder (11 % yield).
MS: 319.1
Mp: 139 °C - 140 °C

### General Procedure C

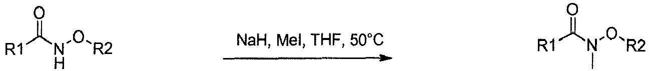

To a solution of carboxylic acid oxy-amide (0.4 mmol, 1 eq) in tetrahydrofuran (5 mL) was added sodium hydride (0.5 mmol, 1.3 eq). The mixture was stirred at room temperature during 15 min and methyl iodide (0.6 mmol, 1.5 eq) was added. The mixture was heated at 50 °C in a sealed tube during 20 h. After cooling, the mixture was poured on water (10 mL) and extracted with ethyl acetate (3 x 20 mL). The organic layers were dried over magnesium sulfate, filtered and evaporated in vacuo. The crude residue was purified by flash chromatography using dichloromethane and methanol (100/0) to (90/10) to afford the expected compound.

### Example 23:

### 6-Methyl-pyridine-2-carboxylic acid benzyloxy-methyl-amide

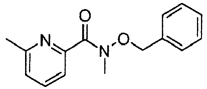

This compound was obtained according to **general procedure C** using 6-methyl-pyridine-2-carboxylic acid benzyloxy-amide (described in **example 10**).The expected compound was isolated as a colorless oil (55 % yield).
MS: 257.1

### Example 24:

### 6-Methyl-pyridine-2-carboxylic acid ethoxy-methyl-amide

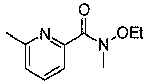

This compound was obtained according to **general procedure C** starting from 6-methylpyridine-2-carboxylic acid ethoxy-amide (described in **example 11).** The expected compound was isolated as a colorless oil (51 % yield).
MS: 195.0

### Example 25:

### 5-Phenyl-pyridine-2-carboxylic acid ethoxy-methyl-amide

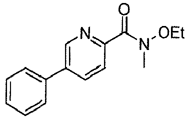

This compound was obtained according to **general procedure C** starting from 5-phenylpyridine-2-carboxylic acid ethoxy-amide (described in **example 13).** The expected compound was isolated as a white powder (41 % yield).
MS: 257.1
Mp: 70 °C - 75 °C

### Example 26:

### 5-Phenyl-pyridine-2-carboxylic acid benzyloxy-methyl-amide

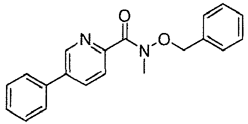

This compound was obtained according to **general procedure C** starting from 5-phenylpyridine-2-carboxylic acid benzyloxy-amide (described in **example 12).** The expected compound was isolated as a yellow oil (30 % yield).
MS: 319.1

### Example 27:

### Isoquinoline-3-carboxylic acid benzyloxy-methyl-amide

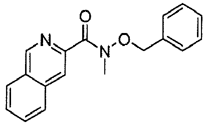

This compound was obtained according to **general procedure C** starting from isoquinoline-3-carboxylic acid benzyloxy-amide (described in **example 16).** The expected compound was isolated as a beige powder (45 % yield).
MS: 293.1
Mp: 70 °C - 75 °C

### Example 28:

### 5-(3-Isopropyl-phenyl)-pyridine-2-carboxylic acid ethoxy-methyl-amide

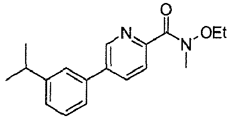

This compound was prepared according to **general procedure C** starting from 5-(3-isopropyl-phenyl)-pyridine-2-carboxylic acid ethoxy-methyl-amide (described in **example 21).** The expected compound was isolated as a colorless oil (50 % yield).
MS: 299.2

### Example 29:

### Isoquinoline-3-carboxylic acid hydroxy-phenethyl-amide

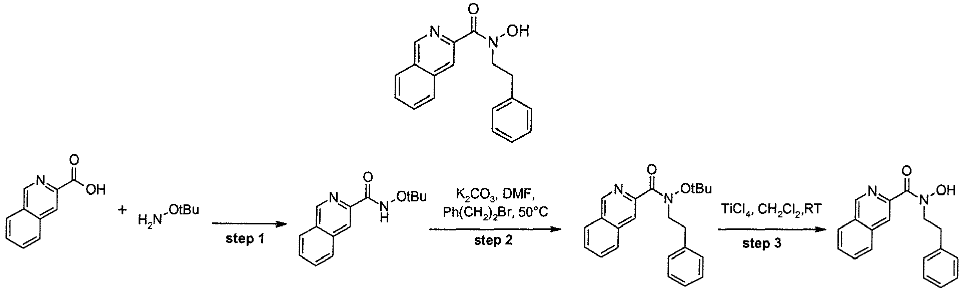

### Step 1:

Isoquinoline-3-carboxylic acid tert-butoxy-amide was prepared according to **general procedure B** using isoquinoline-3-carboxylic acid and tert-butoxy-hydroxylamide hydrochloride. The expected compound was isolated as a white powder (86 % yield).

### Step 2:

To a solution of isoquinoline-3-carboxylic acid tert-butoxy-amide (200 mg, 0.8 mmol, 1 eq) in dimethylformamide (7 mL) was added potassium carbonate (454 mg, 3.3 mmol, 4 eq) and (2-bromoethyl)benzene (220 µL, 1.6 mmol, 2 eq). The mixture was stirred at 50 °C for 20 h. After cooling, the mixture was poured on water (10 mL) and extracted with ethyl acetate (3 x 20 mL). The organic layers were dried over magnesium sulfate, filtered and evaporated in vacuo. The crude residue was purified by flash chromatography using cyclohexane and ethyl acetate (100/0 to 80/20) to afford isoquinoline-3-carboxylic acid tert-butoxy-phenethyl-amide as a colorless oil (220 mg, 77 % yield).

### Step 3:

To a solution of isoquinoline-3-carboxylic acid tert-butoxy-phenethyl-amide (220 mg, 0.63 mmol, 1 eq) in dichloromethane (10 mL) was added drop wise at 0 °C a 1M solution of titanium tetrachloride in dichloromethane (1.7 mL, 1.7 mmol, 3 eq). The mixture was stirred at room temperature for 20 h. It was then added to isopropanol (15 mL) and the resulting mixture was stirred at room temperature for 1 h and evaporated to dryness. The residue was diluted with ethyl acetate (15 mL) and washed with a saturated solution of sodium bicarbonate (3 x 20 mL). The organic layer was filtered on celite and the filtrate was evaporated to dryness. The residue was triturated in diethyl ether and filtered to afford the expected compound as a white solid (75 mg, 11 % yield).
MS: 293.2
Mp: 90 °C - 95 °C

### Example 30:

### Isoquinoline-3-carboxylic acid hydroxy-(3-phenyl-propyl)-amide

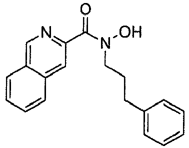

This compound was prepared according to the procedure of **example 29** starting with isoquinoline-3-carboxylic acid. The expected compound was isolated as a colorless oil. MS: 307.2

### Example 31:

### 3,4,5,6-Tetrahydro-2H-[1,4']bipyridinyl-2'-carboxylic acid hydroxy-(3-phenyl-propyl)-amide

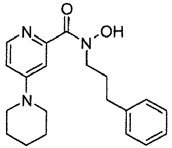

This compound was prepared according to the procedure of **example 29** starting with 3,4,5,6-tetrahydro-2H-[1,4']bipyridinyl-2'-carboxylic acid hydrochloride and using **general procedure A** for step 1 instead of **general procedure B.** The expected compound was isolated as a white powder.
MS: 340.2
Mp: 125 °C - 130 °C

### Example 32:

### 5-(3-Isopropyl-phenyl)-pyridine-2-carboxylic acid hydroxy-phenethyl-amide

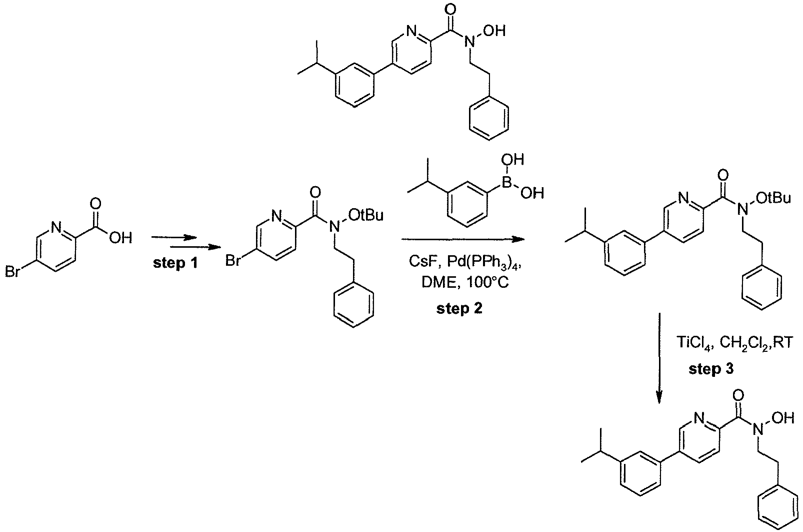

### Step 1:

5-Bromo-pyridine-2-carboxylic acid tert-butoxy-phenethyl-amide was prepared according to **example 29, steps 1 and 2** starting from 5-bromo-pyridine-2-carboxylic acid. The desired compound was obtained as a colorless oil (65 % overall yield).

### Step 2:

5-(3-lsopropyl-phenyl)-pyridine-2-carboxylic acid tert-butoxy-phenethyl-amide was prepared according to **example 21, step 1** starting from 5-bromo-pyridine-2-carboxylic acid tert-butoxy-phenethyl-amide and 3-isopropylphenylboronic acid. The expected compound was isolated as a yellow oil (86 % yield).

### Step 3:

The expected compound was prepared according to **example 29 step 3** starting from 5-(3-isopropyl-phenyl)-pyridine-2-carboxylic acid tert-butoxy-phenethyl-amide. It was isolated as a yellow powder (15 % yield).
MS: 361.2
Mp: 110 °C - 115 °C

### Example 33:

### 5-(3-Isopropyl-phenyl)-pyridine-2-carboxylic acid hydroxyamide

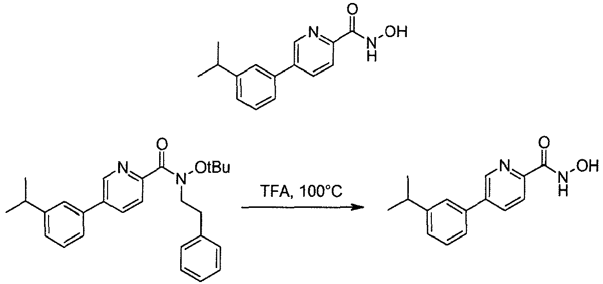

5-(3-lsopropyl-phenyl)-pyridine-2-carboxylic acid tert-butoxy-phenethyl-amide prepared according to step **example 32 steps 1 and 2** (220 mg, 0.53 mmol, 1 eq) was solubilized in trifluoroacetic acid (5 mL) and heated at 100 °C during 10 min under microwave irradiation. The mixture was then evaporated to dryness and the residue was purified by flash chromatography using cyclohexane and ethyl acetate (100/0 to 80/20) to afford the expected compound as a yellow powder (19 mg, 10 % yield).
MS: 257.1
Mp: 130 °C - 135 °C

### Example 34:

### 4-[3-(3-Chloro-phenyl)-propylamino]-pyridine-2-carboxylic acid ethoxy-amide

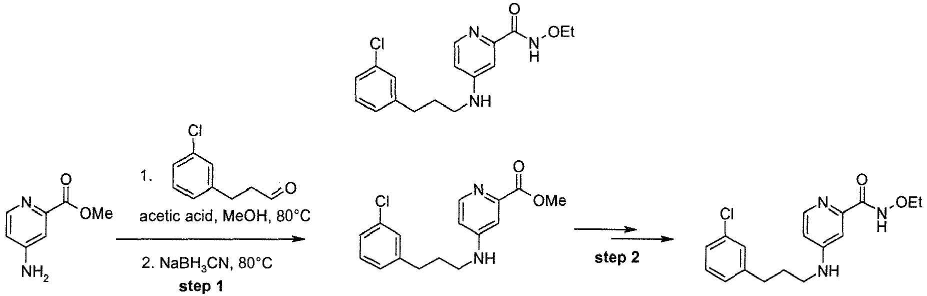

### Step 1:

In a sealed tube, 4-amino-pyridine-2-carboxylic acid methyl ester (200 mg, 1.3 mmol, 1 eq) and 3-(3-chloro-phenyl)-propionaldehyde (0.4 mL, 2.6 mmol, 2 eq) were solubilized in acetic acid (190 µL, 3.3 mmol, 2.5 eq) and anhydrous methanol (7 mL) in the presence of molecular sieves. The mixture was heated at 80 °C for 20 h. After cooling, sodium cyanoborohydride (123 mg, 1.9 mmol, 1.5 eq) was added and the mixture was heated at 80 °C for 4 h. After cooling, the mixture was poured on a saturated solution of sodium bicarbonate (10 mL) and extracted with ethyl acetate (3 x 20 mL). The organic layers were dried over magnesium sulfate, filtered and evaporated in vacuo. The crude residue was purified by flash chromatography using dichloromethane and methanol (100/0 to 90/10) to afford the expected compound as a colorless oil (144 mg, 36 % yield).

### Step 2:

The expected compound was prepared according to **example 21, steps 2 and 3** starting with 4-[3-(3-chloro-phenyl)-propylamino]-pyridine-2-carboxylic acid methyl ester. The expected compound was isolated as a white powder.
MS: 334.2
Mp: 100 °C - 105 °C

### Example 35:

### 4-[(1-Benzyl-piperidin-4-ylmethyl)-amino]-pyridine-2-carboxylic acid ethoxy-amide chlorhydrate

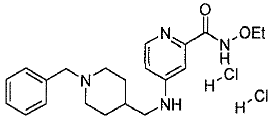

This compound was prepared according to the procedure of **example 34** starting from 4-amino-pyridine-2-carboxylic acid methyl ester and 1-benzyl-piperidine-4-carbaldehyde. The expected compound was isolated as a white powder.
MS: 369.3
Mp: 125 °C-130 °C

### Example 36:

### 4-(3-Benzyloxy-benzylamino)-pyridine-2-carboxylic acid ethoxy-amide hydrochloride

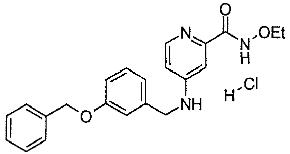

This compound was prepared according to the procedure of **example 34** starting from 4-amino-pyridine-2-carboxylic acid methyl ester and 3-benzyloxy-benzaldehyde. The expected compound was isolated as a pink powder.
MS: 378.2
Mp: 70 °C -75 °C

### Example 37:

### 5-(3-{[Methyl-(3-phenyl-propyl)-amino]-methyl}-phenyl)-pyridine-2-carboxylic acid ethoxy-amide chlorhydrate

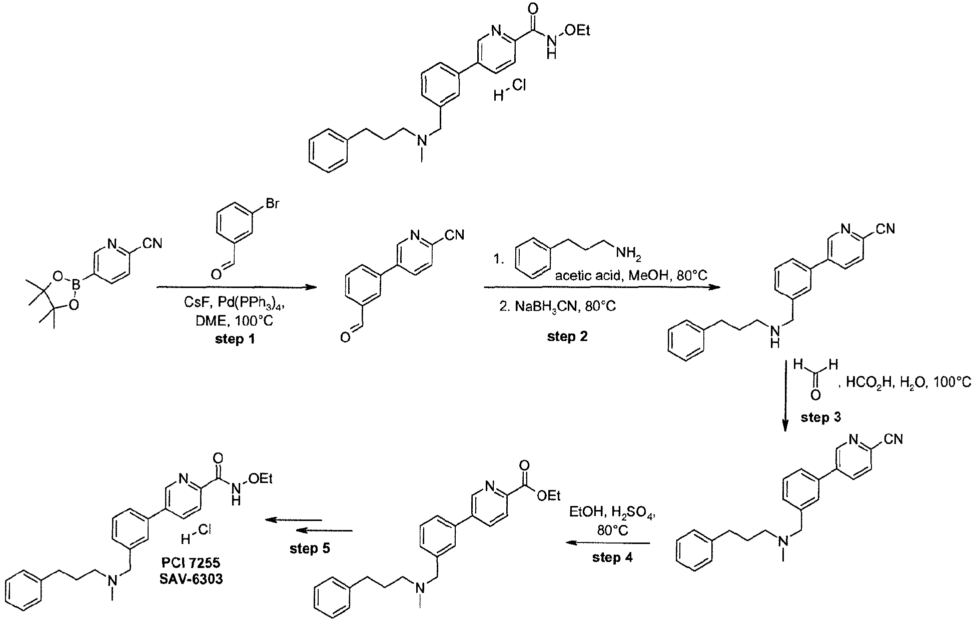

### Step 1:

5-(3-Formyl-phenyl)-pyridine-2-carbonitrile was prepared according to **example 21 step 1** starting from 3-bromo-benzaldehyde and 5-(4,4,5,5-tetramethyl-[1,3,2]dioxaborolan-2-yl)-pyridine-2-carbonitrile. The expected compound was isolated as a white powder (88 % yield).

### Step 2:

5-{3-[(3-Phenyl-propylamino)-methyl]-phenyl}-pyridine-2-carbonitrile was prepared according to **example 34, step 1** starting from 5-(3-formyl-phenyl)-pyridine-2-carbonitrile and 3-phenyl-propylamine. The expected compound was isolated as a colorless oil (quant. yield).

### Step 3:

5-{3-[(3-Phenyl-propylamino)-methyl]-phenyl}-pyridine-2-carbonitrile (384 mg, 1.1 mmol, 1 eq), formaldehyde 37 % in water (210 µL), formic acid (97 µL, 2.6 mmol, 2.4 eq) were solubilized in water (5 mL) and heated at 100 °C for 20 h. After cooling, the mixture was basified with a 5 N solution of sodium hydroxide, poured on water (10 mL) and extracted with ethyl acetate (3 x 20 mL). The organic layer was dried over magnesium sulfate, filtered and evaporated in vacuo. The crude residue was purified by flash chromatography using cyclohexane and ethyl acetate (100/0 to 80/20) to afford 5-(3-{[methyl-(3-phenyl-propyl)-amino]-methyl}-phenyl)-pyridine-2-carbonitrile as a colorless oil (quant. yield).

### Step 4:

In a sealed tube, 5-(3-{[methyl-(3-phenyl-propyl)-amino]-methyl}-phenyl)-pyridine-2-carbonitrile (365 mg, 1.1 mmol, 1 eq), sulfuric acid (5 mL) and ethanol (5 mL) were heated at 80 °C during 48 h. After cooling, the mixture was evaporated to dryness. The residue was taken in ethyl acetate (10 mL) and washed with a saturated solution of sodium bicarbonate (3 x 10 mL). The organic layer was dried over magnesium sulfate, filtered and evaporated in vacuo to afford 5-(3-{[methyl-(3-phenyl-propyl)-amino]-methyl}-phenyl)-pyridine-2-carboxylic acid ethyl ester as a yellow oil (224 mg, quant. yield).

### Step 5:

This compound was prepared according to **example 21 steps 2 and 3** starting from 5-(3-{[methyl-(3-phenyl-propyl)-amino]-methyl}-phenyl)-pyridine-2-carboxylic acid ethyl ester. The expected compound was isolated as a white powder.
MS: 404.3
Mp: 50 °C - 55 °C

### Example 38:

### 5-{3-[(Benzyl-methyl-amino)-methyl]-phenyl}-pyridine-2-carboxylic acid ethoxy-amide chlorhydrate

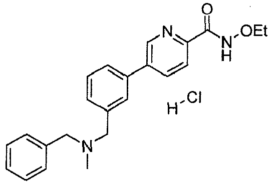

This compound was prepared according to the procedure of **example 37** starting from bromo-benzaldehyde and 5-(4,4,5,5-tetramethyl-[1,3,2]dioxaborolan-2-yl)-pyridine-2-carbonitrile and using benzylamine instead of 3-phenyl-propylamine in step 2. The expected compound was isolated as a white powder.
MS: 376.2
Mp: 85 °C-90 °C

### Example 39:

### 3-Bromo-6-hydroxy-5,6-dihydro-pyrrolo[3,4-b]pyridin-7-one

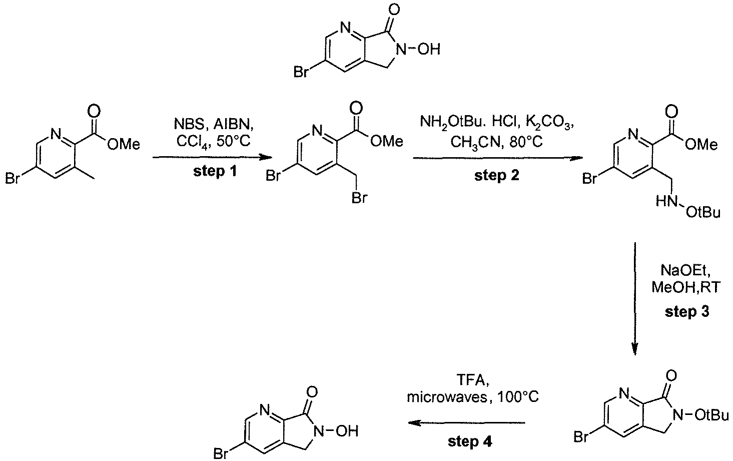

### Step 1:

To a solution of 5-bromo-3-methyl-pyridine-2-carboxylic acid methyl ester (200 mg, 0.87 mmol, 1 eq) in tetrachloromethane (10 mL) were added N-bromosuccinimide (162 mg, 0.91 mmol, 1.05 eq) and 2,2'-azobis(2-methylpropionitrile) (3 mg, 0.017 mmol, 0.02 eq). The mixture was stirred at 50 °C during 5 h. The solvent was then evaporated and the crude residue was purified by flash chromatography using cyclohexane and ethyl acetate (100/0 to 80/20). 5-Bromo-3-bromomethyl-pyridine-2-carboxylic acid methyl ester was isolated as a white powder as a 6/4 mixture with the starting material (160 mg, 39 % yield). The mixture was used in the next step.

### Step 2:

A suspension of 5-bromo-3-bromomethyl-pyridine-2-carboxylic acid methyl ester (160 mg, 0.5 mmol, 1 eq), potassium carbonate (716 mg, 5.2 mmol, 1 eq) and O-tert-butyl-hydroxylamine hydrochloride (325 mg, 2.6 mmol, 5 eq) in acetonitrile (8 mL) was heated at 80 °C during 20 h. After cooling, the mixture was filtered and washed with ethyl acetate (10 mL). The filtrate was evaporated and the crude residue was purified by flash chromatography using cyclohexane and ethyl acetate (100/0 to 70/30) to afford 5-bromo-3-(tert-butoxyamino-methyl)-pyridine-2-carboxylic acid methyl ester as a white powder (70 mg, 43 % yield).

### Step 3:

To a solution of 5-bromo-3-(tert-butoxyamino-methyl)-pyridine-2-carboxylic acid methyl ester (70 mg, 0.22 mmol, 1 eq) in methanol (2 mL) was added sodium ethoxide (30 mg, 0.44 mmol, 2 eq) freshly prepared. The mixture was stirred at room temperature for 20 h. A few drops of acetic acid and water (5 mL) were added. The precipitate was filtered and washed with water (5 mL), solubilized in methanol (10 mL) and evaporated to dryness to afford 3-bromo-6-tert-butoxy-5,6-dihydro-pyrrolo[3,4-b]pyridin-7-one as a white powder (45 mg, 72 % yield).

### Step 4:

3-Bromo-6-tert-butoxy-5,6-dihydro-pyrrolo[3,4-b]pyridin-7-one (45 mg, 0.16 mmol, 1 eq) was solubilized in trifluoroacetic acid (2 mL) and heated at 100 °C during 5 min under microwave irradiation. The mixture was then evaporated to dryness and the residue was triturated water (5 mL). The precipitate was filtered and dried in vacuo to afford the expected compound as a beige powder (22 mg, 60 % yield).
MS: 228.9
Mp: decomposes at 230 °C - 235 °C.

### Example 40:

### 6-Hydroxy-3-(3-isopropyl-phenyl)-5,6-dihydro-pyrrolo[3,4-b]pyridin-7-one

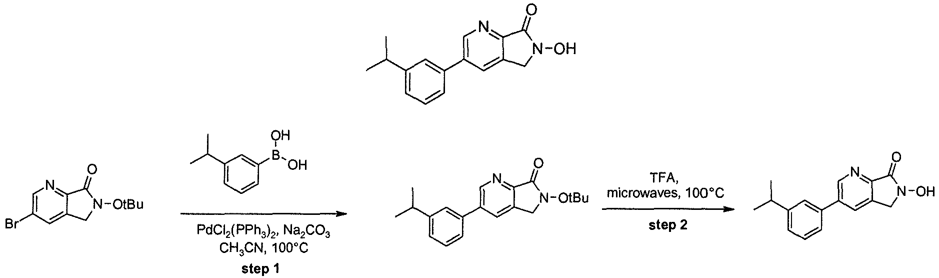

### Step 1:

To a solution of 3-bromo-6-tert-butoxy-5,6-dihydro-pyrrolo[3,4-b]pyridin-7-one described in **example 39, steps 1 to 3** (200 mg, 0.7 mmol, 1 eq) in acetonitrile (3 mL) were added 3-isopropylphenylboronic acid (150 mg, 0.9 mmol, 1.3 eq) and a 2 M solution of sodium carbonate (3 mL). The mixture was degassed for 15 min and trans-dichlorobis(triphenylphosphine)palladium (25 mg, 0.035 mmol, 0.05 eq) was added. The mixture was heated at 100 °C for 10 min under microwave irradiation. After cooling, the mixture was poured on water (10 mL) and extracted with ethyl acetate (3 x 20 mL). The organic layers were dried over magnesium sulphate, filtered and evaporated to dryness. The crude residue was purified by flash chromatography using cyclohexane and ethyl acetate (100/0 to 50/50) to afford 6-tert-butoxy-3-(3-isopropyl-phenyl)-5,6-dihydro-pyrrolo[3,4-b]pyridin-7-one as a white powder (150 mg, 66 % yield).

### Step 2:

The compound was prepared according to **example 39, step 4.** After trituration, the powder was purified by flash chromatography using dichloromethane and methanol (100/0 to 80/20) to afford the expected compound as a yellow powder (16 % yield).
MS: 269.1
Mp: decomposes at 155 °C - 160 °C.

### General procedure D

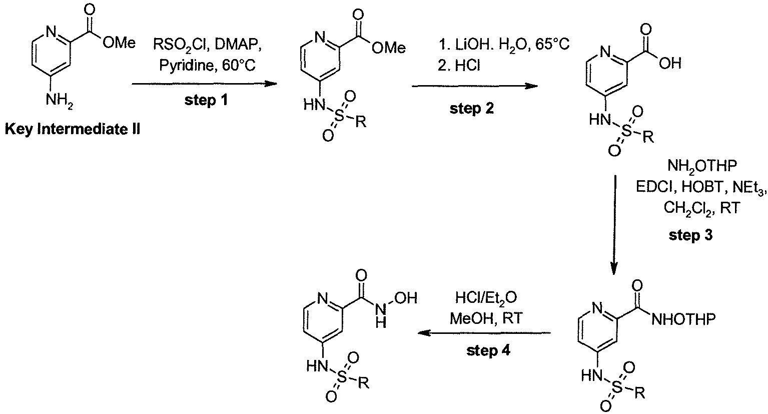

### Step 1:

4-Amino-pyridine-2-carboxylic acid methyl ester **(Key Intermediate II)** (600 mg, 3.9 mmol, 1 eq) was solubilized in pyridine (20 mL). Dimethylaminopyridine (482 mg, 3.9 mmol, 1 eq) and sulfonyl chloride (1.3 eq) were added and the mixture was stirred at 60 °C during 15 h. After cooling down, the solvent was evaporated. Water (10 mL) was added and the aqueous layer was extracted with ethyl acetate (3 x 20 mL). The organic layers were dried over magnesium sulfate, filtered and evaporated in vacuo. The crude residue was purified by flash chromatography to afford the expected compound.

### Step 2:

The sulfonylamino-pyridine-2-carboxylic acid methyl ester (1.0 g, 1 eq) was solubilized in a mixture of methanol / water (17 mL / 1.7 mL) and lithium hydroxide was added (2 eq). The mixture was heated at 65 °C during 18 h. After cooling down, a 2 M solution of hydrogen chloride in diethyl ether was added until pH = 1. The mixture was then evaporated to dryness to afford the corresponding acid with quantitative yield.

### Step 3:

To a solution of sulfonylamino-pyridine-2-carboxylic acid (800 mg, 1 eq) in dichloromethane (13 mL) were added HOBT (2 eq), EDCI (2 eq), triethylamine (3 eq) and O-(tetrahydropyran-2-yl)-hydroxylamine (2 eq). The mixture was stirred at room temperature for 18 h. The reaction was quenched with water (10 mL) and the mixture was extracted with dichloromethane (3 X 15 mL). The organic layers were dried over magnesium sulfate, filtered and evaporated in vacuo. The crude residue was purified by flash chromatography to afford sulfonylamino-pyridine-2-carboxylic acid (tetrahydro-pyran-2-yloxy)-amide.

### Step 4:

To a solution of sulfonylamino-pyridine-2-carboxylic acid (tetrahydro-pyran-2-yloxy)-amide (1 eq) in methanol (10 mL) was added a 2 M solution on hydrogen chloride in diethyl ether (2 eq). The mixture was stirred at room temperature for 1 h. The precipitate was filtered, rinsed with diethyl ether and dried in vacuo to afford sulfonylamino-pyridine-2-carboxylic acid hydroxyamide hydrochloride salt.

### Example 41:

### 4-Phenylmethanesulfonylamino-pyridine-2-carboxylic acid hydroxyamide

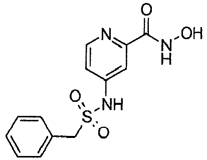

This compound was obtained according to **general procedure D** using phenylmethane-sulfonyl chloride. The expected compound was isolated as a beige powder.
MS: 308.1
Mp: 187 °C - 192 °C

### Example 42:

### 4-(4-Fluoro-phenylmethanesulfonylamino)-pyridine-2-carboxylic acid benzyl-hydroxy-amide

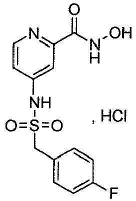

This compound was obtained according to **general procedure D** using (4-fluoro-phenyl)-methanesulfonyl chloride. The expected compound was isolated as a white powder.
MS: 326.1
Mp: 183 °C - 188 °C

### Example 43:

### 4-(3-Fluoro-phenylmethanesulfonylamino)-pyridine-2-carboxylic acid hydroxyamide hydrochloride

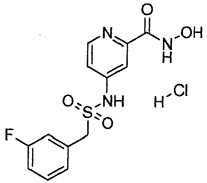

This compound was obtained according to **general procedure D** using (3-fluoro-phenyl)-methanesulfonyl chloride. The expected compound was isolated as a white powder.
MS: 326.1
Mp: 195 °C - 200 °C

### Example 44:

### 4-(2-Fluorophenylmethanesulfonylamino)-pyridine-2-carboxylic acid hydroxyamide hydrochloride

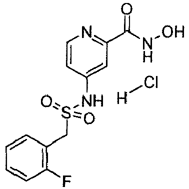

This compound was obtained according to **general procedure D** using 2-fluorophenyl-methanesulfonyl chloride. The expected compound was isolated as a white powder.
MS: 326.1
Mp: 209 °C - 216 °C

### Example 45:

### 4-(3-Chlorophenylmethanesulfonylamino)-pyridine-2-carboxylic acid hydroxyamide hydrochloride

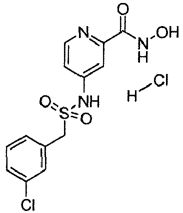

This compound was obtained according to **general procedure D** using 3-chlorophenyl-methanesulfonyl chloride. The expected compound was isolated as a white powder.
MS: 342.1
Mp: 198 °C - 204 °C

### Example 46:

### 4-(2-Chloro-phenylmethanesulfonylamino)-pyridine-2-carboxylic acid hydroxyamide hydrochloride

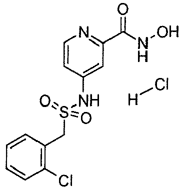

This compound was obtained according to **general procedure D** using 2-chlorophenyl-methanesulfonyl chloride. The expected compound was isolated as a white powder.
MS: 342.1
Mp: 215 °C - 220 °C

### Example 47:

### 4-(4-Chloro-phenylmethanesulfonylamino)-pyridine-2-carboxylic acid hydroxyamide hydrochloride

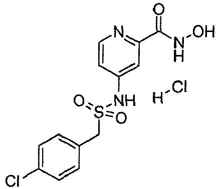

This compound was obtained according to **general procedure D** using 4-chlorophenyl-methanesulfonyl chloride. The expected compound was isolated as a beige powder.
MS: 342.1
Mp: 210 °C - 230 °C

### Example 48:

### 4-(3,5-Dichlorophenylmethanesulfonylamino)-pyridine-2-carboxylic acid hydroxy-amide hydrochloride

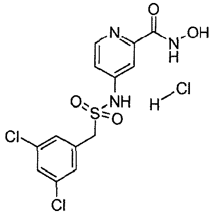

This compound was obtained according to **general procedure D** using 3,5-dichlorophenyl-methanesulfonyl chloride. The expected compound was isolated as a white powder.
MS: 376.2
Mp: 203 °C - 205 °C

### Example 49:

### 4-(3,4-Dichloro-phenylmethanesulfonylamino)-pyridine-2-carboxylic acid hydroxy-amide hydrochloride

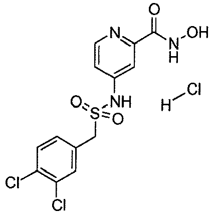

This compound was obtained according to **general procedure D** using 3,4-dichlorophenyl-methanesulfonyl chloride. The expected compound was isolated as a white powder.
MS: 376.2
Mp: 228 °C - 238 °C

### Example 50:

### 4-(2,3-Dichloro-phenylmethanesulfonylamino)-pyridine-2-carboxylic acid hydroxy-amide hydrochloride

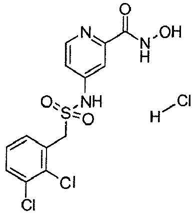

This compound was obtained according to **general procedure D** using 2,3-dichlorophenyl-methanesulfonyl chloride. The expected compound was isolated as a white powder.
MS: 376.2
Mp: 210 °C - 218 °C

### Example 51:

### 4-(3-Bromophenylmethanesulfonylamino)-pyridine-2-carboxylic acid hydroxyamide hydrochloride

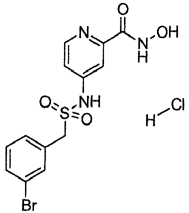

This compound was obtained according to **general procedure D** using 3-bromophenyl-methanesulfonyl chloride. The expected compound was isolated as a white powder.
MS: 386.3
Mp: 197 °C - 205 °C

### Example 52:

### 4-(3-Trifluoromethylphenylmethanesulfonylamino)-pyridine-2-carboxylic acid hydroxyamide hydrochloride

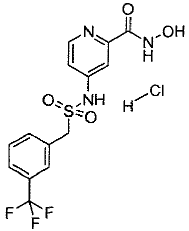

This compound was obtained according to **general procedure D** using 3-trifluoromethyl-phenylmethanesulfonyl chloride. The expected compound was isolated as a white powder.
MS: 376.1
Mp: 201 °C - 204 °C

### Example 53:

### 4-(Quinolin-8-ylmethanesulfonylamino)-pyridine-2-carboxylic acid hydroxyamide hydrochloride

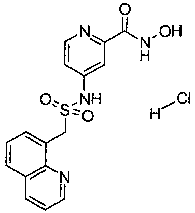

This compound was obtained according to **general procedure D** using quinolin-8-yl-methanesulfonyl chloride. The expected compound was isolated as a white powder.
MS: 359.0
Mp: 220 °C - 228 °C

### Example 54:

### 4-(Diphenylmethanesulfonylamino)-pyridine-2-carboxylic acid hydroxyamide hydrochloride

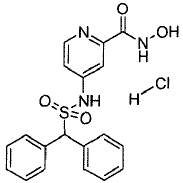

Because diphenylmethanesulfonyl chloride is not commercially available, this compound was obtained according to a **modified version of general procedure D.**

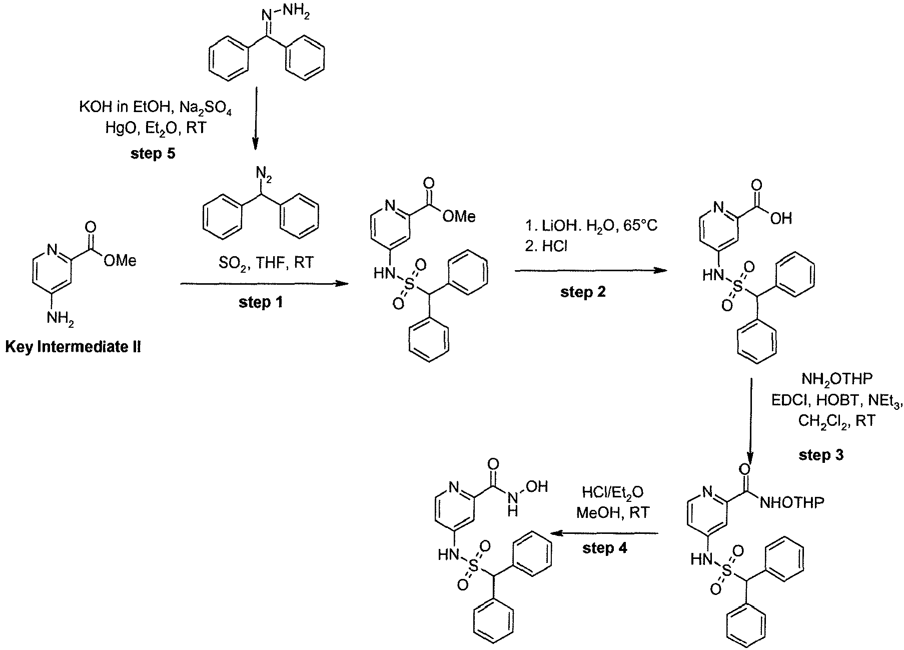

### Step 5:

To a suspension of benzophenone hydrazone (5.0 g, 25.5 mmol, 1 eq) and sodium sulfate (5.4 g, 38.2 mmol, 1.5 eq) in diethyl ether (80 mL) was added a saturated solution of potassium hydroxide in ethanol (2 mL). Mercury oxide (13.8 g, 63.7 mmol, 2.5 eq) was added and the red solution obtained was stirred at room temperature during 1.5h. The solid obtained was filtered and the filtrate was evaporated to dryness. The residue was dissolved with hexane (40 mL) and the solution was placed in the refrigerator overnight. The white crystals obtained were filtered and the filtrate was concentrated to afford diphenyldiazomethane as a partially crystallized purple oil (4.0 g, 80 % yield).

### Step 1:

At 0 °C, in a solution of 4-amino-pyridine-2-carboxylic acid methyl ester **(Key Intermediate II)** (1.2 g, 7.8 mmol, 2 eq) and diphenyldiazomethane (758 mg, 3.9 mmol, 1 eq) in tetrahydrofuran (40 mL), was bubbled sulfur dioxide until the red color disappeared. The solution was then stirred from 0 °C to room temperature for 3 days. The mixture was filtered and the filtrate was evaporated. The crude residue was purified by flash chromatography using cyclohexane and ethyl acetate (0/100 to 100/0) to afford 4-(diphenyl-methanesulfonylamino)-pyridine-2-carboxylic acid methyl ester as a pale yellow powder (665 mg, 45 % yield).

### Step 2 to Step 4:

These steps were similar to **general procedure D, steps 2 to 4.**

The final expected compound was isolated as a beige powder.
MS: 384.0
Mp: 162 °C -168 °C

### Example 55:

### 4-(Methyl-phenylmethanesulfonyl-amino)-pyridine-2-carboxylic acid hydroxyamide

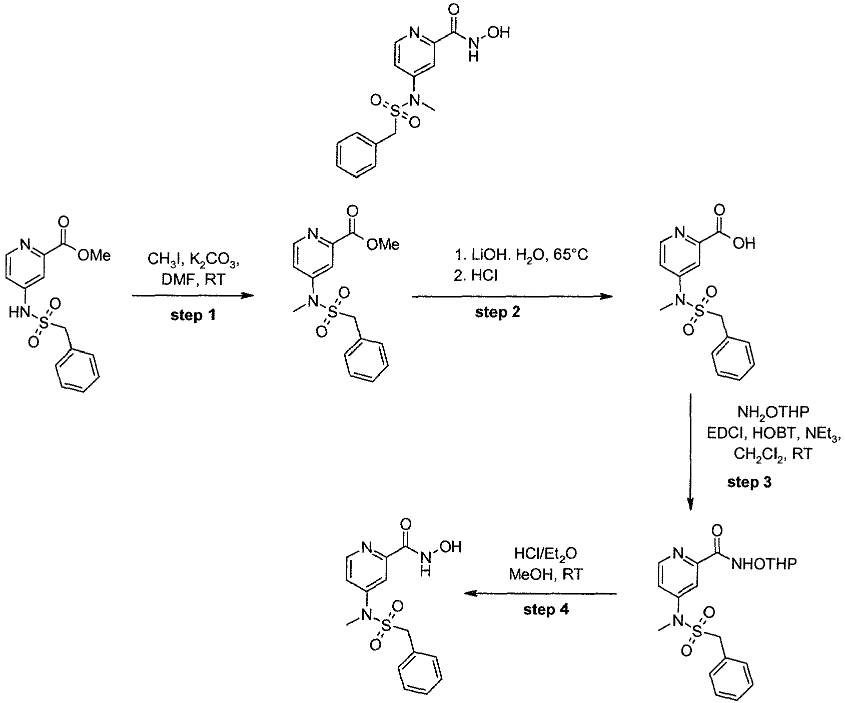

### Step 1:

To a solution of 4-phenylmethanesulfonylamino-pyridine-2-carboxylic acid methyl ester prepared according to **general procedure D step 1** (500 mg, 1.6 mmol, 1 eq) in dimethylformamide (10 mL) were added potassium carbonate (676 mg, 4.9 mmol, 3 eq) and methyl iodide (0.2 mL, 3.3 mmol, 2 eq). The mixture was stirred at room temperature for 20 h. The mixture was then poured on water (10 mL) and extracted with ethyl acetate (3 x 15 mL). The organic layers were washed with brine (3 x 15 mL), dried over magnesium sulfate, filtered and evaporated to dryness to afford 4-(methyl-phenylmethanesulfonyl-amino)-pyridine-2-carboxylic acid methyl ester as an orange oil (400 mg, 77 % yield).

### Steps 2 to 4:

These procedures were similar to **general procedure D, steps 2 to 4.**

The expected compound was isolated as a pale orange foam.
MS: 322.1

### Example 56:

### 4-Benzoylaminopyridine-2-carboxylic acid hydroxyamide

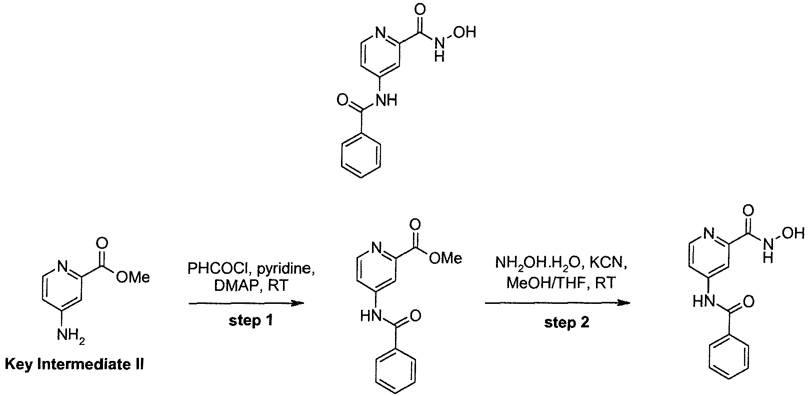

### Step 1:

4-Amino-pyridine-2-carboxylic acid methyl ester **(Key Intermediate II)** (400 mg, 2.6 mmol, 1 eq) was solubilized in pyridine (10 mL). Dimethylaminopyridine (catalytic amount) and benzoyl chloride (366 µL, 3.15 mmol, 1.2 eq) were added and the mixture was stirred at room temperature during 18 h. The solvent was then evaporated, water (10 mL) was added and the aqueous layer was extracted with ethyl acetate (3 x 20 mL). The organic layers were dried over magnesium sulfate, filtered and evaporated in vacuo. The crude residue was purified by flash chromatography using cyclohexane and ethyl acetate (100/0 to 50/50) to afford 4-benzoylamino-pyridine-2-carboxylic acid methyl ester as a white foam (654 mg, 97 % yield).

### Step 2:

To a solution of 4-benzoylamino-pyridine-2-carboxylic acid methyl ester (100 mg, 0.4 mmol, 1 eq) in a mixture of methanol (2 mL) and tetrahydrofuran (2mL) were added potassium cyanide (catalytic amount) and a 50% aqueous solution of hydroxylamine (1.6 mL). The mixture was stirred at room temperature during 4 days. A saturated solution of citric acid (10 mL) and water (10 mL) were then added and the aqueous layer was extracted with ethyl acetate (3 x 20 mL). The organic layers were dried over magnesium sulfate, filtered and evaporated in vacuo. The crude residue was taken in ethyl acetate (5 mL) and dichloromethane (5 mL) and sonicated. The solid was filtered and dried to afford the expected compound as white powder (78 mg, 78 % yield).
MS: 258.0
Mp: 175 °C - 184 °C

### General procedure E

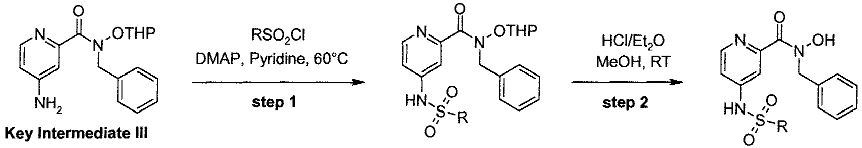

This procedure was similar to **general procedure D, steps 1 and 4.**

### Example 57:

### 4-Phenylmethanesulfonylamino-pyridine-2-carboxylic acid benzyl-hydroxy-amide

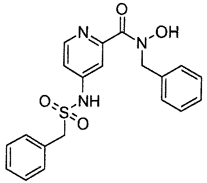

This compound was obtained according to **general procedure E** using phenylmethane-sulfonyl chloride. The expected compound was isolated as a white powder.
MS: 398.2
Mp: 190 °C - 195 °C

### Example 58:

### 4-Benzenesulfonylamino-pyridine-2-carboxylic acid benzyl-hydroxy-amide

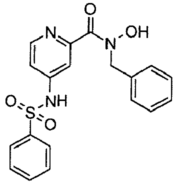

This compound was obtained according to **general procedure E** using benzene sulfonyl chloride. The expected compound was isolated as a pale rose oil.
MS: 384.2
Mp: 175 °C - 180 °C

### Example 59:

### 4-(4-Fluoro-phenylmethanesulfonylamino)-pyridine-2-carboxylic acid benzyl-hydroxy-amide hydrochloride

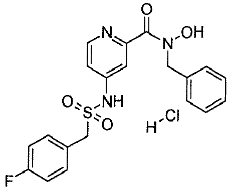

This compound was obtained according to **general procedure E** using 4-fluorophenyl-methanesulfonyl chloride. The expected compound was isolated as a beige powder.
MS: 416.3
Mp: 178 °C - 183 °C

### Example 60:

### 4-(3-Fluoro-phenylmethanesulfonylamino)-pyridine-2-carboxylic acid benzyl-hydroxy-amide hydrochloride

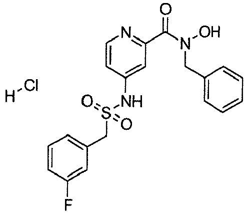

This compound was obtained according to **general procedure E** using 3-fluorophenyl-methanesulfonyl chloride. The expected compound was obtained as a beige powder.
MS: 416.2
Mp: 111 °C - 113 °C

### Example 61:

### 4-(3-Chlorophenylmethanesulfonylamino)-pyridine-2-carboxylic acid benzylhydroxy-amide hydrochloride

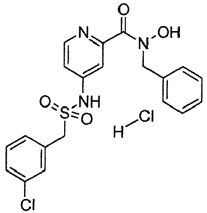

This compound was obtained according to **general procedure E** using 3-chlorophenyl-methanesulfonyl chloride. The expected compound was isolated as a white powder.
MS: 432.3
Mp: 115 °C - 125 °C

### Example 62:

### 4-(3,5-Dichlorophenylmethanesulfonylamino)-pyridine-2-carboxylic acid benzylhydroxyamide hydrochloride

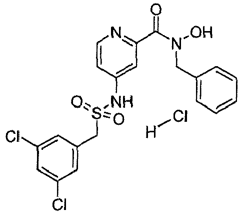

This compound was obtained according to **general procedure E** using 3,5-dichlorophenyl-methanesulfonyl chloride. The expected compound was isolated as a white powder.
MS: 466.3
Mp: 189 °C -194 °C

### Example 63:

### 4-(3-Trifluoromethylphenylmethanesulfonylamino)-pyridine-2-carboxylic acid benzylhydroxyamide hydrochloride

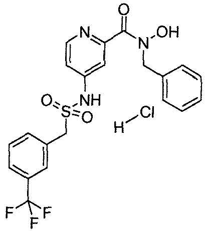

This compound was obtained according to **general procedure E** using 3-trifluoromethyl-phenylmethanesulfonyl chloride. The expected compound was isolated as a beige powder.
MS: 466.2
Mp: 178 °C -182 °C

### General procedure F

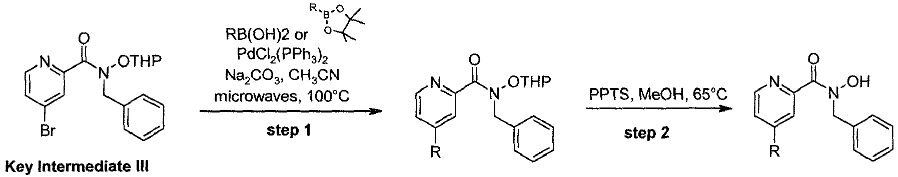

### Step 1:

To a degassed solution of 4-bromo-pyridine-2-carboxylic acid benzyl-(tetrahydro-pyran-2-yloxy)-amide **(Key Intermediate III**) (150 mg, 0.4 mmol, 1 eq) in a mixture of acetonitrile (3 mL) and 1 M solution of sodium carbonate (3 mL) were added boronic acid (0.5 mmol, 1.3 eq) and trans-dichlorobis(triphenylphosphine)palladium (II) (13 mg, 0.02 mmol, 0.05 eq). The mixture was heated under microwave irradiation at 100 °C during 10 min. After cooling, the mixture was poured on water (5 mL) and extracted with ethyl acetate (3 x 10 mL). The organic layers were dried over magnesium sulfate, filtered and evaporated in vacuo. The crude residue was purified by flash chromatography to afford the expected compound.

### Step 2:

The compound from step 1 (1 eq) was solubilized in methanol (10 mL) and pyridinium *p*-toluenesulfonate (1 eq) was added. The mixture was heated at 65 °C for 5 h and evaporated to dryness. The residue was triturated in water, filtered, rinsed with water and dried to afford the expected compound.

### Example 64:

### 4-Phenyl-pyridine-2-carboxylic acid benzyl-hydroxy-amide

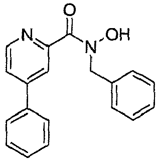

This compound was obtained according to **general procedure F** using phenylboronic acid. The expected compound was isolated as a pale rose powder.
MS: 304.9
Mp: 160 °C -165 °C

### Example 65:

### 4-(4-chloro-phenyl)-pyridine-2-carboxylic acid benzyl-hydroxy-amide

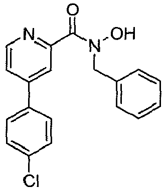

This compound was obtained according to **general procedure F** using 4-chlorophenyl-boronic acid. The expected compound was isolated as a white powder.
MS: 339.2
Mp: 190 °C -195 °C

### Example 66:

### 4-(3,4-Dichloro-phenyl)-pyridine-2-carboxylic acid benzyl-hydroxy-amide

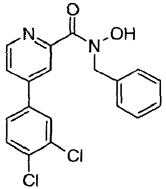

This compound was obtained according to **general procedure F** using 3,4-dichlorophenyl-boronic acid. The expected compound was isolated as a pale orange powder.
MS: 373.2
Mp: 125 °C - 130 °C

### Example 67:

### 4-(3-Carbamoyl-phenyl)-pyridine-2-carboxylic acid benzyl-hydroxy-amide

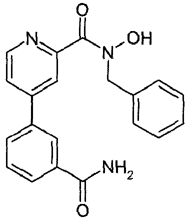

This compound was obtained according to **general procedure F** using 3-carbamoyl-phenylboronic acid. The expected compound was isolated as a beige powder.
MS: 348.1
Mp: 158 °C - 162 °C

### Example 68:

### 4-(4-Carbamoyl-phenyl)-pyridine-2-carboxylic acid benzyl-hydroxy-amide

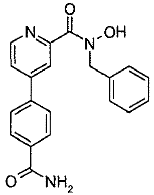

This compound was obtained according to **general procedure F** using 4-carbamoyl-phenylboronic acid. The expected compound was isolated as a pale yellow powder.
MS: 348.2
Mp: 155 °C -160 °C

### Example 69:

### 4-(3-Methylcarbamoyl-phenyl)-pyridine-2-carboxylic acid benzyl-hydroxy-amide

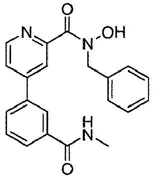

This compound was obtained according to **general procedure F** using 3-methylcarbamoyl-phenylboronic acid. The expected compound was isolated as a pale yellow foam.
MS: 362.2

### Example 70:

### 4-(3-Dimethylcarbamoyl-phenyl)-pyridine-2-carboxylic acid benzyl-hydroxy-amide

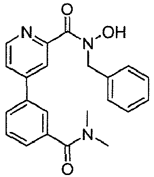

This compound was obtained according to **general procedure F** using 3-dimethyl-carbamoyl-phenylboronic acid. The expected compound was isolated as a yellow foam. MS: 376.2

### Example 71:

### 4-[3-(2-Dimethylamino-ethylcarbamoyl)-phenyl]-pyridine-2-carboxylic acid benzyl-hydroxy-amide

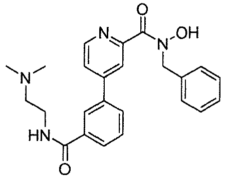

This compound was obtained according to **general procedure F** using 3-(2-(dimethyl-amino)ethylcarbamoyl)phenylboronic acid. The expected compound was isolated as a white foam.
MS: 419.3
Mp: 65 °C - 70 °C

### Example 72:

### 4-(3-Dimethylsulfamoyl-phenyl)-pyridine-2-carboxylic acid benzyl-hydroxy-amide

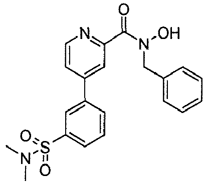

This compound was obtained according to **general procedure F** using 3-dimethylsulfamoyl-phenylboronic acid. The expected compound was isolated as a yellow powder.
MS: 412.2
Mp: 110 °C -115 °C

### Example 73:

### 4-(3-Hydroxymethyl-phenyl)-pyridine-2-carboxylic acid benzyl-hydroxy-amide

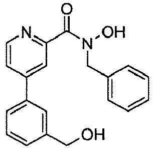

This compound was obtained according to **general procedure F** using 3-hydroxymethyl-phenylboronic acid. The expected compound was isolated as a white powder.
MS: 335.2
Mp: 150 °C - 155 °C

### Example 74:

### 4-Cyclohex-1-enyl-pyridine-2-carboxylic acid benzylhydroxyamide

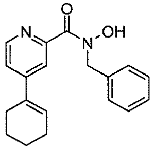

This compound was obtained according to **general procedure F** using cyclohexen-1-ylboronic acid, pinacol ester. The expected compound was isolated as a white powder.
MS: 309.2
Mp: 118 °C -122 °C

### Example 75:

### 4-Cyclohexylpyridine-2-carboxylic acid benzylhydroxy-amide

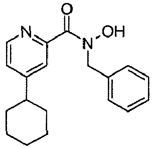

4-Cyclohex-1-enyl-pyridine-2-carboxylic acid benzylhydroxyamide (100 mg, 0.3 mmol, 1 eq) obtained in **example 74** was solubilized in ethanol (10 mL) and palladium 10% w on carbon was added. The mixture was stirred at room temperature over hydrogen atmosphere for 30 min. The mixture was then filtered over a short pad of celite, and rinsed with ethanol and dichloromethane. The crude residue was purified by flash chromatography using cyclohexane and ethyl acetate (100/0 to 70/30) to afford the expected compound as a white powder (72 mg, 72 % yield).
MS: 311.2
Mp: 106 °C -110 °C

### Example 76:

### 4-(1,4-Dioxa-spiro[4.5]dec-7-en-8-yl)-pyridine-2-carboxylic acid benzyl-hydroxy-amide

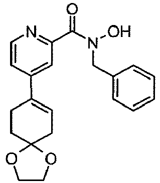

This compound was obtained according to **general procedure F** using 1,4-dioxa-spiro[4,5]dec-7-en-8-boronic acid, pinacol ester. The expected compound was isolated as a yellow foam.
MS: 367.2

### Example 77:

### 1'-Methyl-1',2',3',6'-tetrahydro-[4,4']bipyridinyl-2-carboxylic acid benzyl-hydroxy-amide

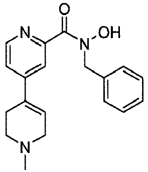

This compound was obtained according to **general procedure F** using 1-methyl-1,2,3,6-tetrahydropyridine-4-boronic acid pinacol ester. The expected compound was isolated as a light yellow powder.
MS: 324.2
Mp: 135 °C -155 °C

### Example 78:

### 2',2',6',6'-Tetramethyl-1',2',3',6'-tetrahydro-[4,4']bipyridinyl-2-carboxylic acid benzyl-hydroxy-amide

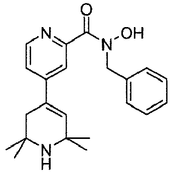

This compound was obtained according to **general procedure F** using 2,2,6,6-tetramethyl-1,2,3,6-tetrahydro-4-pyridineboronic acid pinacol ester. The expected compound was isolated as a yellow crystallized oil.
MS: 366.3

### Example 79:

### 2'-(Benzyl-hydroxy-carbamoyl)-3,6-dihydro-2H-[4,4']bipyridinyl-1-carboxylic acid tert-butyl ester

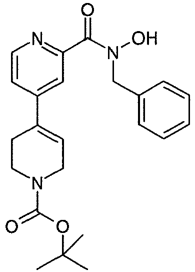

This compound was obtained according to **general procedure F** using N-Boc-1,2,3,6-tetrahydropyridine-4-boronic acid pinacol ester. The expected compound was isolated as a 5 beige powder.
MS: 410.3
Mp: 125 °C

### Example 80:

### ) 2'-(Benzyl-hydroxy-carbamoyl)-5,6-dihydro-4H-[3,4']bipyridinyl-1-carboxylic acid tert-butyl ester

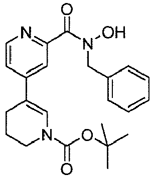

This compound was obtained according to **general procedure F** using 5-(4,4,5,5-tetramethyl-[1,3,2]dioxaborolan-2-yl)-3,4-dihydro-2H-pyridine-1-carboxylic acid tert-butyl ester **(Key Intermediate V).** The expected compound was isolated as a yellow foam.
MS: 410.3

### Example 81:

### 2'-(Benzylhydroxycarbamoyl)-5,6-dihydro-2H-[3,4']bipyridinyl-1-carboxylic acid tert-butyl ester

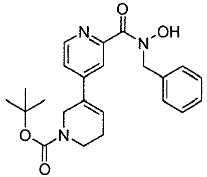

This compound was obtained according to **general procedure F** using 5-(4,4,5,5-tetramethyl-[1,3,2]dioxaborolan-2-yl)-3,6-dihydro-2H-pyridine-1-carboxylic acid tert-butyl ester **(Key Intermediate VI).** The expected compound was isolated as a yellow powder.
MS: 410.3
Mp: 128 °C - 134 °C

### Example 82:

### 3-[2-(Benzylhydroxycarbamoyl)-pyridin-4-yl]-8-azabicyclo[3.2.1]oct-2-ene-8-carboxylic acid tertbutylester

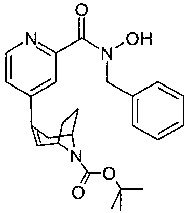

This compound was obtained according to **general procedure F** using 8-boc-3-(4,4,5,5-tetramethyl-[1,3,2]dioxaborolan-2-yl)-8-aza-bicyclo[3.2.1]oct-2-ene. The expected compound was isolated as a yellow oil.
MS: 436.3

### General procedure G

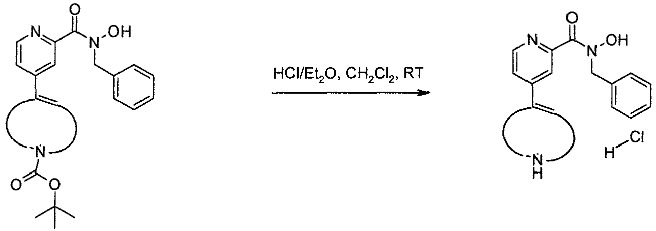

Compound obtained from **general procedure F** (1 eq) was solubilized in dichloromethane (10 mL) and a 2M solution of hydrochloric acid in diethyl ether (16 eq) was added drop wise. The mixture was stirred at room temperature for 2 h. The precipitate was filtered and triturated with dichloromethane and diethyl ether to afford the expected compound (60 % yield).

### Example 83:

### 1',2',3',6'-Tetrahydro-[4,4']bipyridinyl-2-carboxylic acid benzyl-hydroxy-amide dihydrochloride

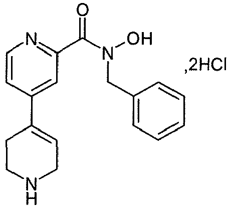

This compound was obtained according to **general procedure G** using 2'-(benzyl-hydroxy-carbamoyl)-3,6-dihydro-2H-[4,4']bipyridinyl-1-carboxylic acid tert-butyl ester described in **example 79.** The expected compound was isolated as a beige powder.
MS: 310.1
Mp: 140 °C - 150 °C

### Example 84:

### 1,2,5,6- Tetrahydro-[3,4']bipyridinyl-2'-carboxylic acid benzylhydroxy-amide hydrochloride

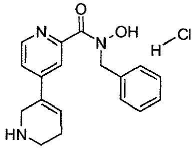

This compound was obtained according to **general procedure G** using 2'-(benzylhydroxy-carbamoyl)-5,6-dihydro-2H-[3,4']bipyridinyl-1-carboxylic acid tert-butyl ester described in **example 81.** The expected compound was isolated as a yellow crystallized oil.
MS: 310.2

### Example 85:

### 4-(8-Azabicyclo[3.2.1]oct-2-en-3-yl)-pyridine-2-carboxylic acid benzylhydroxyamide

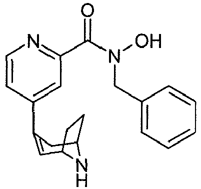

This compound was obtained according to **general procedure G** using 3-[2-(benzylhydroxycarbamoyl)-pyridin-4-yl]-8-azabicyclo[3.2.1]oct-2-ene-8-carboxylic acid tertbutylester described in **example 82.** The expected compound was isolated as a yellow powder.
MS: 336.1
Mp: 95 °C - 100 °C

### General procedure H

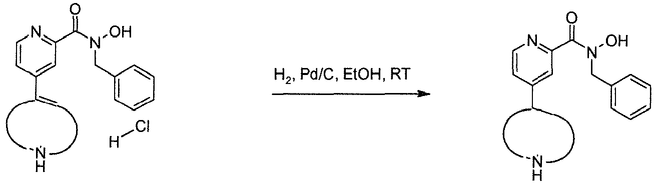

The compound obtained from **general procedure G** (1 eq) was solubilized in ethanol (10 mL) and palladium 10% w on carbon was added. The mixture was stirred at room temperature over hydrogen atmosphere for 30 min. The mixture was then filtered over a short pad of celite and the crude residue was purified by flash chromatography using ethyl acetate and methanol (100/0 to 80/20) to afford the expected compound.

### Example 86:

### 1,2,3,4,5,6-Hexahydro-[3,4']bipyridinyl-2'-carboxylic acid benzylhydroxyamide

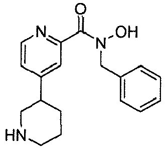

This compound was obtained according to **general procedure H** using 1,2,5,6-tetrahydro-[3,4']bipyridinyl-2'-carboxylic acid benzylhydroxy-amide hydrochloride described in **example 84.** The expected compound was isolated as a yellow crystallized oil.
MS: 312.2

### Example 87:

### 2'-(Benzyl-hydroxy-carbamoyl)-3,4,5,6-tetrahydro-2H-[4,4']bipyridinyl-1-carboxylic acid tert-butyl ester

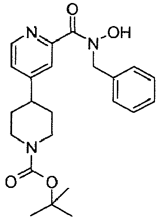

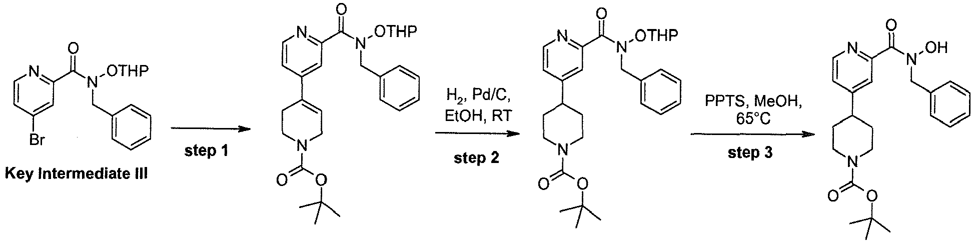

### Step 1:

This compound was obtained according to **general procedure F, step 1** starting from **Key Intermediate III** and N-Boc-1,2,3,6-tetrahydropyridine-4-boronic acid pinacol ester.

### Step 2:

The compound from step 1 (485 mg, 1 mmol, 1 eq) was solubilized in ethanol (20 mL) and palladium 10% w on carbon was added. The mixture was stirred at room temperature over hydrogen atmosphere for 1.5 h. The mixture was then filtered over a short pad of celite and the crude residue was purified by flash chromatography using cyclohexane and ethyl acetate (100/0 to 40/60) to afford 2'-[benzyl-(tetrahydro-pyran-2-yloxy)-carbamoyl]-3,4,5,6-tetrahydro-2H-[4,4']bipyridinyl-1-carboxylic acid tert-butyl ester as a colorless oil (320 mg, 66 % yield).

### Step 3:

The compound from step 2 (360 mg, 0.6 mmol, 1 eq) was solubilized in methanol (20 mL) and pyridinium *p*-toluenesulfonate (182 mg, 0.6 mmol, 1 eq) was added. The mixture was heated at 65 °C for 18 h and evaporated to dryness. Ethyl acetate (10 mL) was added and the organic layer was washed with a saturated solution of sodium bicarbonate (3 x 10 mL), dried over magnesium sulfate, filtered and evaporated in vacuo. The crude residue was purified by flash chromatography using cyclohexane and ethyl acetate (80/20 to 30/70) to afford the expected compound as an orange oil (230 mg, 77 % yield).
MS: 412.3

### Example 88:

### 1',2',3',4',5',6'-Hexahydro-[4,4']bipyridinyl-2-carboxylic acid benzyl-hydroxy-amide hydrochloride

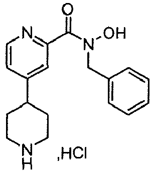

This compound was obtained according to **general procedure G** using 2'-(benzyl-hydroxy-carbamoyl)-3,4,5,6-tetrahydro-2H-[4,4']bipyridinyl-1-carboxylic acid tert-butyl ester described in **example 87.** The expected compound was isolated as a white foam.
MS: 312.1

### Example 89:

### 4-Phenyl-pyridine-2-carboxylic acid(4-fluoro-benzyl)-hydroxy-amide

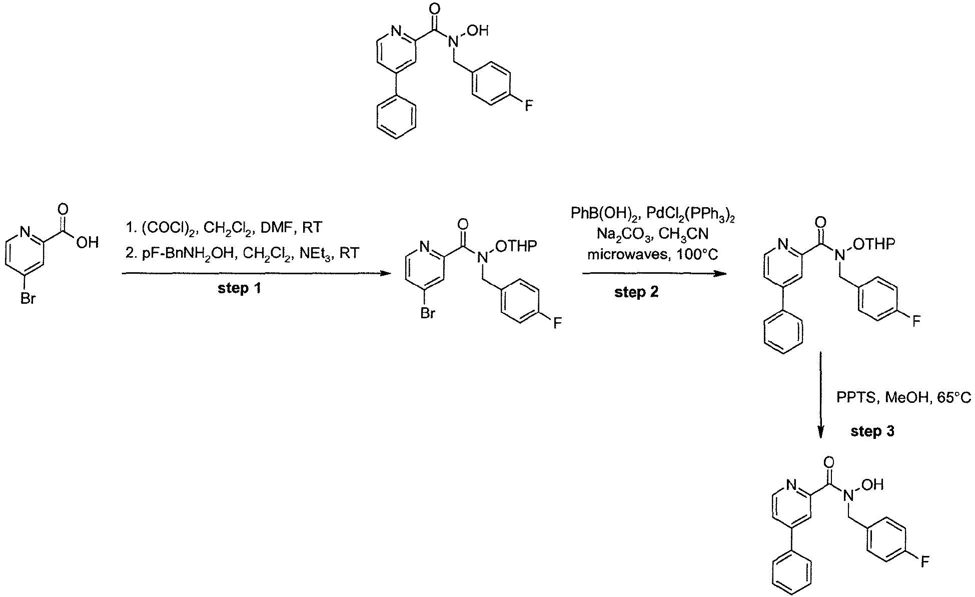

### Step 1:

Oxalyl chloride (0.2 mL, 2.1 mmol, 1.3 eq) was added to a solution of 4-bromo-pyridine-2-carboxylic acid (334 mg, 1.6 mmol, 1 eq) in dichloromethane (15 mL). The solution was cooled down to 0 °C and dimethylformamide (several drops) was added drop wise. The mixture was stirred at room temperature for 30 min and was evaporated to dryness. The residue was diluted in dichloromethane (15 mL) and N-(4-fluoro-benzyl)-O-(tetrahydropyran-2-yl)-hydroxylamine (560 mg, 2.5 mmol, 1.5 eq) was added. Triethylamine (0.7 mL, 4.9 mmol, 3 eq) was added drop wise at 0 °C and the mixture was stirred at room temperature for 18 h and absorbed on silica gel to be purified by flash chromatography using cyclohexane and ethyl acetate (100/0 to 70/30) to afford 4-bromo-pyridine-2-carboxylic acid (4-fluoro-benzyl)-(tetrahydro-pyran-2-yloxy)-amide as a colorless oil (230 mg, 34 % yield).

### Step 2:

To a degassed solution of 4-bromo-pyridine-2-carboxylic acid (4-fluoro-benzyl)-(tetrahydropyran-2-yloxy)-amide (230 mg, 0.6 mmol, 1 eq) in a mixture of acetonitrile (4 mL) and 1 M solution of sodium carbonate (4 mL) were added phenylboronic acid (89 mg, 0.7 mmol, 1.3 eq) and trans-dichlorobis(triphenylphosphine)palladium (20 mg, 0.03 mmol, 0.05 eq). The mixture was heated under microwave irradiation at 100 °C during 10 min. After cooling, the mixture was poured on water (5 mL) and extracted with ethyl acetate (3 x 10 mL). The organic layers were dried over magnesium sulfate, filtered and evaporated in vacuo. The crude residue was purified by flash chromatography using cyclohexane and ethyl acetate (100/0 to 50/50) to afford 4-phenyl-pyridine-2-carboxylic acid (4-fluoro-benzyl)-(tetrahydropyran-2-yloxy)-amide as a colorless oil (130 mg, 57 % yield).

### Step 3:

4-Phenyl-pyridine-2-carboxylic acid (4-fluoro-benzyl)-(tetrahydro-pyran-2-yloxy)-amide (130 mg, 0.3 mmol, 1 eq) was solubilized in methanol (5 mL) and pyridinium *p*-toluenesulfonate (97 mg, 0.4 mmol, 1.2 eq) was added. The mixture was heated at 65 °C for 5 h. The precipitate obtained was filtered and washed with a minimum of methanol to afford the expected compound as a white powder (13 mg, 13 % yield).
MS: 323.1
Mp: 135 °C - 140 °C

### Example 90:

### 5-Phenyl-pyridine-2-carboxylic acid benzyl-hydroxy-amide

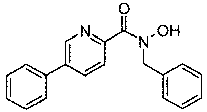

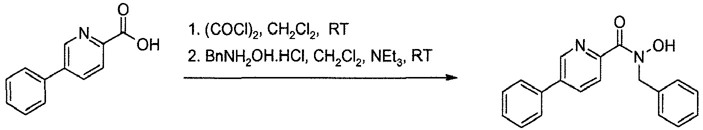

At 0 °C, oxalyl chloride (0.2 mL, 2.3 mmol, 1.5 eq) was added to a solution of 5-phenylpyridine-2-carboxylic acid (300 mg, 1.5 mmol, 1 eq) in dichloromethane (10 mL). The 5 mixture was stirred at room temperature for 30 min and was evaporated to dryness. The residue was diluted in dichloromethane (10 mL) and N-benzyl-hydroxylamine hydrochloride (361 mg, 2.3 mmol, 1.5 eq) and triethylamine (0.6 mL, 4.5 mmol, 3 eq) were added. The mixture was stirred at room temperature for 18 h and absorbed on silica gel to be purified by flash chromatography using cyclohexane and ethyl acetate (100/0 to 0/100) to afford the expected compound as a beige powder (60 mg, 13 % yield).
MS: 305.2
Mp: 145 °C - 150 °C

### Example 91:

### 5-Phenyl-pyridine-2-carboxylic acid hydroxyamide

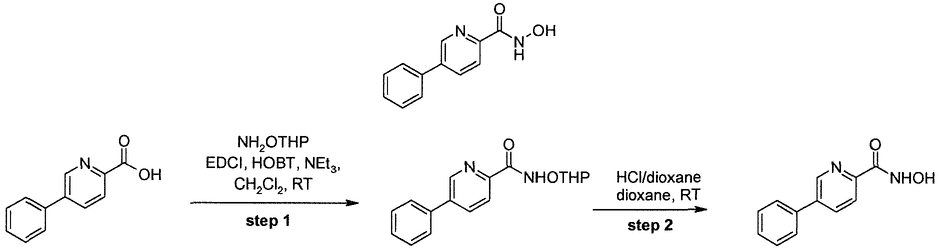

### Step 1:

To a solution of 5-phenyl-pyridine-2-carboxylic acid (130 mg, 0.6 mmol, 1 eq) in dichloromethane (6 mL) were added HOBT (176 mg, 1.3 mmol, 2 eq), EDCI (249 mg, 1.3 mmol, 2 eq), triethylamine (0.3 mL, 1.8 mmol, 3 eq) and O-(tetrahydro-pyran-2-yl)-hydroxylamine (153 mg, 1.3 mmol, 2 eq). The mixture was stirred at room temperature for 18 h and absorbed on silica gel to be purified by flash chromatography using cyclohexane and ethyl acetate (100/0 to 50/50) to afford 5-phenyl-pyridine-2-carboxylic acid (tetrahydropyran-2-yloxy)-amide as a colorless oil (160 mg, 83 % yield).

### Step 2:

To a solution of 5-phenyl-pyridine-2-carboxylic acid (tetrahydro-pyran-2-yloxy)-amide (160 mg, 0.54 mmol, 1 eq) in dioxane (5 mL) was added a 4 N solution on hydrogen chloride in dioxane (0.5 mL). The mixture was stirred at room temperature for 1 h and evaporated to dryness. The residue was diluted in methanol (5 mL) and ammonia 7 N in methanol (0.5 mL) was added. The mixture was evaporated and the residue was triturated in water to afford the expected compound as a pale rose powder (90 mg, 78 % yield).
MS: 215.1
Mp: 175 °C -180 °C

### General procedure I

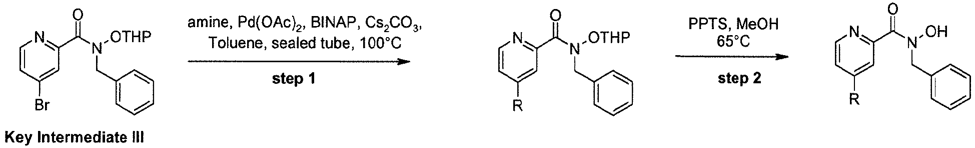

### Step 1:

To a degassed solution of 4-bromo-pyridine-2-carboxylic acid benzyl-(tetrahydro-pyran-2-yloxy)-amide **(Key Intermediate III)** (500 mg, 1.3 mmol, 1 eq) in toluene (10 mL) were added cesium carbonate (1.3 g, 3.8 mmol, 3 eq), amine (1.66 mmol, 1.3 eq), BINAP (40 mg, 0.06 mmol, 0.05 eq) and palladium acetate (15 mg, 0.06 mmol, 0.05 eq). The mixture was heated in a sealed tube at 100 °C during 20 h. After cooling, the mixture was poured on water (10 mL) and extracted with ethyl acetate (3 x 10 mL). The organic layers were dried over magnesium sulfate, filtered and evaporated in vacuo. The crude residue was purified by flash chromatography to afford the expected compound.

### Step 2:

The compound from step 1 (1 eq) was solubilized in methanol (10 mL) and pyridinium *p*-toluenesulfonate (1 eq) was added. The mixture was heated at 65 °C for 20 h. After cooling, a 7 N solution of ammonia in methanol (10 mL) was added and the mixture was evaporated to dryness. The residue was diluted in dichloromethane (10 mL) and the organic layer was washed with water (3 x 10 mL), dried over magnesium sulfate, filtered and evaporated in vacuo. The crude compound was purified by flash chromatography to afford the expected compound.

### Example 92:

### 3,3-Difluoro-3,4,5,6-tetrahydro-2H-[1,4']bipyridinyl-2'-carboxylic acid benzyl-hydroxy-amide

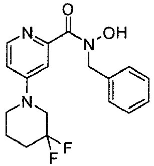

This compound was obtained according to **general procedure I** using 3,3-difluoropiperidine hydrochloride. The expected compound was isolated as a pale yellow powder.
MS: 348.1
Mp: 140 °C - 145 °C

### Example 93:

### 4,4-Difluoro-3,4,5,6-tetrahydro-2H-[1,4']bipyridinyl-2'-carboxylic acid benzyl-hydroxy-amide

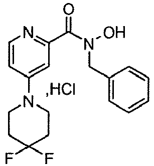

This compound was obtained according to **general procedure I** using 4,4-difluoropiperidine hydrochloride followed by addition of 2 M solution of hydrogen chloride in diethyl ether. After stirring 2 h at room temperature, filtration and trituration with diethyl ether, the expected compound was isolated as a white powder.
MS: 348.2
Mp: 90 °C - 95 °C

### Example 94:

### 4-Fluoro-3,4,5,6-tetrahydro-2H-[1,4']bipyridinyl-2'-carboxylic acid benzyl-hydroxy-amide hydrochloride

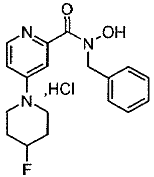

This compound was obtained according to a modified version of **general procedure I** using 4-fluoropiperidine hydrochloride. During step 2, instead of using pyridinium *p*-toluenesulfonate, 2 M solution of hydrogen chloride in diethyl ether (20 eq) was added and the mixture was stirred at room temperature for 2 h. The precipitate was then filtered and triturated with dichloromethane and diethyl ether to afford the expected compound as a light yellow foam.
MS: 330.1

### Example 95:

### 4-(3,3-Difluoro-pyrrolidin-1-yl)-pyridine-2-carboxylic acid benzyl-hydroxy-amide hydrochloride

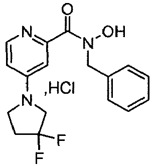

This compound was obtained according to a modified version of **general procedure I** using 3,3-difluoropyrrolidine hydrochloride. During step 2, instead of using pyridinium *p*-toluenesulfonate, 2 M solution of hydrogen chloride in diethyl ether (20 eq) was added and the mixture was stirred at room temperature for 2 h. The precipitate was then filtered and triturated with dichloromethane and diethyl ether to afford the expected compound as a beige powder.
MS: 334.1
Mp: 162 °C - 166 °C

### Example 96:

### [2'-(Benzyl-hydroxy-carbamoyl)-3,4,5,6-tetrahydro-2H-[1,4']bipyridinyl-4-yl]-carbamic acid tert-butyl ester

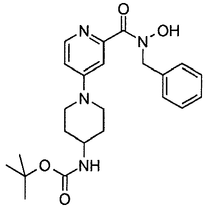

This compound was obtained according to **general procedure I** using 4-N-BOC-aminopiperidine. The expected compound was isolated as a white foam.
MS: 427.3
Mp: 135 °C - 140 °C

### Example 97:

### 4-Amino-3,4,5,6-tetrahydro-2H-[1,4']bipyridinyl-2'-carboxylic acid benzyl-hydroxy-amide chlorhydrate

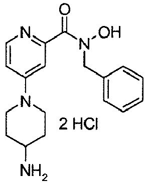

This compound was obtained according to **general procedure G** using [2'-(benzyl-hydroxy-carbamoyl)-3,4,5,6-tetrahydro-2H-[1,4']bipyridinyl-4-yl]-carbamic acid tert-butyl ester described in **example 96.** The expected compound was isolated as a white powder.
MS: 327.2
Mp: decomposes at 160 °C - 165 °C

### Example 98:

### 4-Dimethylamino-3,4,5,6-tetrahydro-2H-[1,4']bipyridinyl-2'-carboxylic acid benzyl-hydroxy-amide

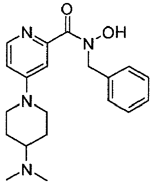

This compound was obtained according to **general procedure I** using dimethyl-piperidin-4-yl-amine. The expected compound was isolated as a yellow oil.
MS: 355.2

### Example 99:

### 4-Pyrrolidin-1-yl-3,4,5,6-tetrahydro-2H-[1,4']bipyridinyl-2'-carboxylic acid benzyl-hydroxy-amide

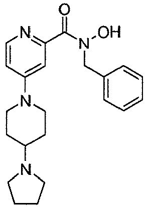

This compound was obtained according to **general procedure I** using 4-(1-pyrrolidinyl)piperidine. The expected compound was isolated as a pale yellow powder.
MS: 381.2
Mp: 135 °C -140 °C

### Example 100:

### 3,4,5,6,3',4',5',6'-Octahydro-2H,2'H-[1,4';1',4"]terpyridine-2"-carboxylic acid benzyl-hydroxy-amide

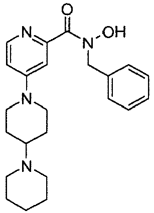

This compound was obtained according to **general procedure I** using 4 N-(4-piperidino)piperidine. The expected compound was isolated as a blue oil.
MS: 395.2

### Example 101:

### 4-(1,4-Dioxa-8-aza-spiro[4.5]dec-8-yl)-pyridine-2-carboxylic acid benzyl-hydroxy-amide

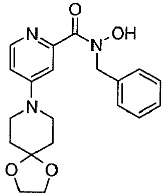

This compound was obtained according to **general procedure I** using 1,4-dioxa-8-azaspiro[4.5]decane. The expected compound was isolated as a yellow powder.
MS: 370.2
Mp: 98 °C - 102 °C

### Example 102:

### 4-[2-(Benzyl-hydroxy-carbamoyl)-pyridin-4-yl]-piperazine-1-carboxylic acid tert-butyl ester

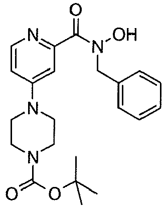

This compound was obtained according to **general procedure I** using N-BOC piperazine. The expected compound was isolated as a yellow foam.
MS: 413.3

### Example 103:

### 4-Piperazin-1-yl-pyridine-2-carboxylic acid benzyl-hydroxy-amide hydrochloride

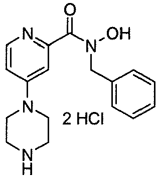

This compound was obtained according to **general procedure G** using 4-[2-(benzyl-hydroxy-carbamoyl)-pyridin-4-yl]-piperazine-1-carboxylic acid tert-butyl ester described in **example 102.** The expected compound was isolated as a yellow foam.
MS: 313.2

### Example 104:

### 4-(4-Methyl-piperazin-1-yl)-pyridine-2-carboxylic acid benzyl-hydroxy-amide

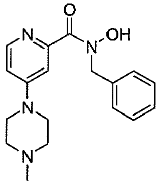

This compound was obtained according to **general procedure I** using N-methyl piperazine. The expected compound was isolated as a yellow oil.
MS: 327.2

### Example 105:

### 4-Morpholin-4-yl-pyridine-2-carboxylic acid benzyl-hydroxy-amide

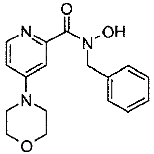

This compound was obtained according to **general procedure I** using morpholine. The expected compound was isolated as a pale yellow powder.
MS: 314.1
Mp: 105 °C - 110 °C

### Example 106:

### 4-Morpholin-4-yl-pyridine-2-carboxylic acid benzyl-hydroxy-amide hydrochloride

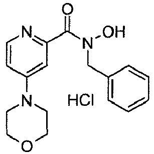

4-Morpholin-4-yl-pyridine-2-carboxylic acid benzyl-hydroxy-amide described in **example 105** was solubilized in dichloromethane (10 mL) and 2 M solution of hydrogen chloride in diethyl ether (1.2 eq) was added. The mixture was stirred at room for 3 h and evaporated to dryness to afford the expected compound as a pale yellow powder.
MS: 314.1
Mp: 185 °C - 190 °C

### Example 107:

### 4-((2R,6S)-2,6-Dimethyl-morpholin-4-yl)-pyridine-2-carboxylic acid benzyl-hydroxy-amide

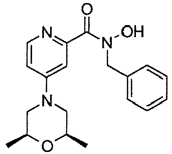

This compound was obtained according to **general procedure I** using (2R,6S)-2,6-dimethyl-morpholine. The expected compound was isolated as an orange powder.
MS: 342.2
Mp: 180 °C - 185 °C

### Example 108:

### 4-Benzylamino-pyridine-2-carboxylic acid hydroxyamide

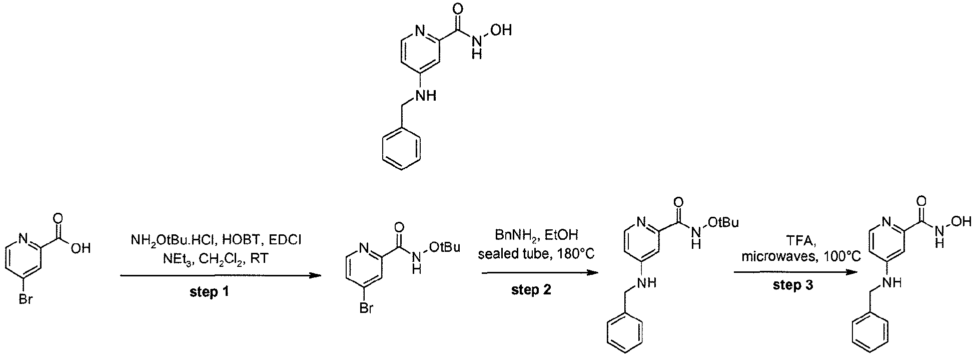

### Step 1:

To a solution of 4-bromo-pyridine-2-carboxylic acid (1.0 g, 4.9 mmol, 1 eq) in dichloromethane (40 mL) were added HOBT (1.3 g, 9.9 mmol, 2 eq), EDCI (1.9 g, 9.9 mmol, 2 eq), triethylamine (2.1 mL, 14.8 mmol, 3 eq) and O-tert-butylhydroxylamine hydrochloride (1.2 g, 9.9 mmol, 2 eq). The mixture was stirred at room temperature for 18 h and poured on water (20 mL). The organic layer was extracted with dichloromethane (3 x 20 mL), dried over magnesium sulfate, filtered and evaporated in vacuo. The crude residue was purified by flash chromatography using cyclohexane and ethyl acetate (100/0 to 50/50) to afford 4-bromo-pyridine-2-carboxylic acid tert-butoxy-amide as a white powder (1.0 g, 74 % yield).

### Step 2:

In a sealed tube, 4-bromo-pyridine-2-carboxylic acid tert-butoxy-amide (410 mg, 1.5 mmol, 1 eq) was solubilized in ethanol (10 mL) and benzylamine (161 mg, 3 mmol, 2 eq) was added. The mixture was heated at 180 °C for 20 h. After cooling, the mixture was absorbed on silica gel to be purified by flash chromatography using cyclohexane and ethyl acetate (100/0 to 0/100) to afford 4-benzylamino-pyridine-2-carboxylic acid tert-butoxy-amide as a colorless oil (57 mg, 13 % yield).

### Step 3:

4-Benzylamino-pyridine-2-carboxylic acid tert-butoxy-amide (57 mg, 0.19 mmol, 1 eq) and trifluoroacetic acid (3 mL) were heated under microwave irradiation at 100 °C during 10 min. After cooling, the mixture was evaporated to dryness. The residue was solubilized in dichloromethane (5 mL) and some drops of ammonium hydroxide solution were added. The mixture was absorbed on silica gel to be purified by flash chromatography using dichloromethane and methanol (100/0 to 85/15) to afford the expected compound as a colorless oil (15 mg, 32 % yield).
MS: 244.1

### Example 109:

### 4-(Benzyl-methyl-amino)-pyridine-2-carboxylic acid benzyl-hydroxy-amide

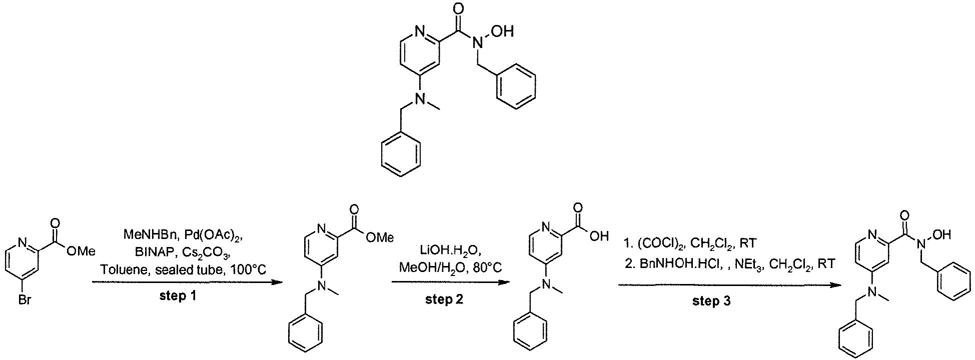

### Step 1:

To a degassed solution of 4-bromo-pyridine-2-carboxylic acid methyl ester (650 mg, 3.0 mmol, 1 eq) in toluene (15 mL) were added cesium carbonate (1.9 g, 6.0 mmol, 2 eq), N-methylbenzylamine (0.5 mL, 3.9 mmol, 1.3 eq), BINAP (93 mg, 0.15 mmol, 0.05 eq) and palladium acetate (34 mg, 0.15 mmol, 0.05 eq). The mixture was heated in a sealed tube at 100 °C during 20 h. After cooling, the mixture was poured on water (10 mL) and extracted with ethyl acetate (3 x 10 mL). The organic layers were dried over magnesium sulfate, filtered and evaporated in vacuo. The crude residue was purified by flash chromatography using dichloromethane and methanol (100/0 to 97/3) to afford 4-(benzyl-methyl-amino)-pyridine-2-carboxylic acid methyl ester as a yellow oil (230 mg, 30 % yield).

### Step 2:

4-(Benzyl-methyl-amino)-pyridine-2-carboxylic acid methyl ester (230 mg, 0.9 mmol, 1 eq) was solubilized in a mixture methanol / water (6 mL / 1 mL) and lithium hydroxide (75 mg, 1.8 mmol, 2 eq) was added. The mixture was heated at 80 °C during 3 h. After cooling down, a 1 M solution of hydrogen chloride in diethyl ether (1.8 mL, 1.8 mmol, 2 eq) was added. The mixture was then evaporated to dryness to afford 4-(benzyl-methyl-amino)-pyridine-2-carboxylic acid in quantitative yield.

### Step 3:

Oxalyl chloride (0.12 mL, 1.3 mmol, 1.5 eq) was added drop wise to a solution of 4-(benzyl-methyl-amino)-pyridine-2-carboxylic acid (0.9 mmol, 1 eq) in dichloromethane (10 mL). The mixture was stirred at room temperature for 15 min and was evaporated to dryness. The residue was diluted in dichloromethane (10 mL) and triethylamine (0.38 mL, 2.7 mmol, 3 eq) and N-benzylhydroxylamine hydrochloride (215 mg, 1.3 mmol, 1.5 eq) were added. After stirring at room temperature for 20 h, the mixture was absorbed on silica gel to be purified using cyclohexane and ethyl acetate (100/0 to 40/60). The expected compound was obtained as a yellow oil (85 mg, 27 % yield).
MS: 348.2

### Example 110:

### 4-Morpholin-4-yl-pyridine-2-carboxylic acid hydroxyamide

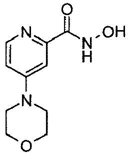

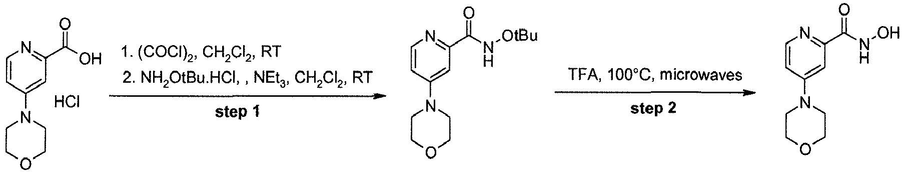

### Step 1:

Oxalyl chloride (0.11 mL, 1.3 mmol, 1.3 eq) was added drop wise to a solution of 4-morpholin-4-yl-pyridine-2-carboxylic acid hydrochloride (240 mg, 1.0 mmol, 1 eq) in dichloromethane (10 mL). At 0 °C, dimethylformamide (2-3 drops) was added drop wise and the mixture was stirred at room temperature for 15 min and was evaporated to dryness. The residue was diluted in dichloromethane (10 mL) and triethylamine (0.41 mL, 2.9 mmol, 3 eq) and O-tert-butylhydroxylamine hydrochloride (185 mg, 1.5 mmol, 1.5 eq) were added. After stirring at room temperature for 20 h, the mixture was absorbed on silica gel to be purified using cyclohexane and ethyl acetate (100/0 to 0/100). 4-Morpholin-4-yl-pyridine-2-carboxylic acid tert-butoxy-amide was obtained as a white powder (110 mg, 40 % yield).

### Step 2:

4-Morpholin-4-yl-pyridine-2-carboxylic acid tert-butoxy-amide (110 mg, 0.4 mmol, 1 eq) and trifluoroacetic acid (3 mL) were heated under microwave irradiation at 100 °C during 10 min. After cooling, the mixture was evaporated to dryness. The residue was solubilized in dichloromethane (5 mL) and some drops of ammonium hydroxide solution were added. The mixture was absorbed on silica gel to be purified by flash chromatography using dichloromethane and methanol (100/0 to 90/10) to afford the expected compound as a beige powder (12 mg, 14 % yield).
MS: 224.1
Mp: 215 °C - 220 °C (dec.)

### Example 111:

### 3,4,5,6-Tetrahydro-2H-[1,3']bipyridinyl-6'-carboxylic acid benzyl-hydroxy-amide

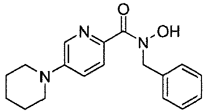

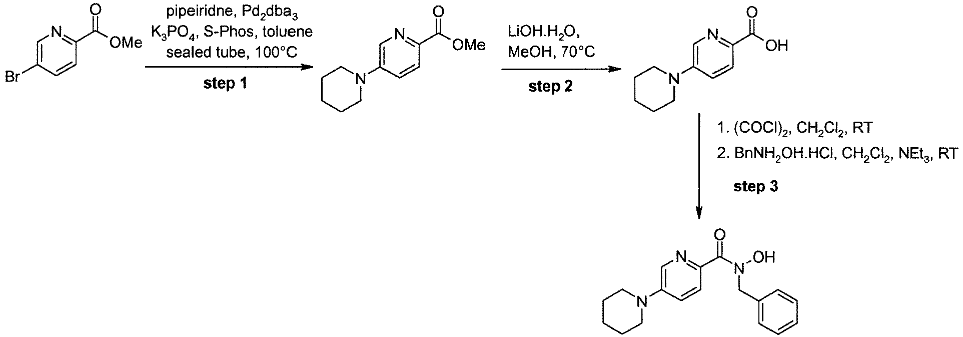

### Step 1:

To a degassed solution of 5-bromo-pyridine-2-carboxylic acid methyl ester (450 mg, 2.1 mmol, 1 eq) in toluene (10 mL) were added piperidine (213 mg, 2.5 mmol, 1.2 eq), potassium phosphate (618 mg, 2.9 mmol, 1.4 eq), 2-dicyclohexylphosphino-2',6'-dimethoxybiphenyl (171 mg, 0.42 mmol, 0.2 eq) and tris(dibenzylideneacetone)dipalladium (95 mg, 0.10 mmol, 0.05 eq). The mixture was heated in a sealed tube at 100 °C during 48 h. After cooling, the mixture was poured on water (5 mL) and extracted with ethyl acetate (3 x 10 mL). The organic layers were dried over magnesium sulfate, filtered and evaporated in vacuo. The crude residue was purified by flash chromatography using cyclohexane and ethyl acetate (100/0 to 0/100) to afford 3,4,5,6-tetrahydro-2H-[1,3']bipyridinyl-6'-carboxylic acid methyl ester as a pale yellow powder (165 mg, 36 % yield).

### Step 2:

3,4,5,6-Tetrahydro-2H-[1,3']bipyridinyl-6'-carboxylic acid methyl ester (165 mg, 0.75 mmol, 1 eq) was solubilized in methanol (8 mL) and lithium hydroxide (63 mg, 1.5 mmol, 2 eq) was added. The mixture was heated at 70 °C during 20 h. After cooling, a 3 N solution of hydrogen chloride (0.2 mL) was added. The mixture was then evaporated to dryness to afford 3,4,5,6-tTetrahydro-2H-[1,3']bipyridinyl-6'-carboxylic acid as a yellow oil in quantitative yield.

### Step 3:

Oxalyl chloride (0.1 mL, 1.12 mmol, 1.5 eq) was added drop wise to a solution of 3,4,5,6-tetrahydro-2H-[1,3']bipyridinyl-6'-carboxylic acid (0.75 mmol, 1 eq) in dichloromethane (6 mL). The mixture was stirred at room temperature for 15 min and was evaporated to dryness. The residue was diluted in dichloromethane (6 mL) and triethylamine (0.31 mL, 2.25 mmol, 3 eq) and N-benzylhydroxylamine hydrochloride (179 mg, 1.12 mmol, 1.5 eq) were added. After stirring at room temperature for 20 h, the mixture was absorbed on silica gel to be purified using cyclohexane and ethyl acetate (100/0 to 30/70). The expected compound was obtained as a pale yellow powder (125 mg, 54 % yield).
MS: 312.2
Mp: 110 °C - 115 °C

**Activity data for the compounds having the general formula (I)**

| **Molstructure** | **activity type** | **activity endpoint** | **activity conc** | **activity result** |
|---|---|---|---|---|
| 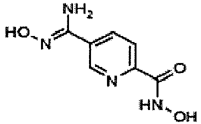 | FRET | IC50 [µM] | | 20 |
| | CPE H3N2 | reduction (%) | 50 | -4,8 |
| 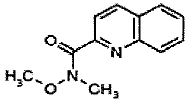 | FRET | IC50 [µM] | | |
| | CPE H3N2 | reduction (%) | 50 | -1,2 |
| 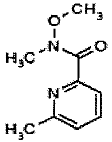 | CPE H3N2 | reduction (%) | 50 | -0,9 |
| 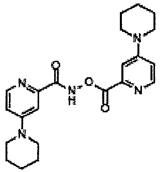 | CPE H3N2 | reduction (%) | 50 | 29 |
| | CPE H3N2 | IC50 [µM] | | 37 |
| 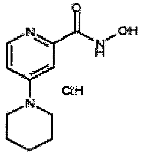 | FRET | IC50 [µM] | | 4,9 |
| | CPE H3N2 | reduction (%) | 5 | -4,1 |
| 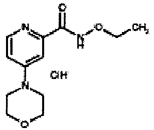 | FRET | IC50 [µM] | | |
| | CPEH3N2 | reduction (%) | 50 | 1,3 |
| 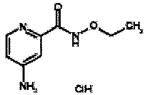 | CPE H3N2 | reduction (%) | 50 | 10,5 |
| 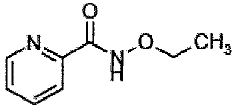 | CPE H3N2 | reduction (%) | 50 | 12,5 |
| 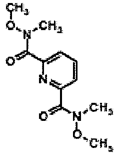 | CPE H3N2 | reduction (%) | 50 | -0,3 |
| 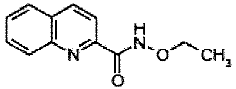 | CPE H3N2 | reduction (%) | 50 | -2,2 |
| 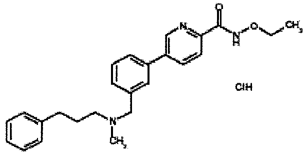 | CPE H3N2 | reduction (%) | 20 | 1,6 |
| 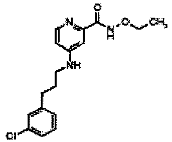 | CPE H3N2 | reduction (%) | 1 | -0,3 |
| 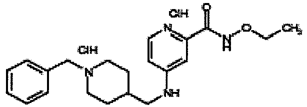 | CPE H3N2 | reduction (%) | 50 | 14,8 |
| 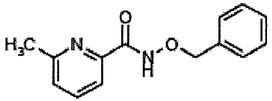 | CPE H3N2 | reduction (%) | 50 | -2,7 |
| 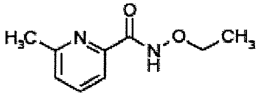 | CPE H3N2 | reduction (%) | 50 | -2,1 |
| 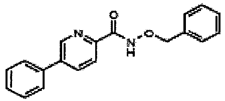 | CPE H3N2 | reduction (%) | 2 | -4 |
| 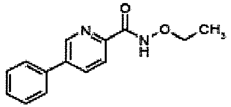 | CPE H3N2 | reduction (%) | 5 | -3 |
| 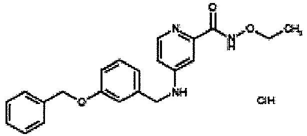 | CPE H3N2 | reduction (%) | 1 | 1,2 |
| 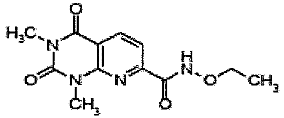 | CPE H3N2 | reduction (%) | 50 | -0,4 |
| 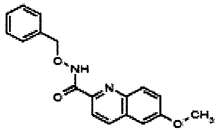 | CPE H3N2 | reduction (%) | 50 | -1,9 |
| 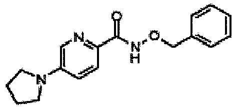 | CPE H3N2 | reduction (%) | 20 | -8,7 |
| 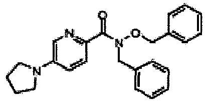 | CPE H3N2 | reduction (%) | 5 | -2,7 |
| 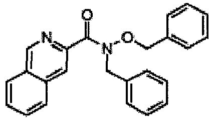 | CPEH3N2 | reduction (%) | 5 | 1,3 |
| 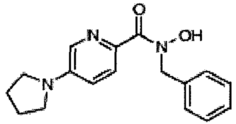 | CPE H3N2 | reduction (%) | 25 | 1 |
| | FRET | IC50 [µM] | | 4,3 |
| 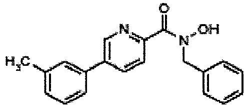 | CPE H3N2 | reduction (%) | 10 | 15,5 |
| 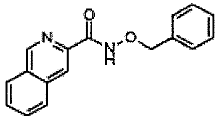 | CPE H3N2 | reduction (%) | 5 | -1,5 |
| 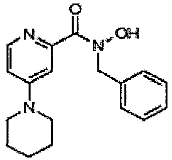 | CPE H3N2 | reduction (%) | 5 | 1,1 |
| | FRET | IC50 [µM] | | 1,4 |
| | FRET | IC50 [µM] | | 1,45 |
| 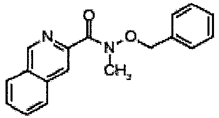 | CPE H3N2 | reduction (%) | 50 | 0,6 |
| 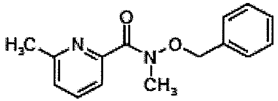 | CPE H3N2 | reduction (%) | 50 | -2,3 |
| 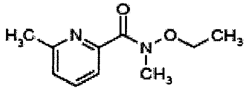 | CPE H3N2 | reduction (%) | 50 | -1,9 |
| 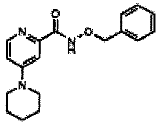 | CPE H3N2 | reduction (%) | 5 | 1 |
| 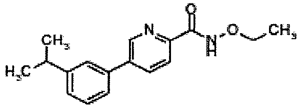 | CPE H3N2 | reduction (%) | 5 | |
| 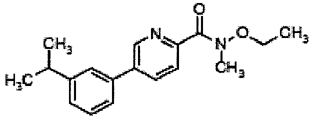 | CPE H3N2 | reduction (%) | 5 | |
| 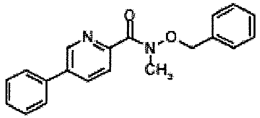 | CPE H3N2 | reduction (%) | 50 | -2,6 |
| 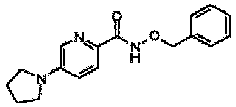 | CPE H3N2 | reduction (%) | 20 | -8,7 |
| 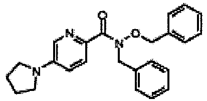 | CPE H3N2 | reduction (%) | 5 | -2,7 |
| 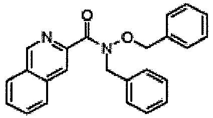 | CPE H3N2 | reduction (%) | 5 | 1,3 |
| 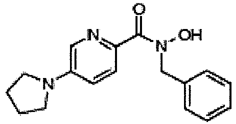 | CPEH3N2 | reduction (%) | 25 | 1 |
| | FRET | IC50 [µM] | | 4,3 |
| 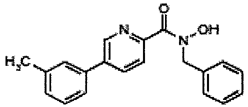 | CPE H3N2 | reduction (%) | 10 | 15,5 |
| 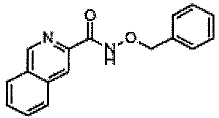 | CPE H3N2 | reduction (%) | 5 | -1,5 |
| 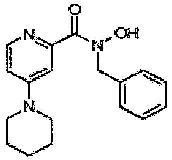 | CPE H3N2 | reduction (%) | 5 | 1,1 |
| | FRET | IC50 [µM] | | 1,4 |
| | FRET | IC50 [µM] | | 1,45 |
| 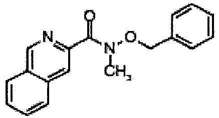 | CPEH3N2 | reduction (%) | 50 | 0,6 |
| 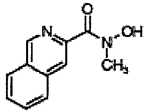 | CPE H3N2 | reduction (%) | 50 | -0,6 |
| 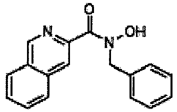 | FRET | IC50 [µM] | | 9,09 |
| | CPE H3N2 | reduction (%) | 50 | 34,2 |
| | FRET | IC50 [µM] | | 19 |
| 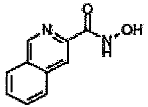 | FRET | IC50 [µM] | | 14,7 |
| | CPE H3N2 | reduction (%) | 50 | 94,3 |
| | FRET | IC50 [µM] | | 16 |
| | CPE H3N2 | IC50 [µM] | | 45 |
| 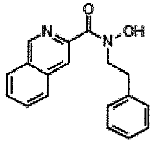 | CPE H3N2 | reduction (%) | 20 | -1,8 |
| 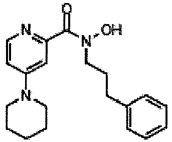 | CPE H3N2 | reduction (%) | 2 | 7,7 |
| 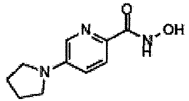 | FRET | IC50 [µM] | | 6,25 |
| | CPEH3N2 | reduction (%) | 50 | -4,3 |
| | FRET | IC50 [µM] | | 5,4 |
| 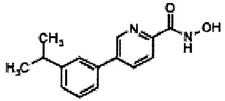 | CPE H3N2 | reduction (%) | 2 | -2,1 |
| 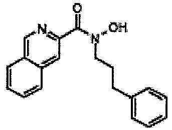 | CPE H3N2 | reduction (%) | 2 | 4,4 |
| 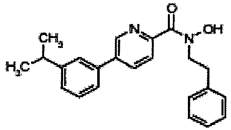 | CPE H3N2 | reduction (%) | 2 | 2,1 |
| 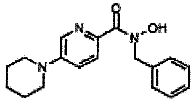 | FRET | IC50 [µM] | | 9,4 |
| | CPEH3N2 | reduction (%) | 5 | -3,4 |
| 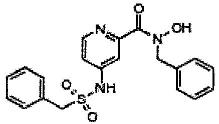 | FRET | IC50 [µM] | | 10,1 |
| | FRET | IC50 [µM] | | 1,7 |
| | CPE H3N2 | reduction (%) | 50 | 6,2 |
| 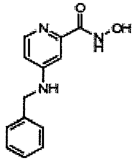 | FRET | IC50 [µM] | | 3,9 |
| | FRET | IC50 [µM] | | 6,4 |
| | CPE H3N2 | reduction (%) | 5 | -0,5 |
| 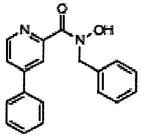 | FRET | IC50 [µM] | | 3,1 |
| | CPE H3N2 | reduction (%) | 10 | -9,3 |
| 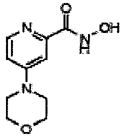 | FRET | IC50 [µM] | | 5,94 |
| | FRET | IC50 [µM] | | 7,1 |
| | CPE H3N2 | reduction (%) | 50 | -7,1 |
| 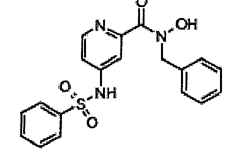 | FRET | IC50 [µM] | | 10 |
| | CPEH3N2 | reduction (%) | 50 | -5,2 |
| 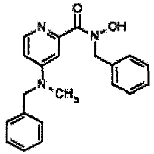 | FRET | IC50 [µM] | | 1,2 |
| | CPE H3N2 | reduction (%) | 5 | 3,8 |
| 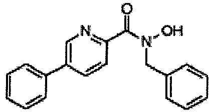 | CPE H3N2 | reduction (%) | 12 | 13,4 |
| 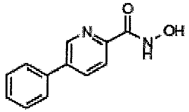 | FRET | IC50 [µM] | | 22 |
| | CPE H3N2 | reduction (%) | 12 | 21,7 |
| 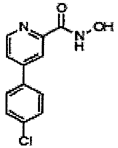 | FRET | IC50 [µM] | | 2,6 |
| | CPE H3N2 | reduction (%) | 5 | 26,6 |
| 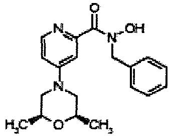 | FRET | IC50 [µM] | | 2,9 |
| | CPE H3N2 | reduction (%) | 50 | 10,5 |
| 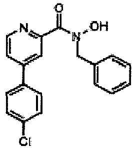 | FRET | IC50 [µM] | | 1,2 |
| | CPEH3N2 | reduction (%) | 2 | -6,7 |
| 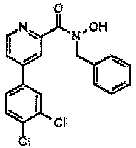 | FRET | IC50 [µM] | | 0,91 |
| | CPE H3N2 | reduction (%) | 5 | 8,1 |
| 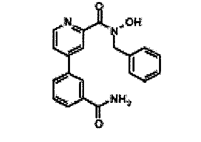 | FRET | IC50 [µM] | | 2 |
| | CPE H3N2 | reduction (%) | 5 | -2,7 |
| 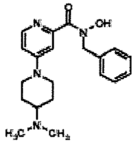 | FRET | IC50 [µM] | | 32 |
| | CPE H3N2 | reduction (%) | 50 | 12,3 |
| 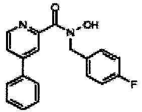 | FRET | IC50 [µM] | | 9,6 |
| | CPE H3N2 | reduction (%) | 1 | -3,2 |
| 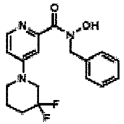 | FRET | IC50 [µM] | | 1,2 |
| | CPE H3N2 | reduction (%) | 50 | 8.9 |
| 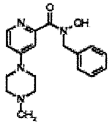 | FRET | IC50 [µM] | | 4,6 |
| | CPE H3N2 | reduction (%) | 50 | 2,4 |
| 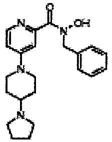 | CPE H3N2 | reduction (%) | 50 | 10,7 |
| 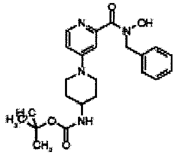 | CPE H3N2 | reduction (%) | 50 | 8,3 |
| 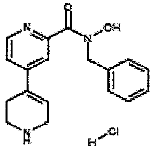 | FRET | IC50 [µM] | | 0,33 |
| | CPE H3N2 | reduction (%) | 50 | 0.4 |
| 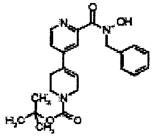 | CPEH3N2 | reduction (%) | 5 | 1,2 |
| 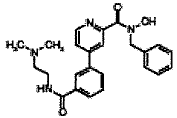 | FRET | IC50 [µM] | | 3,2 |
| | CPE H3N2 | reduction (%) | 50 | 8,9 |
| 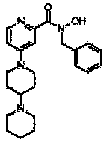 | CPE H3N2 | reduction (%) | 50 | 18,8 |
| 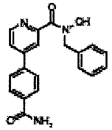 | FRET | IC50 [µM] | | 1 |
| | CPE H3N2 | reduction (%) | 5 | -2.5 |
| 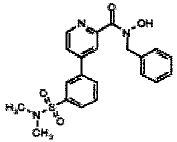 | FRET | IC50 [µM] | | 5,8 |
| | CPE H3N2 | reduction (%) | 5 | -1,8 |
| 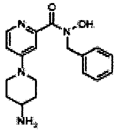 | FRET | IC50 [µM] | | 38 |
| | CPE H3N2 | reduction (%) | 50 | 3,2 |
| 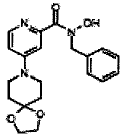 | FRET | IC50 [µM] | | 0,51 |
| | CPE H3N2 | reduction (%) | 50 | 9,5 |
| 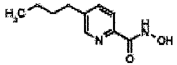 | FRET | IC50 [µM] | | 15 |
| 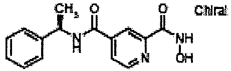 | FRET | **I**C50 [µM] | | 13 |
| 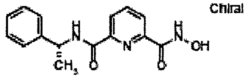 | FRET | IC50 [µM] | | 121 |
| 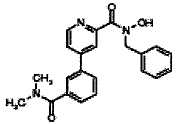 | CPE H3N2 | reduction (%) | 50 | 5.3 |
| 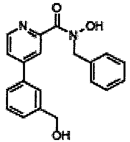 | CPE H3N2 | reduction (%) | 5 | 7.7 |
| | FRET | IC50 [µM] | | 2,6 |
| 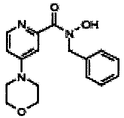 | CPE H3N2 | reduction (%) | 50 | -3,4 |
| | FRET | IC50 [µM] | | 2,3 |
| 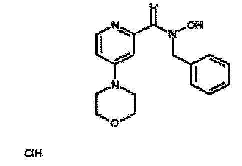 | CPE H3N2 | reduction (%) | 50 | 6,3 |
| | FRET | IC50 [µM] | | 1,9 |
| 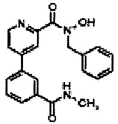 | CPE H3N2 | reduction (%) | 5 | -7.5 |
| | FRET | IC50 [µM] | | 3.2 |
| 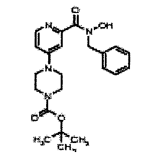 | CPE H3N2 | reduction (%) | 50 | 34 |
| 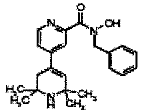 | CPE H3N2 | reduction (%) | 50 | 2,8 |
| 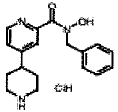 | FRET | IC50 [µM] | | 2,3 |
| | CPE H3N2 | reduction (%) | 50 | -1,4 |
| 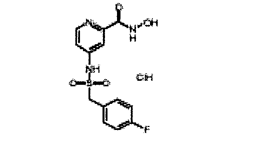 | FRET | IC50 [µM] | | 0,73 |
| | CPE H3N2 | reduction (%) | 50 | -1.4 |
| 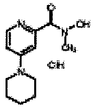 | CPE H3N2 | reduction (%) | 50 | -0,9 |
| 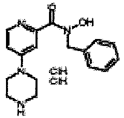 | FRET | IC50 [µM] | | 3.5 |
| | CPEH3N2 | reduction (%) | 50 | -4,5 |
| 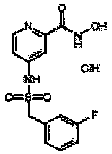 | FRET | IC50 [µM] | | 0,67 |
| | CPE H3N2 | reduction (%) | 50 | -2,4 |
| 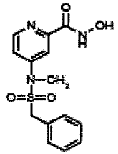 | FRET | IC50 [µM] | | 32 |
| | CPE H3N2 | reduction (%) | 5 | -1,7 |
| 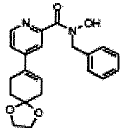 | FRET | IC50 [µM] | | 2 |
| | CPE H3N2 | reduction (%) | 50 | 5.4 |
| 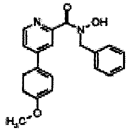 | FRET | IC50 [µM] | | 2.4 |
| | CPE H3N2 | reduction (%) | 2 | -2,5 |
| 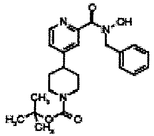 | CPE H3N2 | reduction (%) | 20 | 31,3 |
| 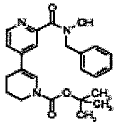 | CPE H3N2 | reduction (%) | 2 | 3,9 |
| 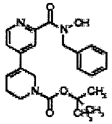 | CPE H3N2 | reduction (%) | 5 | 10,2 |
| 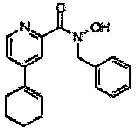 | FRET | IC50 [µM] | | 3.6 |
| | CPE H3N2 | reduction (%) | 1 | 3.4 |
| 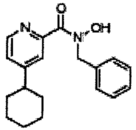 | FRET | IC50 [µM] | | 3 |
| | CPEH3N2 | reduction (%) | 5 | -0.5 |
| 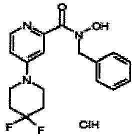 | FRET | IC50 [µM] | | 2,9 |
| | CPE H3N2 | reduction (%) | 50 | -3,2 |
| 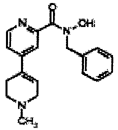 | FRET | IC50 [µM] | | 7.7 |
| | CPE H3N2 | reduction (%) | 50 | 10.6 |
| 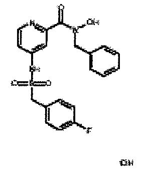 | FRET | IC50 [µM] | | 2 |
| | CPE H3N2 | reduction (%) | 50 | -1.1 |
| 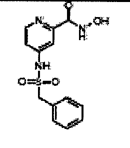 | FRET | IC50 [µM] | | 3 |
| | CPE H3N2 | reduction (%) | 50 | 5.1 |
| 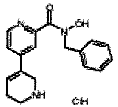 | CPE H3N2 | reduction (%) | 50 | 9.5 |
| 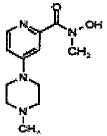 | CPE H3N2 | reduction (%) | 50 | 2.6 |
| 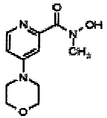 | CPE H3N2 | reduction (%) | 50 | 5.7 |
| 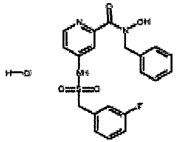 | FRET | IC50 [µM] | | 1.9 |
| | CPE H3N2 | reduction (%) | 50 | 9,6 |
| 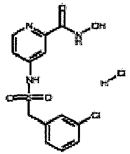 | FRET | IC50 [µM] | | 0,2 |
| | CPE H3N2 | reduction (%) | 50 | 5.9 |
| 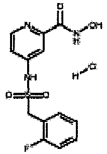 | FRET | IC50 [µM] | | 0.76 |
| | CPE H3N2 | reduction (%) | 50 | -0,5 |
| 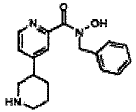 | CPE H3N2 | reduction (%) | 50 | 6,1 |
| 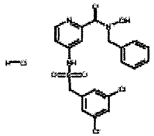 | FRET | IC50 [µM] | | 0.4 |
| | CPE H3N2 | reduction (%) | 20 | 26.9 |
| 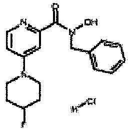 | FRET | IC50 [µM] | | 2.2 |
| | CPE H3N2 | reduction (%) | 50 | 34 |
| 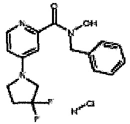 | FRET | IC50 [µM] | | 1.8 |
| | CPE H3N2 | reduction (%) | 20 | 4.6 |
| 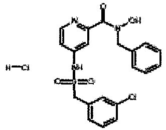 | FRET | IC50 [µM] | | 0.33 |
| | CPE H3N2 | reduction (%) | 50 | 9.5 |
| 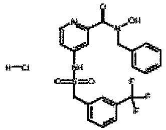 | FRET | IC50 [µM] | | 2 |
| | CPE H3N2 | reduction (%) | 50 | 22,4 |
| 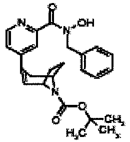 | CPE H3N2 | reduction (%) | 2 | -5.3 |
| 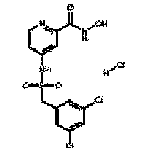 | FRET | IC50 [µM] | | 0.58 |
| | CPE H3N2 | reduction (%) | 50 | 9.3 |
| 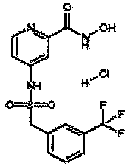 | FRET | IC50 [µM] | | 1.8 |
| | CPE H3N2 | reduction (%) | 50 | -5,6 |
| 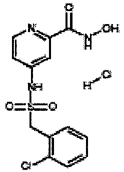 | FRET | IC50 [µM] | | 0,83 |
| | CPE H3N2 | reduction (%) | 50 | 1.5 |
| 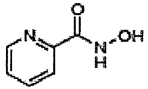 | FRET | IC50 [µM] | | 75 |
| | CPE H3N2 | reduction (%) | 50 | 3,15 |
| 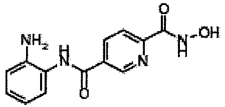 | CPE H3N2 | reduction (%) | 50 | -5.53 |
| 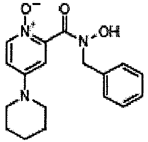 | FRET | IC50 [µM] | | 23 |
| | CPE H3N2 | reduction (%) | 5 | -1.83 |
| 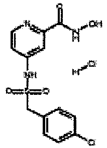 | FRET | IC50 [µM] | | 1.1 |
| | CPE H3N2 | reduction (%) | 50 | -3.98 |
| 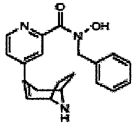 | FRET | IC50 [µM] | | 3.5 |
| | CPE H3N2 | reduction (%) | 50 | 5,49 |
| 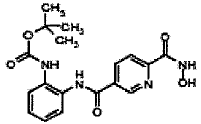 | FRET | IC50 [µM] | | |
| 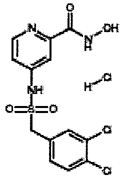 | FRET | IC50 [µM] | | 3.2 |
| | CPE H3N2 | reduction (%) | 50 | 53.43 |
| 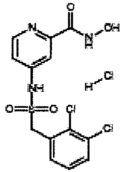 | FRET | IC50 [µM] | | 2 |
| | CPE H3N2 | IC50 [µM] | | |
| | CPE H3N2 | reduction (%) | 50 | 60.07 |
| 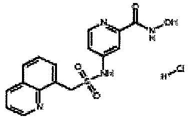 | CPE H3N2 | reduction (%) | 50 | 24.39 |
| | CPE H3N2 | IC50 [µM] | | 78 |
| | FRET | IC50 [µM] | | 0.28 |
| 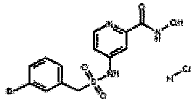 | CPE H3N2 | reduction (%) | 50 | 2.29 |
| | FRET | IC50 [µM] | | 1.8 |

### Compounds having the general formula (II)

### Key Intermediate I

### 5-Bromo-2-tert-butoxycarbonylamino-4-methyl-thiophene-3-carboxylic acid ethyl ester

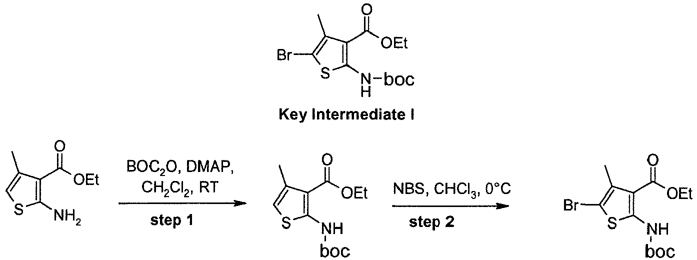

### Step 1:

To a solution of 2-amino-4-methyl-thiophene-3-carboxylic acid ethyl ester (25.0 g, 135 mmol, 1 eq) in dichloromethane (80 mL) were added di-tert-butyl dicarbonate (48.0 g, 220 mmol, 1.6 eq) and 4-dimethylaminopyridine (1.6 g, 13.5 mmol, 0.1 eq). The mixture was stirred at room temperature until completion of the reaction. The solvent was then evaporated and the residue was purified by flash chromatography using cyclohexane and ethyl acetate (100/0 to 90/10) to afford 2-tert-butoxycarbonylamino-4-methyl-thiophene-3-carboxylic acid ethyl ester as a white solid (18.8 g, 49% yield).

### Step 2:

At 0 °C, to a solution of 2-tert-butoxycarbonylamino-4-methyl-thiophene-3-carboxylic acid ethyl ester (10.2 g, 35.9 mmol, 1 eq) in chloroform (40 mL) was added N-bromosuccinimide (6.4 g, 35.9 mmol, 1 eq). The mixture was stirred at 0 °C during 2 h and the solvent was evaporated. The residue was purified by flash chromatography using cyclohexane and ethyl acetate (100/0 to 70/30) to afford the expected compound as a white solid (11.9 g, 91% yield).

### Key Intermediate II

### 5-Methyl-2-methylsulfanyl-6-phenyl-3H-thieno[2,3-d]pyrimidin-4-one

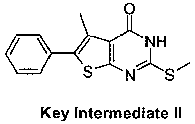

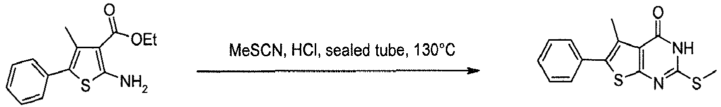

2-Amino-4-methyl-5-phenyl-thiophene-3-carboxylic acid ethyl ester (10.0 g, 38.3 mmol, 1 eq), methyl thiocyanate (2.8 g, 38.3 mmol, 1 eq) and concentrated hydrochloric acid (1.4 mL, 38.3 mmol, 1 eq) were heated in a sealed tube at 130 °C during 18 h. After cooling, the precipitate was filtered, rinsed with ethanol and dried to afford the expected compound as a yellow solid (7.7 g, 70% yield).

### Key Intermediate III

### 2-(2-Amino-ethylamino)-5-methyl-6-phenyl-3H-thieno[2,3-d]pyrimidin-4-one

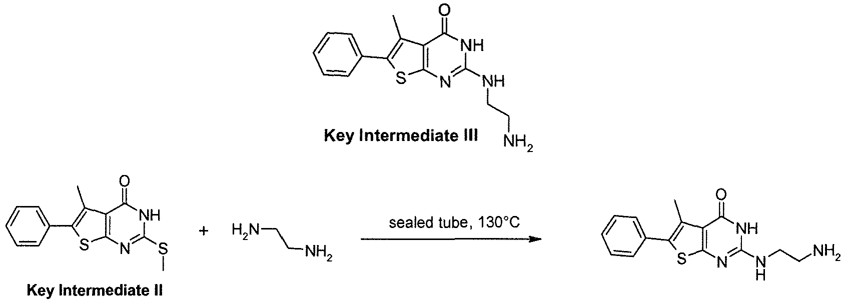

5-Methyl-2-methylsulfanyl-6-phenyl-3H-thieno[2,3-d]pyrimidin-4-one **(Key Intermediate II)** (1.2 g, 4.2 mmol, 1 eq) was solubilized in ethylenediamine (3 mL) and the solution was heated in a sealed tube at 130 °C during 18 h. After cooling down, the yellow suspension was filtered. The precipitate was rinsed with dichloromethane and diethyl ether and dried in vacuo to afford the expected compound as a white powder (550 mg, 44 % yield).

### General Procedure A

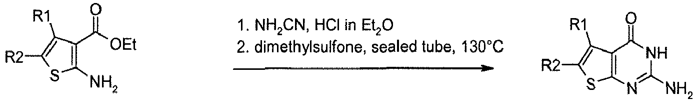

At 0 °C, cyanamide (1.0 mmol, 1.5 eq) was added to a 2M solution of hydrogen chloride in diethyl ether (1.0 mL, 3 eq). After stirring for 15 min, the suspension was filtered. The resulting white solid was added in a sealed tube to 2-amino-thiophene-3-carboxylic acid ethyl ester (0.7 mmol, 1 eq) and dimethylsulfone (250 mg). The mixture was heated at 130 °C during 2 h. After cooling, the residue was dissolved in methanol and a 7N solution of ammonia in methanol (10 mL) was added. The solvent was then evaporated and the solid obtained was washed with dichloromethane (2 x 10 mL) and water (2 x 10 mL) to afford the expected compound (5% to 90% yield).

### Example 1:

### 2-Amino-6-isopropyl-3H-thieno[2,3-d]pyrimidin-4-one

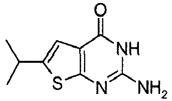

The expected compound was obtained according to **general procedure A** using commercially available 2-amino-5-isopropyl-thiophene-3-carboxylic acid methyl ester.

The expected compound was isolated as a beige powder.
MS: 210.0
Mp: 347 °C - 349 °C

### Reference Example 2:

### 2-Amino-6-phenyl-3H-thieno[2,3-d]pyrimidin-4-one

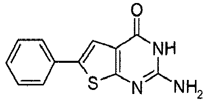

The expected compound was obtained according to **general procedure A** using commercially available 2-amino-5-phenyl-thiophene-3-carboxylic acid methyl ester. The expected compound was isolated as a grey solid.
MS: 244.0
Mp >360 °C

### Reference Example 3:

### 2-Amino-5-methyl-6-phenyl-3H-thieno[2,3-d]pyrimidin-4-one

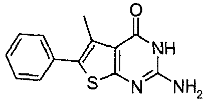

The expected compound was obtained according to **general procedure A** using commercially available 2-amino-4-methyl-5-phenyl-thiophene-3-carboxylic acid ethyl ester. The expected compound was isolated as a beige powder.
MS: 258.1
Mp: 356 °C - 358 °C

### Example 4:

### 2-Amino-5-(4-fluoro-phenyl)-6-methyl-3H-thieno[2,3-d]pyrimidin-4-one

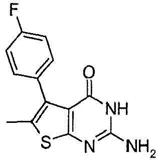

The expected compound was obtained according to **general procedure A** using commercially available 2-amino-4-(4-fluoro-phenyl)-5-methyl-thiophene-3-carboxylic acid methyl ester. The expected compound was isolated as a grey solid.
MS: 276.1
Mp: 360 °C - 362 °C

### Example 5:

### 2-Amino-6-benzyl-3H-thieno[2,3-d]pyrimidin-4-one

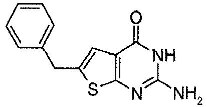

The expected compound was obtained according to **general procedure A** using commercially available 2-amino-5-benzyl-thiophene-3-carboxylic acid ethyl ester. The expected compound was isolated as a green solid.
MS: 258.1
Mp: 294 °C - 296 °C

### Example 6:

### 2-Amino-6-(1-phenyl-ethyl)-3H-thieno[2,3-d]pyrimidin-4-one

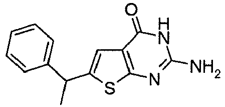

The expected compound was obtained according to **general procedure A** using commercially available 2-amino-5-(1-phenyl-ethyl)-thiophene-3-carboxylic acid methyl ester. The expected compound was isolated as a grey powder.
MS: 272.0
Mp: 260 °C - 270 °C

### Example 7:

### 2-Amino-5-methyl-4-oxo-3,4-dihydro-thieno[2,3-d]pyrimidine-6-carboxylic acid phenylamide

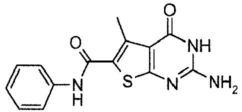

The expected compound was obtained according to **general procedure A** using commercially available 2-amino-4-methyl-5-phenylcarbamoyl-thiophene-3-carboxylic acid ethyl ester. The expected compound was isolated as a yellow powder.
MS: 301.0
Mp: decomposes at 290 °C - 296 °C

### General Procedure B

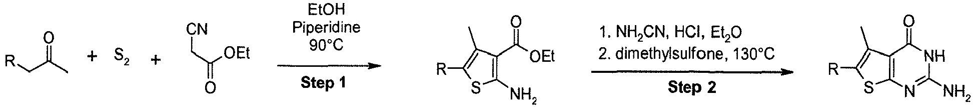

### Step 1:

Propan-2-one (28.0 mmol, 1 eq), sulfur (900 mg, 28.0 mmol, 1 eq), ethyl cyanoacetate (3.0 mL, 28.0 mmol, 1 eq) and a catalytic amount of piperidine were put in suspension in ethanol (15 mL) and were heated in a sealed tube at 90 °C during 18 h. The reaction mixture was then evaporated and the crude residue was purified by flash chromatography using cyclohexane and ethyl acetate (100/0 to 0/100) to afford 2-amino-thiophene-3-carboxylic acid ethyl ester (6% to 95% yield).

### Step 2:

At 0 °C, cyanamide (1.0 mmol, 1.5 eq) was added to a 2M solution of hydrogen chloride in diethyl ether (1.0 mL, 3 eq). After stirring for 15 min, the suspension was filtered. The resulting white solid was added in a sealed tube to 2-amino-thiophene-3-carboxylic acid ethyl ester obtained in step 1 (0.7 mmol, 1 eq) and dimethylsulfone (250 mg). The mixture was heated at 130 °C during 2 h. After cooling, the residue was dissolved in methanol and a 7N solution of ammonia in methanol (10 mL) was added. The solvent was then evaporated and the solid obtained was washed with dichloromethane (2 x 10 mL) and water (2 x 10 mL) to afford the expected compound (5% to 90% yield).

### Example 8:

### 2-Amino-6-(4-chloro-phenyl)-5-methyl-3H-thieno[2,3-d]pyrimidin-4-one

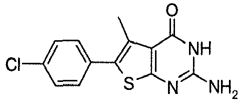

The expected compound was obtained according to **general procedure B** using 1-(4-chloro-phenyl)-propan-2-one. The expected compound was isolated as a grey powder. MS: 292.0
Mp: decomposes at 351 °C

### Example 9:

### 2-Amino-6-(3-chloro-phenyl)-5-methyl-3H-thieno[2,3-d]pyrimidin-4-one

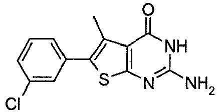

The expected compound was obtained according to **general procedure B** using 1-(3-chloro-phenyl)-propan-2-one. The expected compound was isolated as a white powder.
MS: 292.1
Mp: decomposes at 265 °C

### Example 10:

### 2-Amino-5-methyl-6-p-tolyl-3H-thieno[2,3-d]pyrimidin-4-one

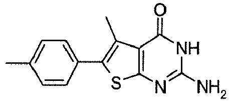

The expected compound was obtained according to **general procedure B** using 1-p-tolyl-propan-2-one. The expected compound was isolated as a white powder.
MS: 272.1
Mp: decomposes at 330 °C

### Example 11:

### 2-Amino-6-(4-methoxy-phenyl)-5-methyl-3H-thieno[2,3-d]pyrimidin-4-one

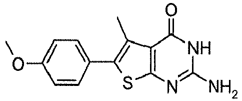

The expected compound was obtained according to **general procedure B** using 1-(4-methoxy-phenyl)-propan-2-one. The expected compound was isolated as a white powder.
MS: 288.1
Mp: decomposes at 311 °C

### Example 12:

### 2-Amino-5-methyl-6-(3-trifluoromethyl-phenyl)-3H-thieno[2,3-d]pyrimidin-4-one

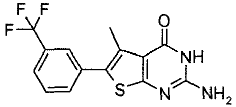

The expected compound was obtained according to **general procedure B** using 1-(3-trifluoromethyl-phenyl)-propan-2-one. The expected compound was isolated as a white powder.
MS: 326.1
Mp: decomposes at 345 °C

### Example 13:

### 2-Amino-5-methyl-6-pyridin-4-yl-3H-thieno[2,3-d]pyrimidin-4-one

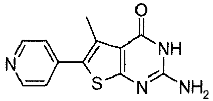

The expected compound was obtained according to **general procedure B** using 1-pyridin-4-yl-propan-2-one. The expected compound was isolated as a yellow powder.
MS: 259.1
Mp: decomposes at 355 °C

### Example 14:

### 2-Amino-5-methyl-6-pyridin-3-yl-3H-thieno[2,3-d]pyrimidin-4-one

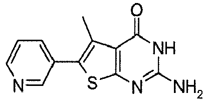

The expected compound was obtained according to **general procedure B u**sing 1-pyridin-3-yl-propan-2-one. The expected compound was isolated as a yellow powder.
MS: 259.0
Mp: 280 °C - 290 °C

### Example 15:

### 2-Amino-5-methyl-6-pyrazin-2-yl-3H-thieno[2,3-d]pyrimidin-4-one

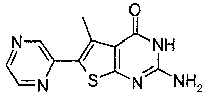

The expected compound was obtained according to **general procedure B** using 1-pyrazin-2-yl-propan-2-one. The expected compound was isolated as an orange powder.
MS: 260.0
Mp: 280 °C - 300 °C

### Example 16:

### 2-Amino-6-benzyl-5-methyl-3H-thieno[2,3-d]pyrimidin-4-one

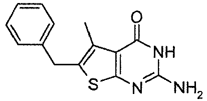

The expected compound was obtained according to **general procedure B** using 4-phenyl-butan-2-one. The expected compound was isolated as a white powder.
MS: 272.1
Mp: 292 °C - 294 °C

### Example 17:

### 2-Amino-6-(4-chloro-benzyl)-5-methyl-3H-thieno[2,3-d]pyrimidin-4-one

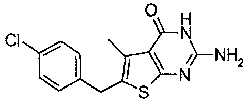

The expected compound was obtained according to **general procedure B** using 4-(4-chloro-phenyl)-butan-2-one. The expected compound was isolated as a white powder.
MS: 306.1
Mp: 300 °C - 320 °C

### General Procedure C

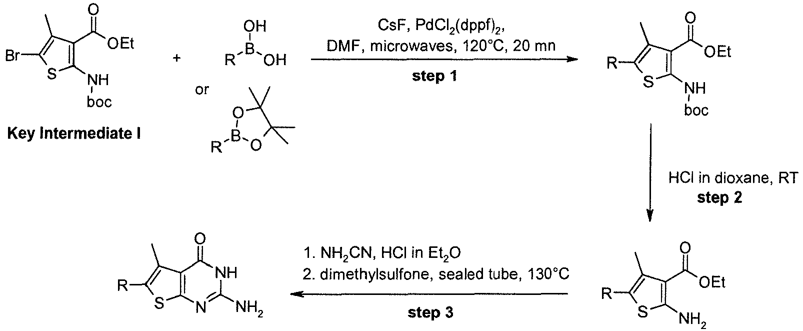

### Step 1:

To a degassed solution of 5-bromo-2-tert-butoxycarbonylamino-4-methyl-thiophene-3-carboxylic acid ethyl ester (**Key Intermediate I**) (200 mg, 0.6 mmol, 1 eq) and boronic acid or ester (1.8 mmol, 3 eq) in dry dimethylformamide (4 mL) were added cesium fluoride (183 mg, 1.2 mmol, 2.2 eq) and dichloro[1,1'-bis(diphenylphosphino)ferrocene]palladium (0.12 mmol, 90 mg, 0.2 eq). The mixture was stirred at 120 °C under microwave radiation during 20 min. After cooling, the mixture was filtered over a short pad of celite and absorbed on silica gel to be purified by flash chromatography (30% to 95% yield).

### Step 2:

The compound from step 1 (2.4 mmol, 1 eq) was solubilized in a 4N solution of hydrogen chloride in dioxane (10 mL) and the mixture was stirred at room temperature during 18 h. The mixture was then concentrated and the residue was taken in dichloromethane (10 mL) and washed with a saturated solution of sodium bicarbonate (3 x 10 mL). The organic layers were dried over magnesium sulfate, filtered and evaporated in vacuo. The crude residue was purified by flash chromatography to afford the amino ester (35% to quantitative yield).

### Step 3:

At 0 °C, cyanamide (1.0 mmol, 1.5 eq) was added to a 2M solution of hydrogen chloride in diethyl ether (1.0 mL, 3 eq). After stirring for 15 min, the suspension was filtered. The resulting white solid was added in a sealed tube to 2-amino-thiophene-3-carboxylic acid ethyl ester (0.7 mmol, 1 eq) and dimethylsulfone (250 mg). The mixture was heated at 130 °C during 2 h. After cooling, the residue was dissolved in methanol and a 7N solution of ammonia in methanol (10 mL) was added. The solvent was then evaporated and the solid obtained was washed with dichloromethane (2 x 10 mL) and water (2 x 10 mL) to afford the expected compound (5% to 90% yield).

### Example 18:

### 2-Amino-5-methyl-6-m-tolyl-3H-thieno [2,3-d]pyrimidin-4-one

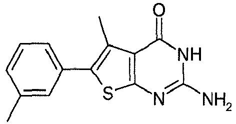

The expected compound was obtained according to **general procedure C** using 3-methylbenzeneboronic acid. The expected compound was isolated as a white powder.
MS: 272.1
Mp: decomposes at 330 °C - 338 °C

### Example 19:

### 2-Amino-6-(2-chloro-phenyl)-5-methyl-3H-thieno[2,3-d]pyrimidin-4-one

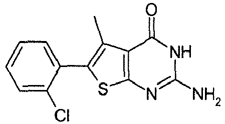

The expected compound was obtained according to **general procedure C** using 2-chlorobenzeneboronic acid. The expected compound was isolated as a pink powder.
MS: 292.1
Mp: 334 °C - 336 °C

### Example 20:

### 2-Amino-6-(2-fluoro-phenyl)-5-methyl-3H-thieno[2,3-d]pyrimidin-4-one

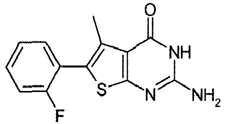

The expected compound was obtained according to **general procedure C** using 2-fluorobenzeneboronic acid. The expected compound was isolated as a beige powder.
MS: 271.1
Mp: 325 °C - 330 °C

### Example 21:

### 2-Amino-6-(4-fluoro-phenyl)-5-methyl-3H-thieno[2,3-d]pyrimidin-4-one

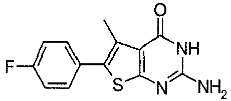

The expected compound was obtained according to **general procedure C** using 4-fluorobenzeneboronic acid. The expected compound was isolated as a grey powder.
MS: 276.0
Mp: 325 °C - 335 °C

### Example 22:

### 2-Amino-6-(3-fluoro-phenyl)-5-methyl-3H-thieno[2,3-d]pyrimidin-4-one

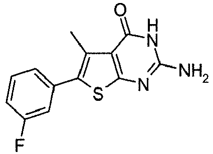

The expected compound was obtained according to **general procedure C** using 3-fluorobenzeneboronic acid. The expected compound was isolated as a purple powder.
MS: 276.0
Mp: 310 °C - 330 °C

### Example 23:

### 2-Amino-6-(2,4-difluoro-phenyl)-5-methyl-3H-thieno[2,3-d]pyrimidin-4-one

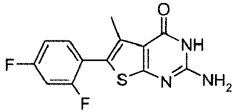

The expected compound was obtained according to **general procedure C** using 2,4-difluorophenylboronic acid. The expected compound was isolated as a purple powder.
MS: 294.1
Mp: 330 °C - 350 °C

### Example 24:

### 2-Amino-6-(3-chloro-2-fluoro-phenyl)-5-methyl-3H-thieno[2,3-d]pyrimidin-4-one

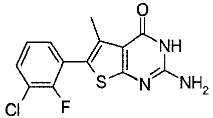

The expected compound was obtained according to **general procedure C** using 3-chloro-2-fluorophenylboronic acid. The expected compound was isolated as a white powder.
MS: 310.1
Mp: 330 °C - 350 °C

### Example 25:

### 2-Amino-6-(4-chloro-2-fluoro-phenyl)-5-methyl-3H-thieno[2,3-d]pyrimidin-4-one

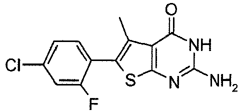

The expected compound was obtained according to **general procedure C** using 4-chloro-2-fluorobenzeneboronic acid. The expected compound was isolated as a white powder.
MS: 310.0
Mp: 320 °C - 340 °C

### Example 26:

### 2-Amino-6-(3-chloro-2,6-difluoro-phenyl)-5-methyl-3H-thieno[2,3-d]pyrimidin-4-one

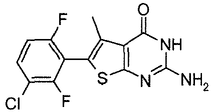

The expected compound was obtained according to **general procedure C** using 3-chloro-2,6-difluorophenylboronic acid. The expected compound was isolated as a white powder.
MS: 328.1
Mp: 330 °C - 350 °C

### Example 27:

### 2-Amino-6-(4-chloro-3-fluoro-phenyl)-5-methyl-3H-thieno[2,3-d]pyrimidin-4-one

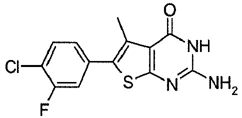

The expected compound was obtained according to **general procedure C** using 4-chloro-3-fluorophenylboronic acid. The expected compound was isolated as a beige powder.
MS: 310.1
Mp: 350 °C - 370 °C

### Example 28:

### 2-Amino-6-(4-chloro-3-methoxy-phenyl)-5-methyl-3H-thieno[2,3-d]pyrimidin-4-one

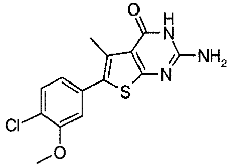

The expected compound was obtained according to **general procedure C** using 4-chloro-3-methoxyphenylboronic acid. The expected compound was isolated as a beige powder.
MS: 322.1
Mp: 312 °C - 322 °C

### Example 29:

### 2-Amino-6-(4-chloro-3-methyl-phenyl)-5-methyl-3H-thieno[2,3-d]pyrimidin-4-one

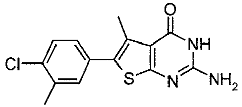

The expected compound was obtained according to **general procedure C** using 4-chloro-3-methylphenylboronic acid. The expected compound was isolated as a white powder.
MS: 306.1
Mp: 330 °C - 350 °C

### Example 30:

### 2-Amino-6-(4-chloro-3-hydroxy-phenyl)-5-methyl-3H-thieno[2,3-d]pyrimidin-4-one

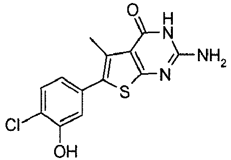

The expected compound was obtained according to **general procedure C** using (4-chloro-3-hydroxyphenyl)boronic acid. The expected compound was isolated as a beige powder.
MS: 308.1
Mp > 350 °C

### Example 31:

### 2-Amino-6-(4-chloro-3-trifluoromethyl-phenyl)-5-methyl-3H-thieno(2,3-d]pyrimidin-4-one

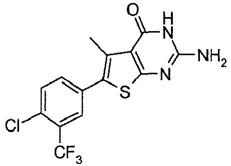

The expected compound was obtained according to **general procedure C** using 4-chloro-3-trifluoromethylphenylboronic acid. The expected compound was isolated as a white powder.
MS: 360.2
Mp > 350 °C

### Example 32:

### 5-(2-Amino-5-methyl-4-oxo-3,4-dihydro-thieno[2,3-d]pyrimidin-6-yl)-2-chloro-N,N-dimethyl-benzamide

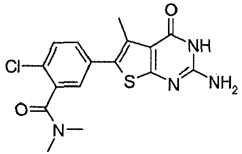

The expected compound was obtained according to **general procedure C** using 4-chloro-3-(dimethylaminocarbonyl)phenylboronic acid. The expected compound was isolated as a white powder.
MS: 363.1
Mp: 300 °C - 320 °C

### Example 33:

### 3-(2-Amino-5-methyl-4-oxo-3,4-dihydro-thieno[2,3-d]pyrimidin-6-yl)-benzonitrile

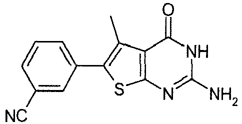

The expected compound was obtained according to **general procedure C** using 3-cyanophenylboronic acid. The expected compound was isolated as a beige powder.
MS: 283.1
Mp > 350 °C

### Example 34:

### 5-(2-Amino-5-methyl-4-oxo-3,4-dihydro-thieno[2,3-d]pyrimidin-6-yl)-2-chloro-benzonitrile

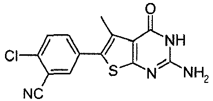

The expected compound was obtained according to **general procedure C** using 4-chloro-3-cyanophenylboronic acid. The expected compound was isolated as a beige powder.
MS: 317.0
Mp > 360 °C

### Example 35:

### 3-(2-Amino-5-methyl-4-oxo-3,4-dihydro-thieno[2,3-d]pyrimidin-6-yl)-4-chloro-benzonitrile

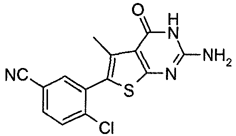

The expected compound was obtained according to **general procedure C** using 2-chloro-5-cyanophenylboronic acid. The expected compound was isolated as a beige powder.
MS: 317.1
Mp > 350 °C

### Example 36:

### 5-(2-Amino-5-methyl-4-oxo-3,4-dihydro-thieno[2,3-d]pyrimidin-6-yl)-2-fluoro-benzonitrile

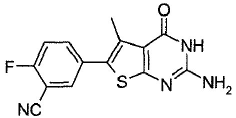

The expected compound was obtained according to **general procedure C** using 3-cyano-4-fluorophenylboronic acid. The expected compound was isolated as a white powder.
MS: 301.1
Mp: 330 °C - 350 °C

### Example 37:

### 3-(2-Amino-5-methyl-4-oxo-3,4-dihydro-thieno[2,3-d]pyrimidin-6-yl)-4-fluoro-benzonitrile

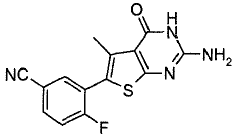

The expected compound was obtained according to **general procedure C** using 5-cyano-2-fluorophenylboronic acid. The expected compound was isolated as a beige powder.
MS: 301.0
Mp: 332 °C - 336 °C

### Example 38:

### 3-(2-Amino-5-methyl-4-oxo-3,4-dihydro-thieno[2,3-d]pyrimidin-6-yl)-2-fluorobenzonitrile

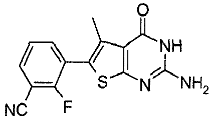

The expected compound was obtained according to **general procedure C** using 3-cyano-2-fluorophenylboronic acid. The expected compound was isolated as a white powder.
MS: 301.0
Mp: 350 °C - 370 °C

### Example 39:

### 3-(2-Amino-5-methyl-4-oxo-3,4-dihydro-thieno[2,3-d]pyrimidin-6-yl)-2,6-difluoro-benzonitrile

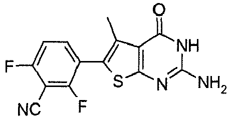

The expected compound was obtained according to **general procedure C** using 2,4-difluoro-3-cyanophenylboronic acid. The expected compound was isolated as a grey powder.
MS: 319.0
Mp: 360 °C - 380 °C

### Example 40:

### 2-Amino-6-(3,5-bis-trifluoromethyl-phenyl)-5-methyl-3H-thieno[2,3-d]pyrimidin-4-one

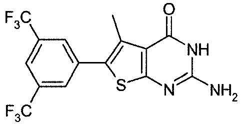

The expected compound was obtained according to **general procedure C** using 3,5-bis(trifluoromethyl)benzeneboronic acid. The expected compound was isolated as a white powder.
MS: 394.1
Mp: 344 °C - 347 °C

### Example 41:

### 2-Amino-6-(4-fluoro-3-trifluoromethyl-phenyl)-5-methyl-3H-thieno[2,3-d]pyrimidin-4-one

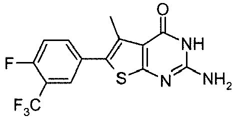

The expected compound was obtained according to **general procedure C** using 4-fluoro-3-trifluoromethylphenylboronic acid. The expected compound was isolated as a white powder.
MS: 343.1
Mp: 310 °C - 330 °C

### Example 42:

### 2-Amino-6-(3-dimethylaminomethyl-phenyl)-5-methyl-3H-thieno[2,3-d]pyrimidin-4-one

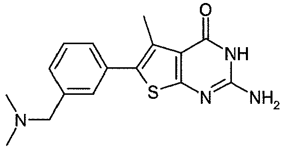

The expected compound was obtained according to **general procedure C** using 3-(N,N-dimethylamino)methylphenylboronic acid, pinacol ester, hydrochloride salt. The expected compound was isolated as a beige powder.
MS: 315.1
Mp: 207 °C - 212 °C

### Example 43:

### 2-Amino-6-(5-dimethylaminomethyl-2-fluoro-phenyl)-5-methyl-3H-thieno[2,3-d]-pyrimidin-4-one

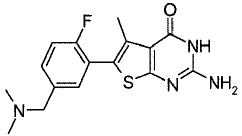

The expected compound was obtained according to **general procedure C** using 2-fluoro-5-(dimethylaminomethyl)phenylboronic acid pinacol ester. The expected compound was isolated as a white powder.
MS: 333.2
Mp: 230 °C - 250 °C

### Example 44:

### 2-Amino-6-(6-chloro-pyridin-3-yl)-5-methyl-3H-thieno[2,3-d]pyrimidin-4-one

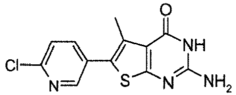

The expected compound was obtained according to **general procedure C** using 2-chloropyridine-5-boronic acid. The expected compound was isolated as a yellow powder.
MS: 293.1
Mp: 230 °C - 250 °C

### Example 45:

### 2-Amino-6-(2-chloro-3-fluoro-pyridin-4-yl)-5-methyl-3H-thieno[2,3-d]pyrimidin-4-one

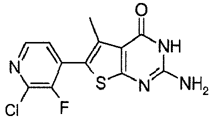

The expected compound was obtained according to **general procedure C** using 2-chloro-3-fluoropyridine-4-boronic acid. The expected compound was isolated as a yellow powder.
MS: 311.1
Mp: 330 °C - 350 °C

### Example 46:

### 2-Amino-6-(2,6-difluoro-pyridin-3-yl)-5-methyl-3H-thieno[2,3-d]pyrimidin-4-one

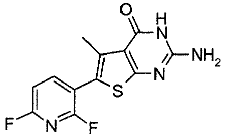

The expected compound was obtained according to **general procedure C** using 2,6-difluoropyridine-3-boronic acid. The expected compound was isolated as a beige powder.
MS: 295.0
Mp: 330 °C - 335 °C

### Example 47:

### 2-Amino-5-methyl-6-(2-trifluoromethyl-pyridin-4-yl)-3H-thieno[2,3-d]pyrimidin-4-one

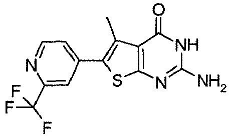

The expected compound was obtained according to **general procedure C** using 2-(trifluoromethyl)pyridine-4-boronic acid. The expected compound was isolated as a yellow powder.
MS: 327.0
Mp: 335 °C - 355 °C

### Example 48:

### 2-Amino-5-methyl-6-(2-methyl-2H-imidazol-4-yl)-3H-thieno[2,3-d]pyrimidin-4-one

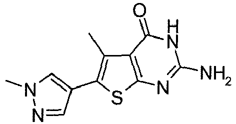

The expected compound was obtained according to **general procedure C** using 1-methyl-4-(4,4,5,5-tetramethyl-1,3,2-dioxaborolan-2-yl)-1H-pyrazole. The expected compound was isolated as a white powder.
MS: 262.0
Mp: 335 °C - 345 °C

### Example 49:

### 2-Amino-5-methyl-6-(1,2,3,6-tetrahydro-pyridin-4-yl)-3H-thieno[2,3-d]pyrimidin-4-one

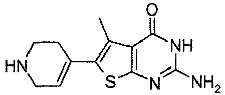

The expected compound was obtained according to **general procedure C** using N-Boc-1,2,3,6-tetrahydropyridine-4-boronic acid pinacol ester. The expected compound was isolated as an orange powder.
MS: 263.1
Mp: 290 °C - 310 °C

### Example 50:

### 3-(2-Amino-5-methyl-4-oxo-3,4-dihydro-thieno[2,3-d]pyrimidin-6-yl)-benzamide

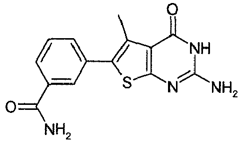

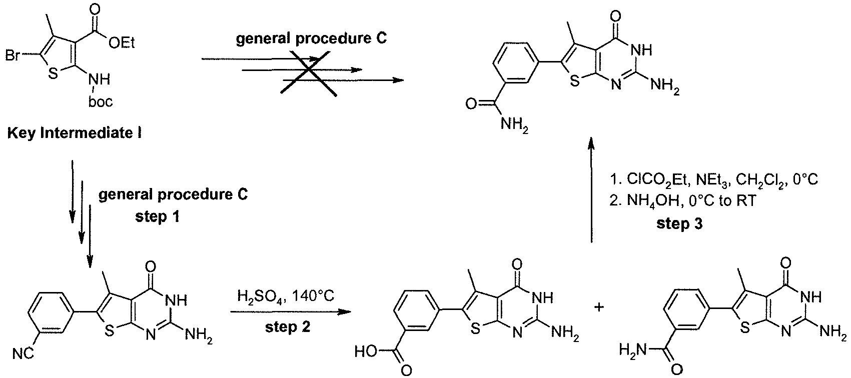

### Step 1:

The procedure to obtain the expected compound began with **general procedure C** using 3-carbamoylphenylboronic acid. After cyclisation, 3-(2-amino-5-methyl-4-oxo-3,4-dihydro-thieno[2,3-d]pyrimidin-6-yl)-benzonitrile was obtained instead of the desired compound.

### Step 2:

3-(2-Amino-5-methyl-4-oxo-3,4-dihydro-thieno[2,3-d]pyrimidin-6-yl)-benzonitrile (100 mg, 0.35 mmol, 1 eq) was solubilized in concentrated sulfuric acid (12 mL) and heated at 140 °C during 3 h. After cooling, water (10 mL) was added and the precipitate obtained was filtered to afford a 60/40 mixture of acid and amide compound.

### Step 3:

The mixture from step 2 was put in suspension in dichloromethane (6 mL). At 0 °C, triethylamine (42 µL, 0.3 mmol, 1.2 eq) and ethyl chloroformate (26 µL, 0.28 mmol, 1.1 eq) were added. After 1 h at 0 °C, ammonium hydroxide solution (15 mL) was added and the mixture was stirred from 0 °C to room temperature for 3 days. The solvent was evaporated and water (10 mL) was added. The precipitate obtained was filtered and dried in vacuo to afford the expected compound as a beige powder.
MS: 301.1
Mp: decomposes at 295 °C - 300 °C

### General Procedure D

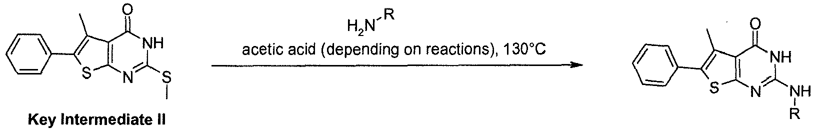

5-Methyl-2-methylsulfanyl-6-phenyl-3H-thieno[2,3-d]pyrimidin-4-one (**Key Intermediate II**) (1.3 g, 4.4 mmol, 1 eq) was put in suspension in the appropriate amine (3 mL) and depending on reactions, in acetic acid (1 mL). The resulting mixture was heated in a sealed tube at 130 °C during 18 h. After cooling, ethanol (20 mL) was added and the precipitate was filtered, rinsed with methanol, dichloromethane and ether and dried in vacuo to afford the expected compound (10% to 70% yield).

### Example 51:

### 2-(2-Hydroxy-ethylamino)-5-methyl-6-phenyl-3H-thieno[2,3-d]pyrimidin-4-one

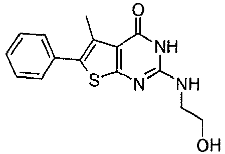

The expected compound was obtained according to **general procedure D** using ethanolamine and acetic acid. The expected compound was isolated as a white powder.
MS: 302.1
Mp: 227 °C - 229 °C

### Example 52:

### 2-(3-Hydroxy-propylamino)-5-methyl-6-phenyl-3H-thieno[2,3-d]pyrimidin-4-one

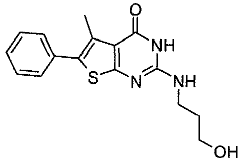

The expected compound was obtained according to **general procedure D** using 3-amino-propan-1-ol and acetic acid. After cooling , the reaction mixture was evaporated and water was added. The obtained precipitate was filtered and rinsed with diethyl ether and dichloromethane. The expected compound was isolated as a beige powder.
MS: 316.2
Mp: 227 °C - 229 °C

### Example 53:

### 2-(2-Amino-ethylamino)-5-methyl-6-phenyl-3H-thieno[2,3-d]pyrimidin-4-one

### Key Intermediate III

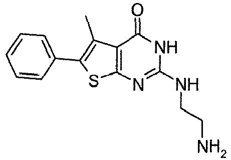

The expected compound was obtained according to **general procedure D** using ethylenediamine. The expected compound was isolated as a white powder.
MS: 301.1
Mp: 192 °C - 194 °C

### Example 54:

### 2-(2-Dimethylamino-ethylamino)-5-methyl-6-phenyl-3H-thieno[2,3-d]pyrimidin-4-one

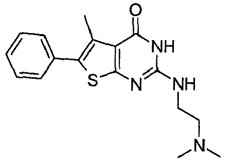

The expected compound was obtained according to **general procedure D** using N,N-dimethyl ethylenediamine and acetic acid. The expected compound was isolated as a beige powder.
MS: 329.2
Mp: 199 °C - 201 °C

### Example 55:

### 5-Methyl-6-phenyl-2-phenylamino-3H-thieno[2,3-d]pyrimidin-4-one

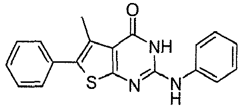

The expected compound was obtained according to **general procedure D** using aniline and acetic acid. The expected compound was isolated as a white powder.
MS: 334.1
Mp: 270 °C - 290 °C

### Example 56:

### 2-Cyclohexylamino-5-methyl-6-phenyl-3H-thieno[2,3-d]pyrimidin-4-one

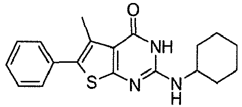

The expected compound was obtained according to **general procedure D** using cyclohexylamine. The expected compound was isolated as a white powder.
MS: 340.2
Mp: 265 °C - 270 °C

### Example 57:

### 5-Methyl-2-(2-morpholin-4-yl-ethylamino)-6-phenyl-3H-thieno[2,3-d]pyrimidin-4-one

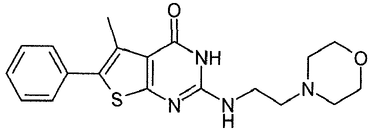

The expected compound was obtained according to **general procedure D** using 4-(2-aminoethyl)morpholine. The expected compound was isolated as a white powder.
MS: 371.1
Mp: 240 °C - 246 °C

### Example 58:

### 5-Methyl-2-morpholin-4-yl-6-phenyl-3H-thieno[2,3-d]pyrimidin-4-one

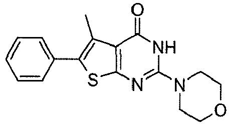

The expected compound was obtained according to **general procedure D** using morpholine. The expected compound was isolated as a white powder.
MS: 328.1
Mp: 300 °C - 320 °C

### General Procedure E

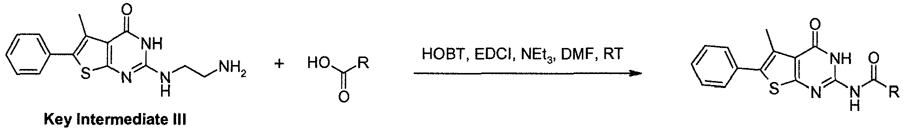

To a solution of 2-(2-amino-ethylamino)-5-methyl-6-phenyl-3H-thieno[2,3-d]pyrimidin-4-one (**Key Intermediate III**) (200 mg, 0.66 mmol, 1 eq) in dimethylformamide (5 mL) were added HOBT (180 mg, 1.33 mmol, 2 eq), EDCI (255 mg, 1.33 mmol, 2 eq), triethylamine (0.28 mL, 1.98 mmol, 3 eq) and the appropriate carboxylic acid (1.33 mmol, 2 eq). The mixture was stirred at room temperature for 20 h. Then the mixture was poured on water (10 mL) and extracted with dichloromethane (3 x 20mL). The organic layers were dried over magnesium sulfate, filtered and evaporated in vacuo. The crude residue was purified by flash chromatography using dichloromethane and ammonia 7N in methanol (100/0 to 80/20) to afford the expected compound (10 to 40% yield).

### Example 59:

### N-[2-(5-Methyl-4-oxo-6-phenyl-3,4-dihydro-thieno[2,3-d]pyrimidin-2-ylamino)-ethyl]-3-(4-methyl-piperazin-1-yl)-propionamide

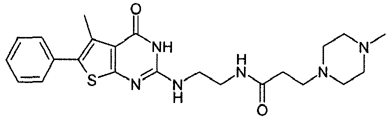

The expected compound was obtained according to **general procedure E** using 3-(4-methyl-piperazin-1-yl)-propionic acid. The expected compound was isolated as a white powder.
MS: 455.1
Mp: 235 °C - 245 °C

### Example 60:

### N-[2-(5-Methyl-4-oxo-6-phenyl-3,4-dihydro-thieno(2,3-d]pyrimidin-2-ylamino)-ethyl]-4-(4-methyl-piperazin-1-yl)-butyramide

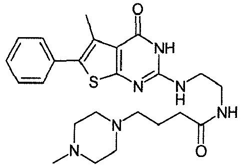

The expected compound was obtained according to **general procedure E** using 4-(4-methylpiperazin-1-yl)butanoic acid hydrochloride. The expected compound was isolated as a white powder.
MS: 469.2
Mp: 192 °C - 196 °C

### Example 61:

### 2-Amino-5-bromo-6-phenyl-3H-thieno [2,3-d]pyrimidin-4-one, hydrobromide salt

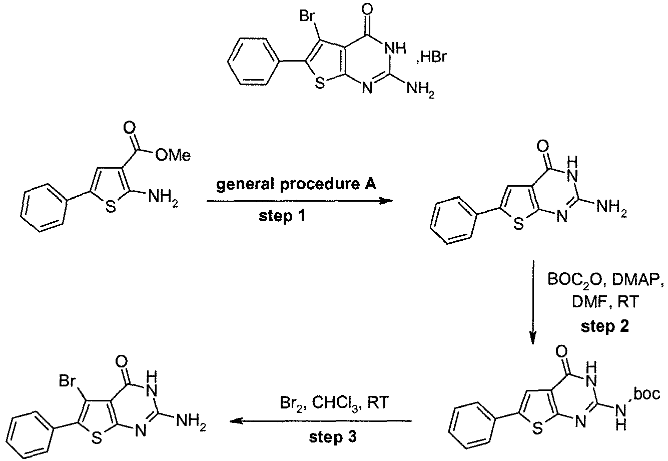

### Step 1:

The expected compound was obtained according to **general procedure A** using 2-amino-5-phenyl-thiophene-3-carboxylic acid methyl ester. The expected compound was isolated as a beige powder (3.5 g, 70% yield).

### Step 2:

To a solution of 2-amino-6-phenyl-3H-thieno[2,3-d]pyrimidin-4-one (2.0 g, 8.2 mmol, 1 eq) in dimethylformamide (100 mL) were added di-tert-butyl dicarbonate (3.6 g, 16.4 mmol, 2 eq) and 4-dimethylaminopyridine (200 mg, 1.6 mmol, 0.2 eq). The mixture was stirred at room temperature for 18 h. The solvent was then evaporated and the residue was taken up with dichloromethane (20 mL). The insoluble yellow solid was filtered off and the filtrate was washed with a saturated solution of sodium bicarbonate (2 X 20 mL). The organic layers were dried over magnesium sulfate, filtered and evaporated. The crude residue was purified by flash chromatography using dichloromethane and methanol (100/0 to 80/20) to afford (4-oxo-6-phenyl-3,4-dihydro-thieno[2,3-d]pyrimidin-2-yl)-carbamic acid tert-butyl ester as a light yellow solid (900 mg, 32% yield).

### Step 3:

The compound from step 2 (350 mg, 1.0 mmol, 1 eq) was solubilized in chloroform (10 mL) and bromine (52 µL, 1.0 mmol, 1 eq) was added. The mixture was stirred at room temperature for 1 h. More bromine (52 µL, 1.0 mmol, 1 eq) was added and the mixture was stirred for one additional hour at room temperature. The mixture was then evaporated and the residue was washed with dichloromethane (5 mL) and methanol (5 mL) to afford the expected compound as a beige powder (150 mg, 46% yield).
MS: 324.1

### Example 62:

### 2-Amino-5-chloro-6-phenyl-3H-thieno [2,3-d]pyrimidin-4-one

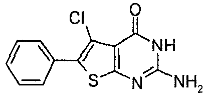

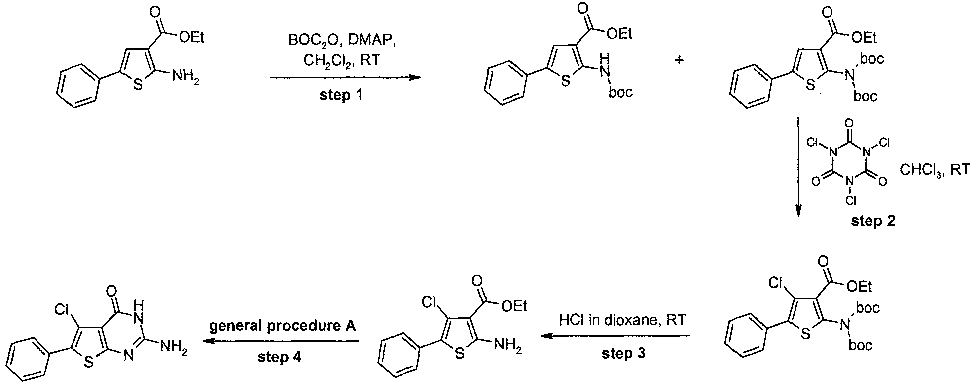

### Step 1:

To a solution of 2-amino-5-phenyl-thiophene-3-carboxylic acid ethyl ester (5.0 g, 20.2 mmol, 1 eq) in dichloromethane (40 mL) were added di-tert-butyl dicarbonate (6.6 g, 30.3 mmol, 1.5 eq) and 4-dimethylaminopyridine (247 mg, 2.0 mmol, 0.1 eq). The mixture was stirred at room temperature for 48 h. The mixture was washed with a saturated solution of sodium bicarbonate (3 X 20 mL) and the organic layers were dried over magnesium sulfate, filtered and evaporated. The crude residue was purified by flash chromatography using cyclohexane and ethyl acetate (100/0 to 90/10) to afford separately 2-tert-butoxycarbonylamino-5-phenyl-thiophene-3-carboxylic acid ethyl ester (1.9 g, 27% yield) as a yellow oil and bis(2-tert-butoxycarbonylamino)-5-phenyl-thiophene-3-carboxylic acid ethyl ester (4.1 g, 45% yield) as a light orange powder.

### Step 2:

To a solution of the diBoc compound from step 1 (3.1 g, 6.9 mmol, 1 eq) in chloroform (100 mL) was added trichloroisocyanuric acid (640 mg, 2.8 mmol, 0.4 eq). The mixture was stirred at room temperature for 18 h. The precipitate was filtered off and the filtrate was purified by flash chromatography using cyclohexane and ethyl acetate (100/0 to 90/10) to afford the expected compound as a light orange oil (1.1 g, 33% yield).

### Step 3:

The compound from step 2 (950 mg, 2.0 mmol, 1 eq) was solubilized in a 4N solution of hydrogen chloride in dioxane (20 mL) and the mixture was stirred at room temperature during 18 h. The mixture was then concentrated and the residue was taken up in dichloromethane (10 mL) and washed with a saturated solution of sodium bicarbonate (3 x 10 mL). The organic layers were dried over magnesium sulfate, filtered and evaporated in vacuo. The crude residue was purified by flash chromatography using cyclohexane and ethyl acetate (100/0 to 85/15) to afford 2-amino-4-chloro-5-phenyl-thiophene-3-carboxylic acid ethyl ester as a light orange oil (300 mg, 54% yield).

### Step 4:

The expected compound was obtained according to **general procedure A** using 2-amino-4-chloro-5-phenyl-thiophene-3-carboxylic acid ethyl ester. The expected compound was isolated as a beige powder (175 mg, 61% yield).
MS: 278.0
Mp > 350°C

**Activity data for the compounds having the general formula (II)**

| **molregno** | **structure** | **activity type** | **activity endpoint** | **activity conc** | **activity result** |
|---|---|---|---|---|---|
| SAV-7475 | 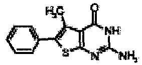 | Biacore | Binding (RU) | 10 | 94,5 |
| | | Biacore | KD (µM) | | 4,6 |
| | | CPE H3N2 | reduction (%) | 5 | 8,9 |
| | | Biacore PA-Nter | Binding (RU) | 50 | 13,16 |
| | | CPE H3N2 | IC50 (µM) | | 21 |
| SAV-7517 | 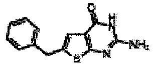 | Biacore | Binding (RU) | 10 | 39,3 |
| | | Biacore | KD(µM) | | 13 |
| | | CPE H3N2 | reduction (%) | 10 | -2,64 |
| SAV-7521 | 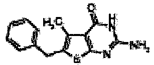 | Biacore | Binding (RU) | 10 | 64,7 |
| | | Biacore | KD (µM) | | 6 |
| | | CPEH3N2 | reduction (%) | 25 | 33,6 |
| SAV-7549 | 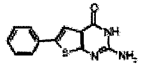 | Biacore | Binding (RU) | 10 | 65,5 |
| | | CPE H3N2 | reduction (%) | 50 | -2,4 |
| | | Biacore | KD (µM) | | 8,8 |
| SAV-7575 | 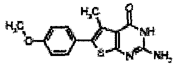 | Biacore | Banding (RU) | 10 | 35,9 |
| | | CPEH3N2 | reduction (%) | 20 | 9,02 |
| | | Biacore | KD (µM) | | 4 |
| SAV-7577 | 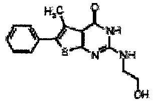 | Biacore | Binding (RU) | 10 | 81,1 |
| | | Biacore | KD (µM) | | 6,8 |
| | | CPE H3N2 | reduction (%) | 20 | 2,85 |
| SAV-7579 | 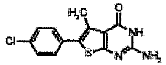 | Biacore | Binding (RU) | 10 | 21,4 |
| | | CPE H3N2 | reduction (%) | 20 | 11,3 |
| | | Biacore | KD (µM) | | 0,27 |
| SAV-7580 | 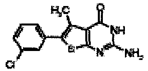 | Biacore | Binding (RU) | 10 | 25,2 |
| | | CPE H3N2 | reduction (%) | 2 | 89,8 |
| | | CPE H3N2 | IC50 (µM) | | 11 |
| | | Biacore | KD (µM) | | 8,9 |
| SAV-75B1 | 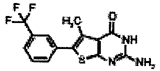 | Biacore | Binding (RU) | 10 | 20,5 |
| | | CPE H3N2 | reduction (%) | 50 | 87,8 |
| | | Biacore | KD (µM) | | 1,4 |
| | | CPE H3N2 | IC50 (µM) | | 17 |
| SAV-7582 | 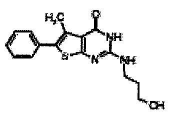 | Biacore | Binding (RU) | 10 | 102,9 |
| | | Biacore | KD (µM) | | 4,2 |
| | | CPE H3N2 | reduction (%) | 50 | 53,1 |
| SAV-7583 | 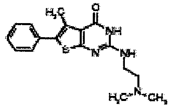 | Biacore | Binding (RU) | 10 | 77,8 |
| | | CPEH3N2 | reduction (%) | 5 | 4,8 |
| | | Biacore | KD (µM) | | 68 |
| SAV-7585 | 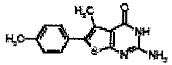 | CPE H3N2 | reduction (%) | 2.5 | 28,9 |
| | | Biacore | Binding (RU) | 10 | 29,2 |
| | | CPE H3N2 | IC50 (µM) | | 3,5 |
| | | Biacore | KD (µM) | | 2 |
| SAV-7586 | 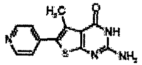 | CPE H3N2 | reduction (%) | 15 | 81,2 |
| | | Biacore | Binding (RU) | 10 | 100,2 |
| | | Biacore | KD (µM) | | 12 |
| | | CPE H3N2 | IC50 (µM) | | 5,3 |
| SAV-7588 | 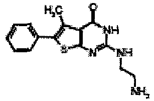 | CPE H3N2 | reduction (%) | 5 | 2,2 |
| | | Biacone | Binding (RU) | 10 | 110,6 |
| | | Biacore | KD (µM) | | |
| SAV-7589 | 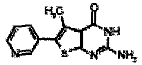 | CPE H3N2 | reduction (%) | 50 | 63,1 |
| | | Biacore | Binding (RU) | 10 | 88 |
| | | Biacore | KD (µM) | | 10 |
| | | CPE H3N2 | IC50 (µM) | | 27 |
| SAV-7594 | 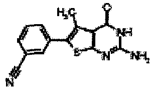 | Biacore | KD (µM) | | 2 |
| | | Biacore | Binding (RU) | 10 | 31,9 |
| | | CPE H3N2 | reduction (%) | 5 | 14,5 |
| | | CPE H3N2 | IC50 (µM) | | 14 |
| SAV-7596 | 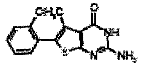 | Biacore | KD (µM) | | 11 |
| | | Biacore | Binding (RU) | 10 | 29,6 |
| | | CPE H3N2 | reduction (%) | 5 | 13,6 |
| SAV-7598 | 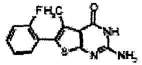 | Biacore | Binding (RU) | 10 | 21,3 |
| | | Biacore | KD (µM) | | 3,7 |
| | | CPE H3N2 | reduction (%) | 2 | 11,4 |
| SAV-7599 | 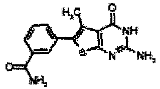 | CPE H3N2 | reduction (%) | 20 | -0,7 |
| | | Biacore | Binding (RU) | 12.5 | 65,3 |
| | | Biacore | KD (µM) | | 5.3 |
| SAV-7600 | 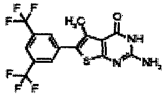 | CPE H3N2 | reduction (%) | 2 | 5,8 |
| | | Biacore | KD (µM) | | |
| | | Biacore | Binding (RU) | 10 | 3,8 |
| SAV-7601 | 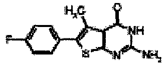 | CPE H3N2 | reduction (%) | 50 | 69,2 |
| | | Biacore | KD (µM) | | 1,1 |
| | | Biacore | Binding(RU) | 10 | 44,3 |
| SAV-7602 | 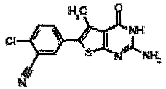 | CPE H3N2 | reduction (%) | 20 | 67,9 |
| | | Biacore | KD (µM) | | 1,7 |
| | | Biacore | Binding (RU) | 10 | 35,1 |
| SAV-7603 | 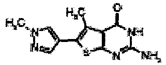 | CPE H3N2 | reduction (%) | 20 | 1,1 |
| | | Biacore | KD (µM) | | 42 |
| SAV-7604 | 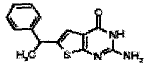 | CPE H3N2 | reduction (%) | 20 | -4,7 |
| | | Biacore | KD (µM) | | 6,4 |
| SAV-7606 | 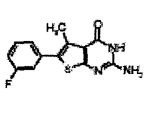 | Biacore | KD (µM) | | 8 |
| | | CPE H3N2 | reduction (%) | 5 | 21,8 |
| SAV-7607 | 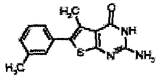 | Biacore | KD (µM) | | 5,4 |
| | | CPE H3N2 | reduction (%) | 2 | -0,4 |
| SAV-7608 | 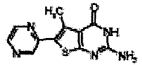 | Biacore | KD (µM) | | 5,7 |
| | | CPE H3N2 | reduction (%) | 10 | 3,8 |
| SAV-7609 | 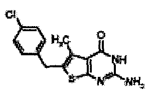 | CPE H3N2 (insoluble) | reduction (%) insoluble | | |
| SAV-7610 | 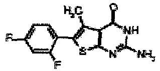 | Biacore | KD (µM) | | 3,8 |
| | | CPE H3N2 | reduction (%) | 2 | -1,6 |
| | | ALPHA screen | EC50 (µM) | | 0,62 |
| SAV-7611 | 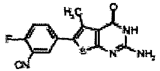 | Biacore | KD (µM) | | 1,1 |
| | | CPEH3N2 | reduction (%) | 2 | -1 |
| SAV-7613 | 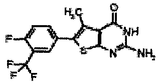 | Biacore | KD (µM) | | 1,5 |
| | | CPE H3N2 | reduction (%) | 50 | 2,5 |
| SAV-7614 | 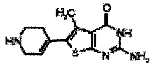 | Biacore | KD (µM) | | 190 |
| | | CPE H3N2 | reduction (%) | 50 | 10,6 |
| SAV-7615 | 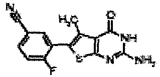 | Biacore | KD (µM) | | 3 |
| | | CPE H3N2 | reduction (%) | 20 | 59,2 |
| | | CPE H3N2 | IC50 (µM) | | 12 |
| SAV-7616 | 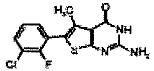 | Biacore | KD (µM) | | 3,1 |
| | | CPE H3N2 | reduction (%) | 10 | 68,7 |
| | | CPE H3N2 | IC50 (µM) | | 34 |
| SAV-7617 | 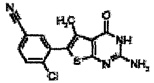 | Biacore | KD (µM) | | 2 |
| | | CPE H3N2 | reduction (%) | 2 | 5,1 |
| SAV-7618 | 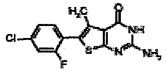 | Biacore | KD (µM) | | 0,65 |
| | | CPE H3N2 | reduction (%) | 50 | 77,4 |
| | | CPE H3N2 | IC50 (µM) | | 43 |
| SAV-7619 | 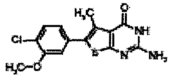 | Biacore | KD (µM) | | 1,1 |
| | | CPE H3N2 | reduction (%) | 2 | 3,4 |
| SAV-7620 | 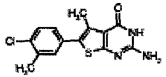 | Biacore | KD (µM) | | |
| | | CPE H3N2 | reduction (%) | 50 | 57,8 |
| | | CPE H3N2 | IC50 (µM) | | 29 |
| SAV-7621 | 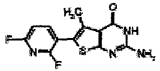 | Biacore | KD (µM) | | |
| | | CPE H3N2 | IC50 (µM) | | 70 |
| | | CPE H3N2 | reduction (%) | 25 | 14,3 |
| SAV-7622 | 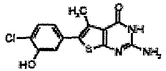 | Biacore | KD (µM) | | 2,4 |
| | | CPEH3N2 | reduction (%) | 2 | 6,2 |
| SAV-7623 | 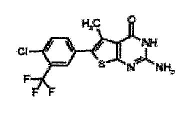 | Biacore | KD (µM) | | |
| | | CPE H3N2 | reduction (%) | 5 | 1,9 |
| SAV-7624 | 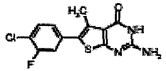 | Biacore | KD (µM) | | 0,2 |
| | | CPE H3N2 | IC50 (µM) | | 64 |
| SAV-7625 | 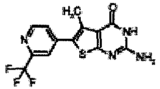 | Biacore | KD (µM) | | 12 |
| | | CPE H3N2 | reducbon (%) | 2.5 | -4 |
| SAV-7626 | 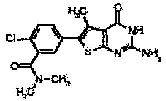 | Biacore | KD (µM) | | 6,4 |
| | | CPE H3N2 | reduction (%) | 25 | -4,6 |
| SAV-7627 | 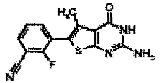 | Biacore | KD (µM) | | 4,2 |
| | | CPE H3N2 | reduction (%) | 50 | 71.2 |
| | | CPE H3N2 | IC50 (µM) | | 10 |
| SAV-7628 | 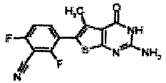 | Biacore | KD(µM) | | 7,3 |
| | | CPE H3N2 | reduction (%) | 50 | 84,3 |
| | | CPE H3N2 | IC50 (µM) | | 43 |
| SAV-7629 | 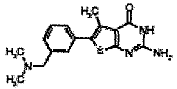 | Biacore | KD (µM) | | 61 |
| | | CPE H3N2 | reduction (%) | 50 | 38,3 |
| | | CPE H3N2 | IC50 (µM) | | 35 |
| SAV-7630 | 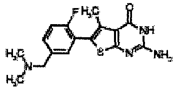 | Biacore | KD (µM) | | 57 |
| | | CPE H3N2 | reduction (%) | 50 | 23,2 |
| | | CPE H3N2 | IC50 (µM) | | |
| SAV-7631 | 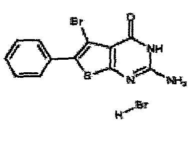 | Biacore | KD (µM) | | 0,86 |
| | | CPE H3N2 | reduction (%) | 5 | 4 |
| | | CPE H3N2 | IC50 (µM) | | |
| SAV-7632 | 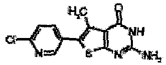 | Biacore | KD (µM) | | 0,75 |
| | | CPE H3N2 | reduction (%) | 50 | 53 |
| | | CPEH3N2 | IC50 (µM) | | |
| SAV-7633 | 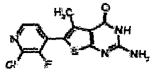 | Biacore | KD (µM) | | 1,2 |
| | | CPE H3N2 | reduction (%) | 50 | 63,1 |
| | | CPEH3N2 | IC50 (µM) | | 39 |
| SAV-7637 | 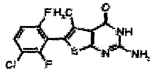 | CPE H3N2 | reduction (%) | 5 | 1,5 |
| | | CPE H3N2 | IC50 (µM) | | 18 |
| | | Biacore | KD (µM) | | 1,1 |
| SAV-7638 | 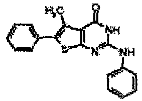 | CPE H3N2 | reduction (%) | 5 | 8,7 |
| | | CPE H3N2 | IC50 (µM) | | |
| | | Biacore | KD (µM) | | |
| SAV-7639 | 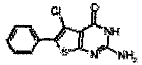 | CPE H3N2 | reduction (%) | 50 | 59,9 |
| | | CPE H3N2 | IC50 (µM) | | |
| | | Biacore | KD (µM) | | 1,8 |
| SAV-7640 | 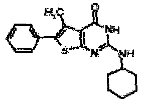 | CPE H3N12 | reduction (%) | 5 | -3,2 |
| | | CPE H3N2 | IC50 (µM) | | |
| | | Biacore | KD (µM) | | |

## Claims

1. A compound having the general formula (II), optionally in the form of a pharmaceutically acceptable salt, solvate, polymorph, tautomer, racemate, enantiomer, or diastereomer or mixture thereof, 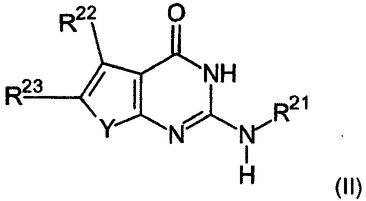 wherein
**Y** is S;
**R²¹** is selected from -H, -C₁₋₆alkyl, -(CH₂)_{q}-aryl, -(CH₂)_{q}-heterocyclyl, -(CH₂)_{q}-cycloalkyl, -(CH₂)ₚ-OR²⁵, and -(CH₂)ₚ-NR²⁵R²⁶;
**R²²** is selected from -H, -C₁₋₆ alkyl, -(CH₂)_{q}-cycloalkyl, -Hal, -CF₃ and -CN;
**R²³** is selected from -aryl, -heterocyclyl, -cycloalkyl, -C(-R²⁸)(-R²⁹)-aryl, -C(-R²⁸)(-R²⁹)-heterocyclyl, and -C(-R²⁸)(-R²⁹)-cycloalkyl;
**R²⁵** is selected from -H, -C₁₋₆ alkyl, and -(CH₂CH₂O)ᵣH;
**R²⁶** is selected from -H, and -C₁₋₆ alkyl;
**R²⁷** is independently selected from -C₁₋₆ alkyl, -C(O)-C₁₋₆ alkyl, -Hal, -CF₃, -CN, -COOR²⁵, -OR²⁵, -(CH2)_{q}NR²⁵R²⁶, -C(O)-NR²⁵R²⁶, and -NR²⁵-C(O)-C₁-₆ alkyl;
**R²⁸** and **R²⁹** are independently selected from -H, -C₁₋₆ alkyl, -(CH₂)_{q}-aryl, -(CH₂)_{q}-heterocyclyl, -(CH₂)_{q}-cycloalkyl, -OH, -O-C₁₋₆ alkyl, -O-(CH₂)q-aryl**, -**O-(CH₂)_{q}-heterocyclyl, and -O-(CH₂)_{q}-cycloalky);
or **R²⁸** and **R²⁹** are together =O -CH₂CH₂-. -CH₂CH₂CH₂-, or -CH₂CH₂CH₂CH₂-;
**p** is 1 to 4;
**q** is 0 to 4; and
**r** is 1 to 3;
wherein the aryl group, heterocyclyl group and/or cycloalkyl group can be optionally substituted with one or more substituents **R²⁷**;
with the proviso that the compound is not one of the following compounds:

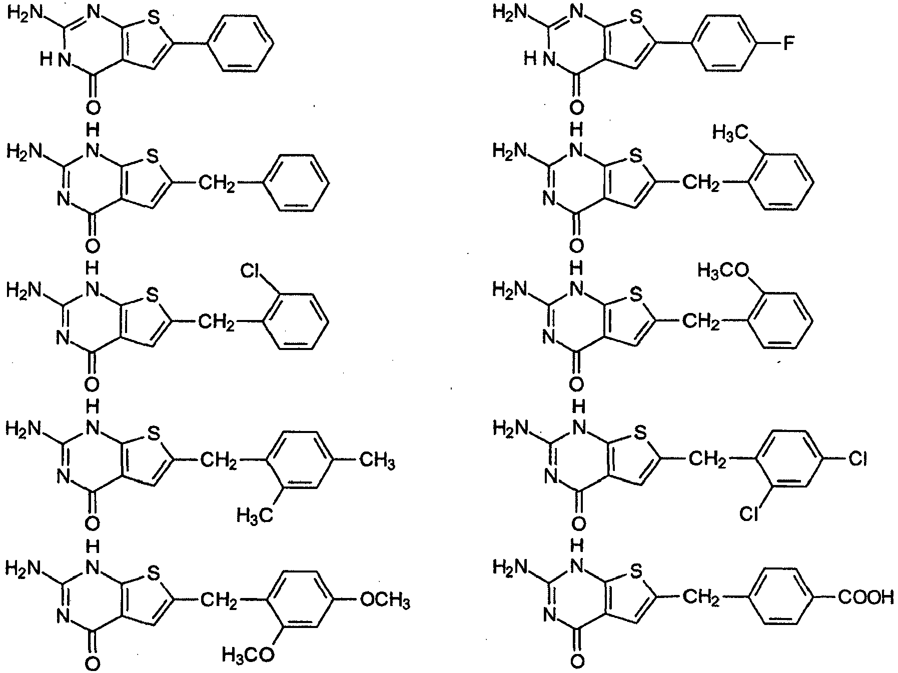

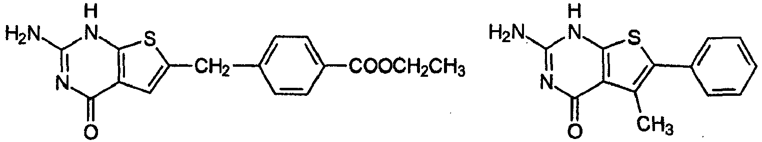

2. The compound according to claim 1, wherein R²¹ is -H, -C₁₋₆ alkyl, or -(CH₂)ₚ-OR²⁵.

3. The compound according to claim 1 or 2, wherein R²² is -H, -C₁₋₆ alkyl or Hal.

4. The compound according to any of the preceding claims, wherein R²³ is -(CH₂)_{q}-aryl; or -(CH₂)_{q}-heteroaryl, and wherein the aryl group and/or heteroaryl group can be optionally substituted with one or more substituents R²⁷.

5. The compound according to any of the preceding claims, wherein R²³ is -phenyl, -benzyl or -pyridyl and wherein the substituents are independently selected from -Hal, -CF₃, -CN, -C₁₋₆ alkyl, -C(O)-C₁₋₆ alkyl, or -(CH₂)_{q}NR²⁵R²⁶, wherein R²⁵ and R²⁶ are independently selected from H and -C₁₋₆ alkyl.

6. A pharmaceutical composition comprising:
a compound having the general formula (II), optionally in the form of a pharmaceutically acceptable salt, solvate, polymorph, tautomer, racemate,
enantiomer, or diastereomer or mixture thereof, 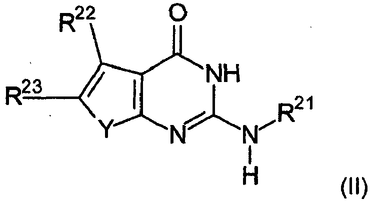 wherein
**Y** is S;
**R²¹** is selected from -H, -C₁₋₆alkyl, -(CH₂)_{q-}aryl, -(CH₂)_{q}-heterocyclyl), -(CH₂)_{q}-cycloalkyl, -(CH₂)ₚ₋OR²⁵, and -(C_{H2})ₚ-NR²⁵R²⁶;
**R²²** is selected from -H, -C₁₋₆ alkyl, -(CH₂)_{q}-cycloalkyl, -Hal, -CF₃ and -CN;
**R²³** is selected from -aryl, -heterocyclyl, -cycloalkyl, -C(-R²⁸)(-R²⁹)-aryl, -C(-R²⁸)(-R²⁹)-heterocyclyl, and -C(-R²⁸)(-R²⁹)-cycloalkyl;
**R²⁵** is selected from -H, -C₁₋₆ alkyl, and -(CH₂CH₂O)ᵣH;
**R²⁸** is selected from -H, and -C₁₋₆ alkyl;
**R²⁷** is independently selected from -C₁₋₆ alkyl, -C(O)-C₁₋₆ alkyl, -Hal, -CF₃, -CN, -COOR²⁵, -OR²⁵, -(CH₂)_{q}NR²⁵R²⁶, -C(O)-NR²⁵R²⁶, and -NR²⁵-C(O)-C₁₋₆ alkyl;
**R²⁸** and **R²⁹** are independently selected from -H, -C₁₋₆ alkyl, -(CH₂)_{q}-aryl, -(CH₂)_{q}-heterocyclyl, -(CH₂)_{q}-cycloalkyl, -OH, -O-C₁₋₆alkyl, -O-(CH₂)_{q}-aryl, -O-(CH₂)_{q}-heterocyclyl, and -O-(CH₂)_{q}-cycloalkyl;
or **R²⁸** and **R²⁹** are together =O, -CH₂CH₂-, -CH₂CH₂CH₂-, or -CH₂CH₂CH₂CH₂-;
**p** is 1 to 4;
**q** is 0 to 4; and
**r** is 1 to 3;
wherein the aryl group, heterocyclyl group and/or cycloalkyl group can be optionally substituted with one or more substituents R²⁷;
with the proviso that the compound is not one of the following compounds: 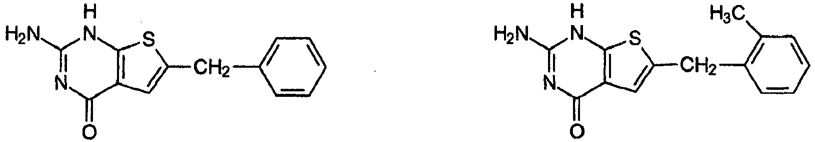 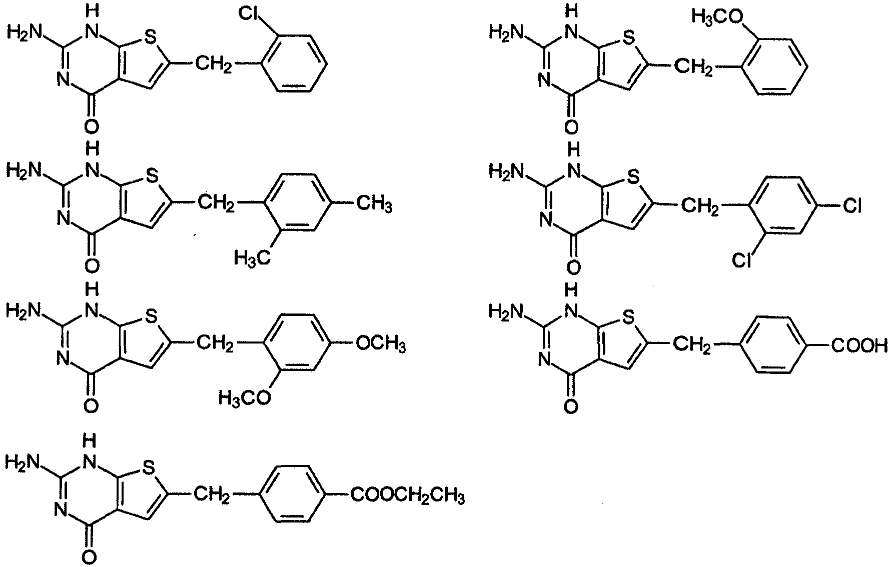 and optionally one or more pharmaceutically acceptable excipient(s) and/or carrier(s).

7. The pharmaceutical composition according to claim 6, wherein R²¹ is -H, -C₁₋₆ alkyl, or-(CH₂)ₚ-OR²⁵.

8. The pharmaceutical composition according to claim 6 or 7, wherein R²² is -H, -C₁₋₆ alkyl or Hal.

9. The pharmaceutical composition according to any of claims 6 to 8, wherein R²³ is -(CH₂)_{q}-aryl; or -(CH₂)_{q}-heteroaryl, and wherein the aryl group and/or heteroaryl group can be optionally substituted with one or more substituents R²⁷.

10. The pharmaceutical composition according to any of claims 6 to 9, wherein R²³ is -phenyl, -benzyl or -pyridyl and wherein the substituents are independently selected from -Hal, -CF₃, -CN, -C₁₋₆ alkyl, -C(O)-C₁₋₆ alkyl, or -(CH₂)_{q}NR²⁵R²⁶, wherein R²⁵ and R²⁶ are independently selected from H and -C₁₋₆ alkyl.

11. A compound having the general formula (II), optionally in the form of a pharmaceutically acceptable salt, solvate, polymorph, tautomer, racemate, enantiomer, or diastereomer or mixture thereof, 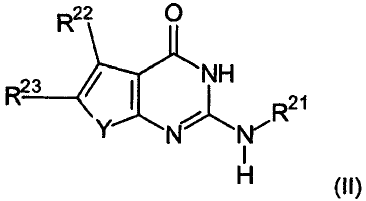 wherein
YisS;
**R²¹** is selected from -H, -C₁₋₆alkyl, -(CH₂)_{q}-aryl, -(CH₂)_{q}-heterocyclyl, -(CH₂)_{q}-cycloalkyl, -(CH₂)ₚ-OR²⁵, and -(CH₂)ₚ-NR²⁵R²⁶;
**R²²** is selected from -H, -C₁₋₆ alkyl, -(CH₂)_{q}-cycloalkyl, -Hal, -CF₃ and -CN;
**R²³** is selected from -aryl, -heterocyclyl, -cycloalkyl, -C(-R²⁸)(-R²⁹)-aryl, -C(-R²⁸)(-R²⁹)-heterocyclyl, and -C(-R²⁸)(-R²⁹)-cycloalkyl;
**R²⁵** is selected from -H, -C₁₋₆ alkyl, and -(CH₂CH₂O)ᵣH;
**R²⁶** is selected from -H, and -C₁₋₆ alkyl;
**R²⁷** is independently selected from -C₁₋₆ alkyl, -C(O)-C₁₋₆ alkyl, -Hal, -CF₃, -CN, -COOR²⁵, -OR²⁵, -(CH₂)_{q}NR²⁵R²⁶, -C(O)-NR²⁵R²⁶, and -NR²⁵-C(O)-C₁₋₆ alkyl;
**R²⁸** and **R²⁹** are independently selected from -H, -C₁₋₆ alkyl, -(CH₂)_{q}-aryl, -(CH₂)_{q}-heterocyclyl, -(CH₂)_{q}-cycloalkyl, -OH, -O-C₁₋₆ alkyl, -O-(CH₂)_{q}-aryl, -O-(CH₂)_{q}-heterocyclyl, and -O-(CH₂)_{q}-cycloalkyl;
or **R²⁸** and **R²⁹** are together =O, -CH₂CH₂-, -CH₂CH₂CH₂-, or -CH₂CH₂CH₂CH₂-;
**p** is 1 to 4;
**q** is 0 to 4; and
**r** is 1 to 3;
wherein the aryl group, heterocyclyl group and/or cycloalkyl group can be optionally substituted with one or more substituents **R²⁷**;
wherein the compound is for use in the treatment, amelioration or prevention of a viral disease.

12. The compound for use according to claim 11, wherein the viral disease is caused by Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae, or Flaviviridae; more specifically wherein the viral disease is influenza.

13. The compound for use according to claim 11 or 12, wherein R²¹ is -H, -C₁₋₆ alkyl, or -(CH₂)ₚ-OR²⁵.

14. The compound for use according to any of claims 11 to 13, wherein R²² is -H, -C₁₋₆ alkyl or Hal.

15. The compound for use according to any of claims 11 to 14, wherein R²³ is -(CH₂)_{q}-aryl; or -(CH₂)_{q}-heteroaryl, and wherein the aryl group and/or heteroaryl group can be optionally substituted with one or more substituents R²⁷.

16. The compound for use according to any of claims 11 to 15, wherein R²³ is -phenyl, -benzyl or -pyridyl and wherein the substituents are independently selected from -Hal, -CF₃, -CN, -C₁₋₆ alkyl, -C(O)-C₁₋₆ alkyl, or -(CH₂)_{q}NR²⁵R²⁶, wherein R²⁵ and R²⁶ are independently selected from H and -C₁₋₆ alkyl.
and optionally one or more pharmaceutically acceptable excipient(s) and/or carrier(s).

17. A pharmaceutical composition comprising:
(i) a compund having the general formula (II), optionally in the form of a Pharmaceutical acceptable salt, solvate, polymorph, tautomer, racemate, enantiomer, or diastereomer or mixture thereof, 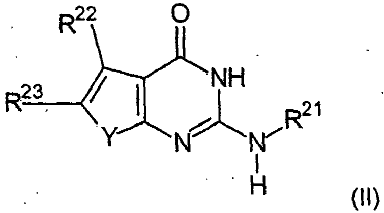 wherein
**Y** is S;
**R²¹** is selected from -H, -C₁₋₆alkyl, -(CH₂)_{q}-aryl, -(CH₂)_{q}-heterocyclyl, -(CH₂)_{q}-cycloalkyl, -(CH₂)ₚ-OR²⁵, and -(CH₂)ₚ-NR²⁵R²⁶;
**R²²** is selected from -H, -C₁₋₆alkyl, -(CH₂)_{q}-cycloalkyl, -Hal, -CF₃ and -CN;
**R²³** is selected from -aryl, -heterocyclyl, -cycloalkyl, -C(-R²⁸)(-R²⁹)-aryl, -C(-R²⁸)(-R²⁹)-heterocyclyl, and -C(-R²⁸)(-R²⁹)-cycloalkyl);
**R²⁵** is selected from -H, -C₁₋₆ alkyl, and -(CH₂CH₂O)ᵣH;
**R²⁶** is selected from -H, and -C₁₋₆ alkyl;
**R²⁷** is independently selected from -C₁₋₆ alkyl, -C(O)-C₁₋₆ alkyl, -Hal, -CF₃, -CN, -COOR²⁵, -OR²⁵, -(CH₂)_{q}NR²⁵R²⁶, -C(O)-NR²⁵R²⁶, and -NR²⁵-C(O)-C₁₋₆ alkyl;
**R²⁸** and **R²⁹** are independently selected from -H, -C₁₋₆ alkyl, -(CH₂)_{q}-aryl,-(CH₂)_{q}-heterocyclyl, -(CH₂)_{q}-cycloalkyl, -OH, -O-C₁₋₆ alkyl, -O-(CH₂)_{q}-aryl, -O-(CH₂)_{q}-heterocyclyl, and -O-(CH₂)_{q}-cycloalkyl;
or **R²⁸** and **R²⁹** are together =O -CH₂CH₂-, -CH₂CH₂CH₂-, or -CH₂CH₂CH₂CH₂-;
**p** is 1 to 4;
**q** is 0 to 4; and
**r** is 1 to 3;
wherein the aryl group, heterocyclyl group and/or cycloalkyl group can be optionally substituted with one or more substituents R²⁷;
and
(ii) a further compound selected from:
(a) at least one polymerase inhibitor which is different from the compound having the general formula (II);
(b) at least one neuramidase inhibitor;
(c) at least one M2 channel inhibitor;
(d) at least one alpha glucosidase inhibitor;
(e) at least one ligand of another influenza target; and
(f) at least one medicament selected from antibiotics, anti-inflammatory agents, lipoxygenase inhibitors, EP ligands, bradykinin ligands, and cannabinoid ligands;
and optionally one or more pharmaceutically acceptable excipient(s) and/or carrier(s).

18. The pharmaceutical composition as defined in claim 17 for use in the treatment, amelioration or prevention of a viral disease.

19. The pharmaceutical composition for use according to claim 18, wherein the viral disease is caused by Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae, or Flaviviridae; more specifically wherein the viral disease is influenza.

## Patentansprüche

1. Eine Verbindung mit der allgemeinen Formel (II), gegebenenfalls in Form eines pharmazeutisch verträglichen Salzes, Solvats, Polymorphs, Tautomers, Racemats, Enantiomers oder Diastereomers oder Gemischs davon, 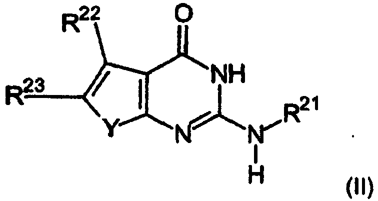 wobei
Y gleich S ist;
R²¹ ausgewählt ist aus -H, -C₁₋₆-Alkyl, -(CH₂)_{q}-Aryl, -(CH₂)_{q}-Heterocyclyl, -(CH₂)_{q}-Cycloalkyl, -(CH₂)ₚ-OR²⁵ und -(CH₂)ₚ-NR²⁵R²⁶;
R²² ausgewählt ist aus -H, -C₁₋₆-Alkyl, -(CH₂)_{q}-Cycloalkyl, -Hal, -CF₃ und -CN;
R²³ ausgewählt ist aus -Aryl, -Heterocyclyl, -Cycloalkyl, -C(-R²⁸)(-R²⁹)-Aryl, -C(-R²⁸)(-R²⁹)-Heterocyclyl und -C(-R²⁸)(-R²⁹⁾-Cycloalkyl;
R²⁵ ausgewählt ist aus -H, -C₁₋₆-Alkyl und -(CH₂CH₂O)ᵣH;
R²⁶ ausgewählt ist aus -H und -C₁₋₆-Alkyl;
R²⁷ unabhängig ausgewählt ist aus -C₁₋₆-Alkyl, -C(O)-C₁₋₆-Alkyl, -Hal, -CF₃, -CN, -COOR²⁵, -OR²⁵, -(CH₂)_{q}NR²⁵R²⁶, -C(O)-NR²⁵R²⁶ und NR²⁵-C(O)-C₁₋₆-Alkyl;
R²⁸ und R²⁹ unabhängig ausgewählt sind aus -H, -C₁₋₆-Alkyl, -(CH₂)_{q}-Aryl, -(CH₂)_{q}-Heterocyclyl, -(CH₂)_{q}-Cycloalkyl, -OH, -O-C₁₋₆-Alkyl, -O-(CH₂)_{q}-Aryl, -O-(CH₂)_{q}-Heterocyclyl und -O-(CH₂)_{q}-Cycloalkyl;
oder R²⁸ und R²⁹ zusammen =O, -CH₂CH₂-, -CH₂CH₂CH₂- oder -CH₂CH₂CH₂CH₂-sind;
p gleich 1 bis 4 ist;
q gleich 0 bis 4 ist; und
r gleich 1 bis 3 ist;
wobei die Arylgruppe, Heterocyclylgruppe und/oder Cycloalkylgruppe gegebenenfalls mit einem oder mehreren Substituenten R²⁷ substituiert sein kann/können;
mit der Maßgabe, dass die Verbindung nicht eine der folgenden Verbindungen ist:

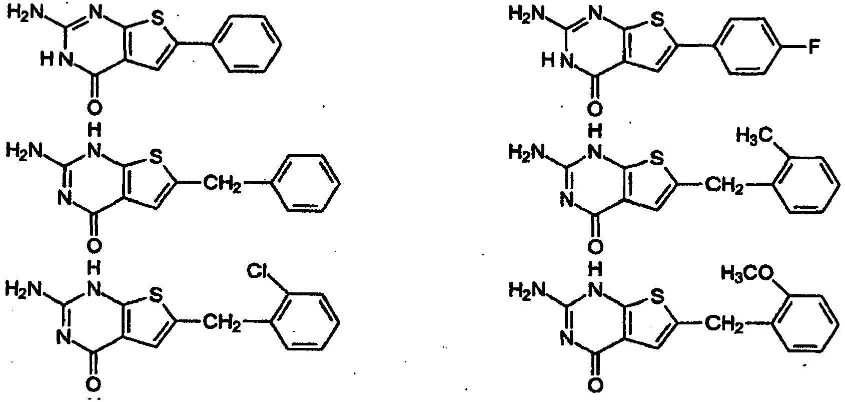

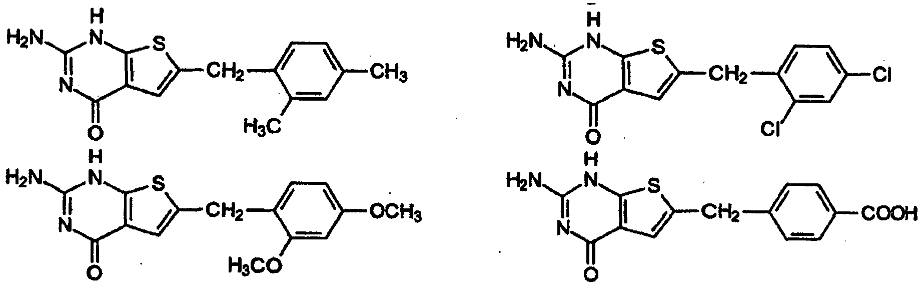

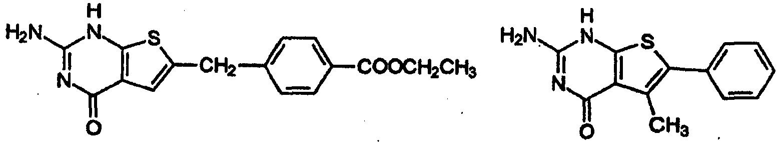

2. Die Verbindung gemäß Anspruch 1, wobei R²¹ gleich -H, -C₁₋₆-Alkyl oder -(CH₂)ₚ-OR²⁵ ist.

3. Die Verbindung gemäß Anspruch 1 oder 2, wobei R²² gleich -H, -C₁₋₆-Alkyl oder Hal ist.

4. Die Verbindung gemäß einem der vorhergehenden Ansprüche, wobei R²³ gleich -(CH₂)_{q}-Aryl oder -(CH₂)_{q}-Heteroaryl ist und wobei die Arylgruppe und/oder Heteroarylgruppe gegebenenfalls mit einem oder mehreren Substituenten R²⁷ substituiert sein kann/können.

5. Die Verbindung gemäß einem der vorhergehenden Ansprüche, wobei R²³ -Phenyl, -Benzyl oder -Pyridyl ist und wobei die Substituenten unabhängig voneinander ausgewählt sind aus -Hal, -CF₃, -CN, -C₁₋₆-Alkyl, -C(O)-C₁₋₆-Alkyl oder -(CH₂)_{q}NR²⁵R²⁶, wobei R²⁵ und R²⁶ unabhängig aus H und -C₁₋₆-Alkyl ausgewählt sind.

6. Ein Arzneimittel, umfassend: eine Verbindung der allgemeinen Formel (II), gegebenenfalls in Form eines pharmazeutisch verträglichen Salzes, Solvats, Polymorphs, Tautomers, Racemats, Enantiomers oder Diastereomers oder Gemischs davon, 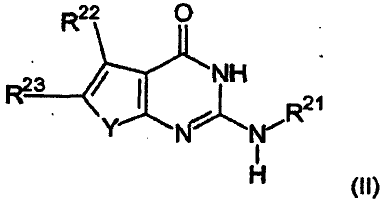 wobei
Y gleich S ist;
R²¹ ausgewählt ist aus -H, -C₁₋₆-Alkyl, -(CH₂)_{q}-Aryl, -(CH₂)_{q}-Heterocyclyl, -(CH₂)_{q}-Cycloalkyl, -(CH₂)ₚ-OR²⁵ und -(CH₂)ₚ-NR²⁵R²⁶;
R²² ausgewählt ist aus -H, -C₁₋₆-Alkyl, -(CH₂)_{q}-Cycloalkyl, -Hai, -CF₃ und -CN;
R²³ ausgewählt ist aus -Aryl, -Heterocyclyl, -Cycloalkyl, -C(-R²⁸)(-R²⁹)-Aryl, -C(-R²⁸)(-R²⁹)-Heterocyclyl und -C(-R²⁸)(-R²⁹)Cycloalkyl;
R²⁵ ausgewählt ist aus -H, -C₁₋₆-Alkyl und -(CH₂CH₂O)ᵣH;
R²⁶ ausgewählt ist aus -H und -C₁₋₆-Alkyl;
R²⁷ unabhängig ausgewählt ist aus -C₁₋₆-Alkyl, -C(O)-C₁₋₆-Alkyl, -Hal, -CF₃, -CN, -COOR²⁵, -OR²⁵, -(CH₂)_{q}NR²⁵R²⁶, -(O)NR²⁵R²⁶ und -NR²⁵-C(O)C₁₋₆-Alkyl;
R²⁸ und R²⁹ unabhängig ausgewählt sind aus -H, -C₁₋₆-Alkyl, -(CH₂)_{q}-Aryl, -(CH₂)_{q}-Heterocyclyl, -(CH₂)_{q}-Cycloalkyl, -OH, -O-C₁₋₆-Alkyl, -O-(CH₂)_{q}-Aryl, -O-(CH₂)_{q}-Heterocyclyl und -O-(CH₂)_{q}-Cycloalkyl;
oder R²⁸ und R²⁹ zusammen =O, -CH₂CH₂-, -CH₂CH₂CH₂- oder -CH₂CH₂CH₂CH₂-sind;
p gleich 1 bis 4 ist;
q gleich 0 bis 4 ist; und
r gleich 1 bis 3 ist;
wobei die Arylgruppe, Heterocyclylgruppe und/oder Cycloalkylgruppe gegebenenfalls mit einem oder mehreren Substituenten R²⁷ substituiert sein kann/können;
mit der Maßgabe, dass die Verbindung nicht eine der folgenden Verbindungen ist: 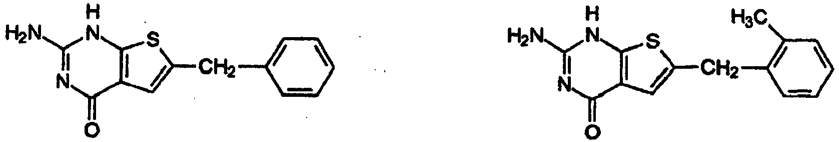 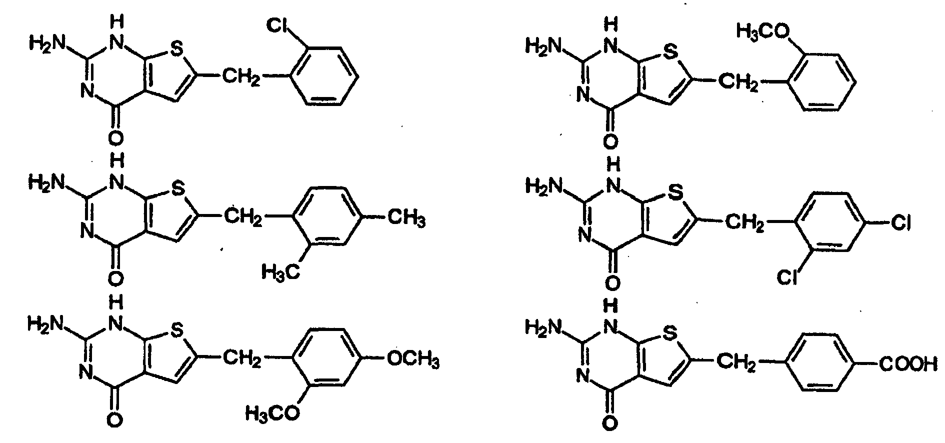 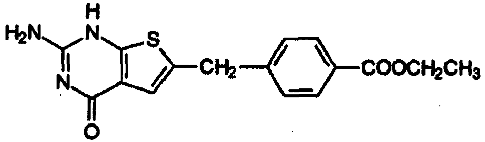 und gegebenenfalls einen oder mehrere pharmazeutisch verträgliche(n) Exzipienten und/oder Träger.

7. Das Arzneimittel gemäß Anspruch 6, wobei R²¹-H, -C₁₋₆-Alkyl oder -(CH₂)ₚ-OR²⁵ ist.

8. Das Arzneimittel gemäß Anspruch 6 oder 7, wobei R²² -H, -C₁₋₆-Alkyl oder Hal ist.

9. Das Arzneimittel gemäß einem der Ansprüche 6 bis 8, wobei R²³ -(CH₂)_{q}-Aryl oder -(CH₂)_{q}-Heteroaryl ist und wobei die Arylgruppe und/oder Heteroarylgruppe gegebenenfalls mit einem oder mehreren Substituenten R²⁷ substituiert sein kann/können.

10. Das Arzneimittel gemäß einem der Ansprüche 6 bis 9, wobei R²³ -Phenyl, -Benzyl oder -Pyridyl ist und wobei die Substituenten unabhängig voneinander ausgewählt sind aus -Hal, -CF₃, -CN, -C₁₋₆-Alkyl, -C(O)-C₁₋₆-Alkyl oder -(CH₂)_{q}NR²⁵R²⁶, wobei R²⁵ und R²⁶ unabhängig aus H und -C₁₋₆-Alkyl ausgewählt sind.

11. Eine Verbindung mit der allgemeinen Formel (II), gegebenenfalls in Form eines pharmazeutisch verträglichen Salzes, Solvats, Polymorphs, Tautomers, Racemats, Enantiomers oder Diastereomers oder Gemischs davon, 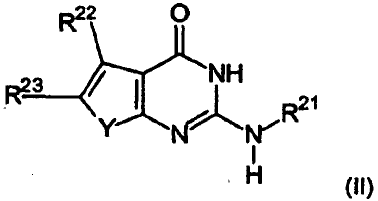 wobei
Y gleich S ist;
R²¹ ausgewählt ist aus -H, -C₁₋₆-Alkyl, -(CH₂)_{q}-Aryl, -(CH₂)_{q}-Heterocyclyl, -(CH₂)_{q}-Cycloalkyl, -(CH₂)ₚ-OR²⁵ und -(CH₂)ₚ-NR²⁵R²⁶;
R²² ausgewählt ist aus -H, -C₁₋₆-Alkyl, -(CH₂)_{q}-Cycloalkyl, -Hal, -CF₃ und -CN;
R²³ ausgewählt ist aus -Aryl, -Heterocyclyl, -Cycloalkyl, -C(-R²⁸)(-R²⁹)-Aryl, -C(-R²⁸)(-R²⁹)-Heterocyclyl und -C(-R²⁸)(-R²⁹)-Cycloalkyl;
R²⁵ ausgewählt ist aus -H, -C₁₋₆-Alkyl und -(CH₂CH₂O)ᵣH;
R²⁶ ausgewählt ist aus -H und -C₁₋₆-Alkyl;
R²⁷ unabhängig ausgewählt ist aus -C₁₋₆-Alkyl, -C(O)-C₁₋₆-Alkyl, -Hal, -CF₃, -CN, -COOR²⁵, -OR²⁵, -(CH₂)_{q}NR²⁵R²⁶, -C(O)-NR²⁵R²⁶ und -NR²⁵-C(O)-C₁₋₆-Alkyl;
R²⁸ und R²⁹ unabhängig ausgewählt sind aus -H, -C₁₋₆-Alkyl, -(CH₂)_{q}-Aryl, -(CH₂)_{q}-Heterocyclyl, -(CH₂)_{q}-Cycloalkyl, -OH, -O-C₁₋₆-Alkyl, -O-CH₂)_{q}-Aryl, -O-(CH₂)_{q}-Heterocyclyl und -O-(CH₂)_{q}-Cycloalkyl;
oder R²⁸ und R²⁹ zusammen =O, -CH₂CH₂-, -CH₂CH₂CH₂- oder -CH₂CH₂CH₂CH₂-sind;
p gleich 1 bis 4 ist;
q gleich 0 bis 4 ist; und
r gleich 1 bis 3 ist;
wobei die Arylgruppe, Heterocyclylgruppe und/oder Cycloalkylgruppe gegebenenfalls mit einem oder mehreren Substituenten R²⁷ substituiert sein kann/können;
wobei die Verbindung zur Verwendung bei der Behandlung, Verbesserung oder Prävention einer viralen Erkrankung ist.

12. Die Verbindung zur Verwendung gemäß Anspruch 11, wobei die Viruserkrankung durch Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae oder Flaviviridae verursacht wird, insbesondere wobei die Viruserkrankung Influenza ist.

13. Die Verbindung zur Verwendung gemäß Anspruch 11 oder 12, wobei R²¹ -H, -C₁₋₆-Alkyl oder -(CH₂)ₚ-OR²⁵ ist.

14. Die Verbindung zur Verwendung gemäß einem der Ansprüche 11 bis 13, wobei R²² -H, -C₁₋₆-Alkyl oder Hal ist.

15. Die Verbindung zur Verwendung gemäß einem der Ansprüche 11 bis 14, wobei R²³ -(CH₂)_{q}-Aryl oder -(CH₂)_{q}-Heteroaryl ist und wobei die Arylgruppe und/oder Heteroarylgruppe gegebenenfalls mit einem oder mehreren Substituenten R²⁷ substituiert sein kann/können.

16. Die Verbindung zur Verwendung gemäß einem der Ansprüche 11 bis 15, wobei R²³ -Phenyl, -Benzyl oder -Pyridyl ist und wobei die Substituenten unabhängig voneinander ausgewählt sind aus -Hal, -CF₃, -CN, -C₁₋₆-Alkyl, -C(O)-C₁₋₆-Alkyl oder -(CH₂)_{q}NR²⁵R²⁶, wobei R²⁵ und R²⁶ unabhängig aus H und -C₁₋₆-Alkyl ausgewählt sind. und gegebenenfalls einen oder mehrere pharmazeutisch verträgliche(n) Exzipienten und/oder Träger.

17. Ein Arzneimittel, umfassend:
(i) eine Verbindung der allgemeinen Formel (II), gegebenenfalls in Form eines pharmazeutisch verträglichen Salzes, Solvats, Polymorphs, Tautomers, Racemats, Enantiomers oder Diastereomers oder Gemischs davon 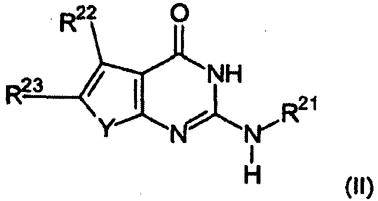 wobei
Y gleich S ist;
R²¹ ausgewählt ist aus -H, -C₁₋₆-Alkyl, -(CH₂)_{q}-Aryl, -(CH₂)_{q}-Heterocyclyl, -(CH₂)_{q}-Cycloalkyl, -(CH₂)ₚ-OR²⁵ und -(CH₂)ₚ-NR²⁵R²⁶;
R²² ausgewählt ist aus -H, -C₁₋₆-Alkyl, -(CH₂)_{q}-Cycloalkyl, -Hal, -CF₃ und -CN;
R²³ ausgewählt ist aus -Aryl, -Heterocyclyl, -Cycloalkyl, -C(-R²⁸)(-R²⁹)-Aryl, -C(-R²⁸)(-R²⁹)-Heterocyclyl und -C(-R²⁸)(-R²⁹)-Cycloalkyl;
R²⁵ ausgewählt ist aus -H, -C₁₋₆-Alkyl und -(CH₂CH₂O)ᵣH;
R²⁶ ausgewählt ist aus -H und -C₁₋₆-Alkyl;
R²⁷ unabhängig ausgewählt ist aus -C₁₋₆-Alkyl, -C(O)-C₁₋₆-Alkyl, -Hal, -CF₃, -CN, -COOR²⁵, -OR²⁵, -(CH₂)_{q}NR²⁵R²⁶, -C(O)-NR²⁵R²⁶ und -NR²⁵-C(O)-C₁₋₆-Alkyl;
R²⁸ und R²⁹ unabhängig ausgewählt sind aus -H, -C₁₋₆-Alkyl, -(CH₂)_{q}-Aryl, -(CH₂)_{q}-Heterocyclyl, -(CH₂)_{q}-Cycloalkyl, -OH, -O-C₁₋₆-Alkyl, -O-(CH₂)_{q}-Aryl, -O-(CH₂)_{q}-Heterocyclyl und -O-(CH₂)_{q}-Cycloalkyl;
oder R²⁸ und R²⁹ zusammen =O, -CH₂CH₂-, -CH₂CH₂CH₂- oder -CH₂CH₂CH₂CH₂-sind;
p gleich 1 bis 4 ist;
q gleich 0 bis 4 ist; und
r gleich 1 bis 3 ist;
wobei die Arylgruppe, Heterocyclylgruppe und/oder Cycloalkylgruppe gegebenenfalls mit einem oder mehreren Substituenten R²⁷ substituiert sein kann/können; und
(ii) eine weitere Verbindung ausgewählt aus:
(a) mindestens einem Polymerase-Inhibitor, der von der Verbindung der allgemeinen Formel (II) verschieden ist;
(b) mindestens einem Neuraminidase-Inhibitor;
(c) mindestens einem M2-Kanal-Inhibitor;
(d) mindestens einem alpha-Glucosidase-Inhibitor;
(e) mindestens einem Liganden eines anderen Influenza-Targets; und
(f) mindestens einem Arzneimittel ausgewählt aus Antibiotika, entzündungshemmenden Mitteln, Lipoxygenase-Inhibitoren, EP-Liganden, Bradykinin-Liganden und Cannabinoid-Liganden;
und gegebenenfalls einen oder mehrere pharmazeutisch verträgliche(n) Exzipienten und/oder Träger.

18. Das Arzneimittel wie in Anspruch 17 definiert, zur Verwendung in der Behandlung, Verbesserung oder Prävention einer Viruserkrankung.

19. Das Arzneimitel zur Verwendung gemäß Anspruch 18, wobei die Viruserkrankung durch Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae oder Flaviviridae verursacht wird, insbesondere wobei die Viruserkrankung Influenza ist.

## Revendications

1. Composé possédant la formule générale (II), facultativement sous la forme d'un sel pharmaceutiquement acceptable, d'un solvate, d'un polymorphe, d'un tautomère, d'un racémate, d'un énantiomère ou d'un diastéréoisomère ou d'un mélange de ceux-ci, 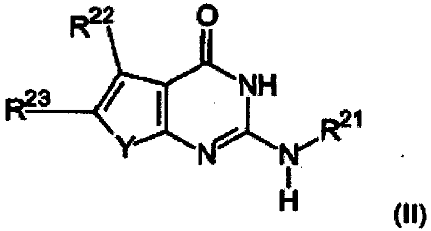 dans laquelle
Y est S ;
R²¹ est choisi parmi -H, un alkyle en C₁ à C₆, un -(CH₂)_{q}-aryle, un -(CH₂)_{q}-hétérocycle, un -(CH₂)_{q}-cycloalkyle, un -(CH₂)ₚ-OR²⁵ et -(CH₂)ₚ-NR²⁵R²⁶ ;
R²² est choisi parmi -H, un alkyle en C₁ à C₆, un -(CH₂)_{q}-cycloalkyle, -Hal, -CF₃ et -CN ;
R²³ est choisi parmi un aryle, un hétérocycle, un cycloalkyle, un -C(-R²⁸)(-R²⁹)-aryle, un -C(-R²⁸)(-R²⁹)-hétérocycle et un -C(-R²⁸)(-R²⁹)-cycloalkyle ;
R²⁵ est choisi parmi -H, un alkyle en C₁ à C₆ et -(CH₂CH₂O)ᵣH ;
R²⁶ est choisi parmi -H et un alkyle en _{C1} à C₆ ;
R²⁷ est choisi indépendamment parmi un alkyle en C₁ à C₆, un -C(O)-alkyle en C₁ à C₆, -Hal, -CF₃, -CN, -COOR²⁵, -OR²⁵, -(CH₂)_{q}NR²⁵R²⁶, -C(O)-NR²⁵R²⁶ et un -NR²⁵-C(O)-alkyle en C₁ à C₆ ;
R²⁸ et R²⁹ sont choisis indépendamment parmi -H, un alkyle en C₁ à C₆, un -(CH₂)_{q}-aryle, un -(CH₂)_{q}-hétérocycle, un -(CH₂)_{q}-cycloalkyle, -OH, un -O-alkyle en C₁ à C₆, un -O-(CH₂)_{q}-aryle, un -O-(CH₂)_{q}-hétérocycle et un -O-(CH₂)_{q}-cycloalkyle ;
ou R²⁸ et R²⁹ sont ensemble =O, -CH₂CH₂-, -CH₂CH₂CH₂- ou -CH₂CH₂CH₂CH₂- ;
p est 1 à 4 ;
q est 0 à 4 ; et
r est 1 à 3 ;
dans lequel le groupe aryle, le groupe hétérocycle et/ou le groupe cycloalkyle peuvent être facultativement substitués avec un ou plusieurs substituants R²⁷ ;
à condition que le composé ne soit pas un des composés suivants :

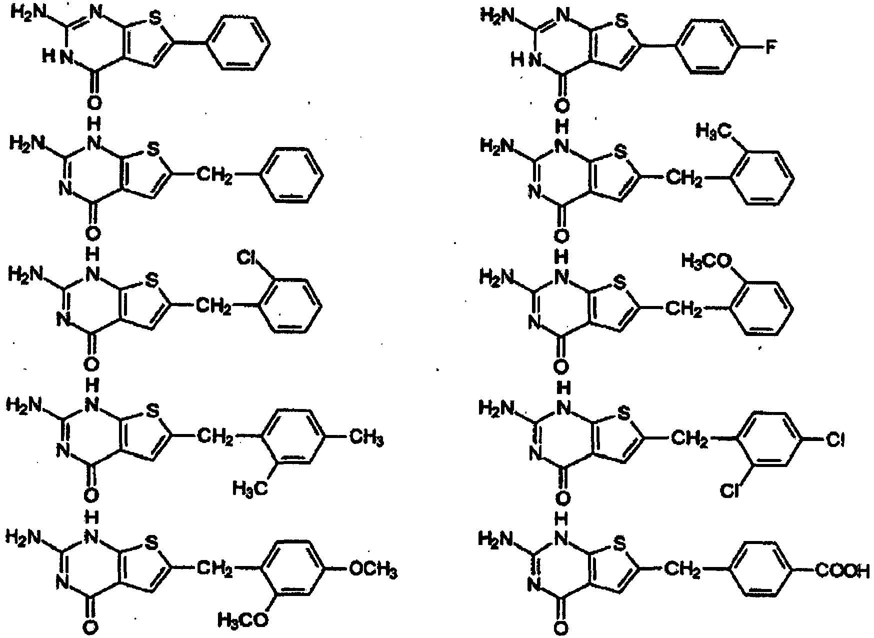

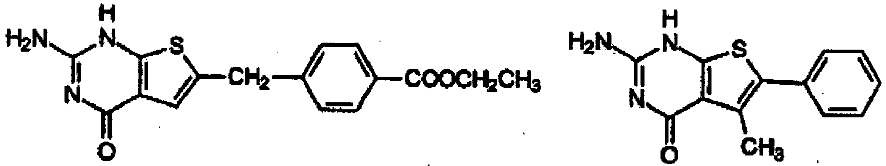

2. Composé selon la revendication 1, dans lequel R²¹ est -H, un alkyle en C₁ à C₆ ou -(CH₂)ₚ-OR²⁵.

3. Composé selon la revendication 1 ou 2, dans lequel R²² est -H, un alkyle en C₁ à C₆ ou -Hal.

4. Composé selon l'une quelconque des revendications précédentes, dans lequel R²³ est un -(CH₂)_{q}-aryle ; ou un -(CH₂)_{q}-hétéroaryle, et dans lequel le groupe aryle et/ou le groupe hétéroaryle peuvent être facultativement substitués avec un ou plusieurs substituants **R²⁷**_{.}

5. Composé selon l'une quelconque des revendications précédentes, dans lequel R²³ est un phényle, un benzyle ou un pyridyle et dans lequel les substituants sont choisis indépendamment parmi -Hal, -CF₃, -CN, un alkyle en C₁ à C₆, un -C(O)-alkyle en C₁ à C₆ ou -(CH₂)_{q}NR²⁵R²⁶, où R²⁵ et R²⁶ sont choisis indépendamment parmi H et un alkyle en C₁ à C₆.

6. Composition pharmaceutique comprenant :
un composé possédant la formule générale (II), facultativement sous la forme d'un sel pharmaceutiquement acceptable, d'un solvate, d'un polymorphe, d'un tautomère, d'un racémate, d'un énantiomère ou d'un diastéréoisomère ou d'un mélange de ceux-ci, 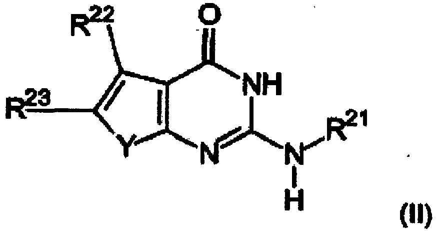 dans laquelle
Y est S ;
R²¹ est choisi parmi -H, un alkyle en C₁ à C₆, un -(CH₂)_{q}-aryle, un -(CH₂)_{q}-hétérocycle, un -(CH₂)_{q}-cycloalkyle, un -(CH₂)ₚ-OR²⁵ et -(CH₂)ₚ-NR²⁵R²⁶ ;
R²² est choisi parmi -H, un alkyle en C₁ à C₆, un -(CH₂)_{q}-cycloalkyle, -Hal, -CF₃ et -CN;
R²³ est choisi parmi un aryle, un hétérocycle, un cycloalkyle, un -C(-R²⁸)(-R²⁹)-aryle, un -C(-R²⁸)(-R²⁹)-hétérocycle et un -C(-R²⁸)(-R²⁹)-cycloalkyle ;
R²⁵ est choisi parmi -H, un alkyle en C₁ à C₆ et -(CH₂CH₂O)ᵣH ;
R²⁶ est choisi parmi -H et un alkyle en C₁ à C₆ ;
R²⁷ est choisi indépendamment parmi un alkyle en C₁ à C₆, un -C(O)-alkyle en C₁ à C₆, -Hal, -CF₃, -CN, -COOR²⁵, -OR²⁵, -(CH₂)_{q}NR²⁵R²⁶, -C(O)-NR²⁵R²⁶ et un -NR²⁵-C(O)-alkyle en C₁ à C₆ ;
R²⁸ et R²⁹ sont choisis indépendamment parmi -H, un alkyle en C₁ à C₆, un -(CH₂)_{q}-aryle, un -(CH₂)_{q}-hétérocycle, un -(CH₂)_{q}-cycloalkyle, -OH, un -O-alkyle en C₁ à C₆, un -O-(CH₂)_{q}-aryle, un -O-(CH₂)_{q}-hétérocycle et un -O-(CH₂)_{q}-cycloalkyle ;
ou R²⁸ et R²⁹ sont ensemble =O, -CH₂CH₂-, -CH₂CH₂CH₂- ou -CH₂CH₂CH₂CH₂- ;
p est 1 à 4 ;
q est 0 à 4 ; et
r est 1 à 3 ;
dans lequel le groupe aryle, le groupe hétérocycle et/ou le groupe cycloalkyle peuvent être facultativement substitués avec un ou plusieurs substituants R²⁷ ;
à condition que le composé ne soit pas un des composés suivants : 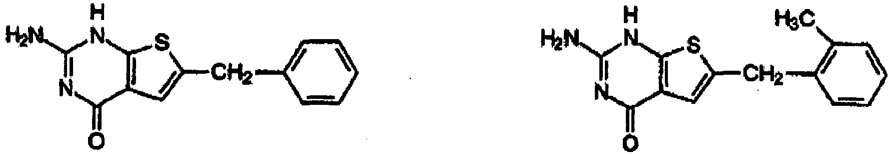 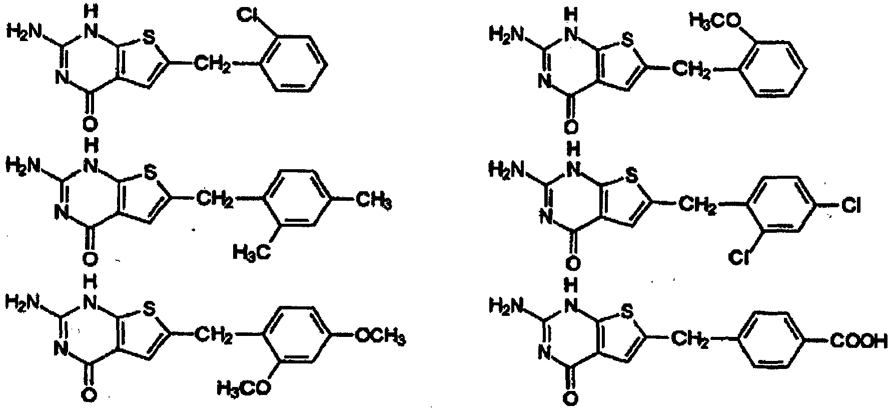 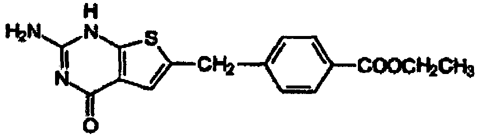 et facultativement un ou plusieurs excipient(s) et/ou support(s) pharmaceutiquement acceptables.

7. Composition pharmaceutique la revendication 6, dans laquelle R²¹ est -H, un alkyle en C₁ à C₆ ou -(CH₂)ₚ-OR²⁵.

8. Composition pharmaceutique la revendication 6 ou 7, dans laquelle R²² est -H, un alkyle en C₁ à C₆ ou -Hal.

9. Composition pharmaceutique selon l'une quelconque des revendications 6 à 8, dans laquelle R²³ est un -(CH₂)_{q}-aryle ; ou un -(CH₂)_{q}-hétéroaryle, et dans lequel le groupe aryle et/ou le groupe hétéroaryle peuvent être facultativement substitués avec un ou plusieurs substituants R²⁷.

10. Composition pharmaceutique selon l'une quelconque des revendications 6 à 9, dans laquelle R²³ est un phényle, un benzyle ou un pyridyle et dans lequel les substituants sont choisis indépendamment parmi -Hal, -CF₃, -CN, un alkyle en C₁ à C₆, un -C(O)-alkyle en C₁ à C₆ ou -(CH₂)_{q}NR²⁵R²⁶, où R²⁵ et R²⁶ sont choisis indépendamment parmi H et un alkyle en C₁ à C₆.

11. Composé possédant la formule générale (II), facultativement sous la forme d'un sel pharmaceutiquement acceptable, d'un solvate, d'un polymorphe, d'un tautomère, d'un racémate, d'un énantiomère ou d'un diastéréoisomère ou d'un mélange de ceux-ci, 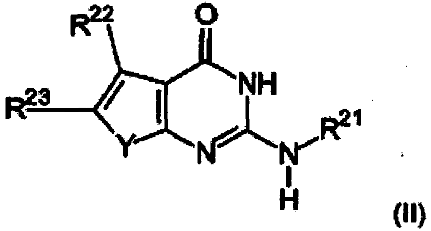 dans laquelle
Y est S ;
R²¹ est choisi parmi -H, un alkyle en C₁ à C₆, un -(CH₂)_{q}-aryle, un -(CH₂)_{q}-hétérocycle, un -(CH₂)_{q}-cycloalkyle, un -(CH₂)ₚ-OR²⁵ et -(CH₂)ₚ-NR²⁵R²⁶ ;
R²² est choisi parmi -H, un alkyle en C₁ à C₆, un -(CH₂)_{q}-cycloalkyle, -Hal, -CF₃ et -CN;
R²³ est choisi parmi un aryle, un hétérocycle, un cycloalkyle, un -C(-R²⁸)(-R²⁹)-aryle, un -C(-R²⁸)(-R²⁹)-hétérocycle et un -C(-R²⁸)(-R²⁹)-cycloalkyle;
R²⁵ est choisi parmi -H, un alkyle en C₁ à C₆ et -(CH₂CH₂O)ᵣH ;
R²⁶ est choisi parmi -H et un alkyle en C₁ à C₆ ;
R²⁷ est choisi indépendamment parmi un alkyle en C₁ à C₆, un -C(O)-alkyle en C₁ à C₆, -Hal, -CF₃, -CN, -COOR²⁵, -OR²⁵, -(CH₂)_{q}NR²⁵R²⁶, -C(O)-NR²⁵R²⁶ et un -NR²⁵-C(O)-alkyle en C₁ à C₆;
R²⁸ et R²⁹ sont choisis indépendamment parmi -H, un alkyle en C₁ à C₆, un -(CH₂)_{q}-aryle, un -(CH₂)_{q}-hétérocycle, un -(CH₂)_{q}-cycloalkyle, -OH, un -O-alkyle en C₁ à C₆, un -O-(CH₂)_{q}-aryle, un -O-(CH₂)_{q}-hétérocycle et un -O-(CH₂)_{q}-cycloalkyle ;
ou R²⁸ et R²⁹ sont ensemble =O, -CH₂CH₂-, -CH₂CH₂CH₂- ou -CH₂CH₂CH₂CH₂- ;
p est 1 à 4 ;
q est 0 à 4 ; et
r est 1 à 3 ;
dans lequel le groupe aryle, le groupe hétérocycle et/ou le groupe cycloalkyle peuvent être facultativement substitués avec un ou plusieurs substituants R²⁷ ;
dans lequel le composé est destiné à être utilisé dans le traitement, l'amélioration ou la prévention d'une maladie virale.

12. Composé pour son utilisation selon la revendication 11, dans lequel la maladie virale est provoquée par *Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae* ou *Flaviviridae ;* plus précisément, dans lequel la maladie virale est la grippe.

13. Composé pour son utilisation selon la revendication 11 ou 12, dans lequel R²¹ est -H, un alkyle en C₁ à C₆ ou -(CH₂)ₚ-OR²⁵.

14. Composé pour son utilisation selon l'une quelconque des revendications 11 à 13, dans lequel R²² est -H, un alkyle en C₁ à C₆ ou -Hal.

15. Composé pour son utilisation selon l'une quelconque des revendications 11 à 14, dans lequel R²³ est un -(CH₂)_{q}-aryle ; ou un -(CH₂)_{q}-hétéroaryle, et dans lequel le groupe aryle et/ou le groupe hétéroaryle peuvent être facultativement substitués avec un ou plusieurs substituants R²⁷.

16. Composé pour son utilisation selon l'une quelconque des revendications 11 à 15, dans lequel R²³ est un phényle, un benzyle ou un pyridyle et dans lequel les substituants sont choisis indépendamment parmi -Hal, -CF₃, -CN, un alkyle en C₁ à C₆, un -C(O)-alkyle en C₁ à C₆ ou -(CH₂)_{q}NR²⁵R²⁶, où R²⁵ et R²⁶ sont choisis indépendamment parmi H et un alkyle en C₁ à C₆,
et facultativement un ou plusieurs excipient(s) et/ou support(s) pharmaceutiquement acceptables.

17. Composition pharmaceutique comprenant :
(i) un composé possédant la formule générale (II), facultativement sous la forme d'un sel pharmaceutiquement acceptable, d'un solvate, d'un polymorphe, d'un tautomère, d'un racémate, d'un énantiomère ou d'un diastéréoisomère ou d'un mélange de ceux-ci, 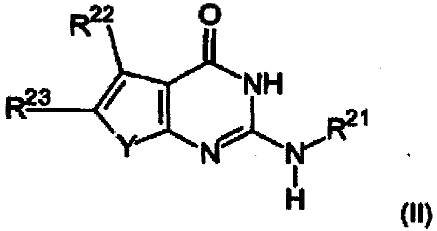 dans laquelle
Y est S ;
R²¹ est choisi parmi -H, un alkyle en C₁ à C₆, un -(CH₂)_{q}-aryle, un -(CH₂)_{q}-hétérocycle, un -(CH₂)_{q}-cycloalkyle, un -(CH₂)ₚ-OR²⁵ et -(CH₂)ₚ-NR²⁵R²⁶ ;
R²² est choisi parmi -H, un alkyle en C₁ à C₆, un -(CH₂)_{q}-cycloalkyle, -Hal, -CF₃ et -CN;
R²³ est choisi parmi un aryle, un hétérocycle, un cycloalkyle, un -C(-R²⁸)(-R²⁹)-aryle, un -C(-R²⁸)(-R²⁹)-hétérocycle et un -C(-R²⁸)(-R²⁹)-cycloalkyle ;
R²⁵ est choisi parmi -H, un alkyle en C₁ à C₆ et -(CH₂CH₂O)ᵣH ;
R²⁶ est choisi parmi -H et un alkyle en C₁ à C₆ ;
R²⁷ est choisi indépendamment parmi un alkyle en C₁ à C₆, un -C(O)-alkyle en C₁ à C₆, -Hal, -CF₃, -CN, -COOR²⁵, -OR²⁵, -(CH₂)_{q}NR²⁵R²⁶, -C(O)-NR²⁵R²⁶ et un -NR²⁵-C(O)-alkyle en C₁ à C₆ ;
R²⁸ et R²⁹ sont choisis indépendamment parmi -H, un alkyle en C₁ à C₆, un -(CH₂)_{q}-aryle, un -(CH₂)_{q}-hétérocycle, un -(CH₂)_{q}-cycloalkyle, -OH, un -O-alkyle en C₁ à C₆, un -O-(CH₂)_{q}-aryle, un -O-(CH₂)_{q}-hétérocycle et un -O-(CH₂)_{q}-cycloalkyle ;
ou R²⁸ et R²⁹ sont ensemble =O, -CH₂CH₂-, -CH₂CH₂CH₂- ou -CH₂CH₂CH₂CH₂- ;
p est 1 à 4 ;
q est 0 à 4 ; et
r est 1 à 3 ;
dans lequel le groupe aryle, le groupe hétérocycle et/ou le groupe cycloalkyle peuvent être facultativement substitués avec un ou plusieurs substituants R²⁷ ;
et
(ii) un composé supplémentaire choisi parmi :
(a) au moins un inhibiteur de polymérase qui est différent du composé possédant la formule générale (II) ;
(b) au moins un inhibiteur de neuramidase ;
(c) au moins un inhibiteur de canal M2 ;
(d) au moins un inhibiteur d'alpha glucosidase ;
(e) au moins un ligand d'une autre cible de la grippe ; et
(f) au moins un médicament choisi parmi les antibiotiques, les agents anti-inflammatoires, les inhibiteurs de lipoxygénases, les ligands EP, les ligands de la bradykinine et les ligands cannabinoïdes ;
et facultativement un ou plusieurs excipient(s) et/ou support(s) pharmaceutiquement acceptables.

18. Composition pharmaceutique telle que définie dans la revendication 17, pour son utilisation dans le traitement, l'amélioration ou la prévention d'une maladie virale.

19. Composition pharmaceutique pour son utilisation selon la revendication 18, dans laquelle la maladie virale est provoquée par *Herpesviridae, Retroviridae, Filoviridae, Paramyxoviridae, Rhabdoviridae, Orthomyxoviridae, Bunyaviridae, Arenaviridae, Coronaviridae, Picornaviridae, Togaviridae* ou *Flaviviridae ;* plus précisément, dans laquelle la maladie virale est la grippe.
